# EUROPEAN PATENT SPECIFICATION

(11) **EP 0 635 055 B1**
(45) Date of publication and mention of the grant of the patent: **23.12.1998**
(21) Application number: 93907710.3
(22) Date of filing: 05.04.1993
(51) Int. Cl.: C12N 15/31, C07K 14/195, C12P 21/02, A61K 39/102, C12N 1/21

(54) **HAEMOPHILUS SOMNUS IMMUNOGENIC PROTEINS**
HAEMOPHILUS SOMNUS IMMUNOGENE PROTEINE
PROTEINES IMMUNOGENES DERIVEES DE HAEMOPHILUS SOMNUS

(30) Priority: 09.04.1992 US 865050; 04.06.1992 US 893424; 04.06.1992 US 893426; 29.03.1993 US 38287; 29.03.1993 US 38288; 29.03.1993 US 38719
(43) Date of publication of application: 25.01.1995
(73) Proprietor: The University of Saskatchewan, Saskatoon, Saskatchewan S7N 0W0 (CA)
(72) Inventor: POTTER, Andrew, A., Saskatoon, Saskatchewan S7H 3S5 (CA); PONTAROLLO, Reno, A., Saskatoon, Saskatchevan S7N 1H6 (CA); PFEIFFER, Cheryl, G., Vancouver, B.C., V6E 1S8. (CA); THEISEN, Michael, DK-1957 Frederiks Ber GC (DK); HARLAND, Richard, J., Saskatoon, Saskatchewan S7H 2M5 (CA); RIOUX, Clement, Saskatoon, Saskatchewan S7K 3J5 (CA)
(74) Representative: Silveston, Judith
(86) International application number: CA9300135
(87) International publication number: WO9321323

(56) References cited:
- ABSTRACTS OF THE GENERAL MEETING OF THE AMERICAN SOCIETY OF MICROBIOLOGY vol. 92, no. 0, 8 April 1992, page 88 M. THEISEN ET AL 'Cloning and characterization of lppB, agene encoding an Antigenic 40-kilodalton lipoprotein of Haemophilus somnus'
- INFECTION AND IMMUNITY. vol. 60, no. 3, March 1992, WASHINGTON US pages 826 - 831 M. THEISEN ET AL 'Molecular cloning , nucleotide sequence , and characterization of a 40,000-molecular-weight Lipoprotein of Haemophilus somnus' cited in the application
- INFECTION AND IMMUNITY. vol. 56, no. 10, October 1988, WASHINGTON US pages 2736 - 2742 L. B. CORBEIL ET AL 'Cloning and Expression of genes encoding Haemophilussomnus Antigens'
- INFECTION AND IMMUNITY. vol. 59, no. 12, December 1991, WASHINGTON US pages 4295 - 4301 L. B. CORBEIL ET AL 'Characterization of Immunodominant Surface Antigens of Haemophilus somnus'
- INFECTION AND IMMUNITY. vol. 61, no. 5, May 1993, WASHINGTON US pages 1793 - 1798 M. THEISEN ET AL 'Molecular cloning, Nucleotide sequence, and characterization of lppB, encoding an Antigenic 40-Kilodalton Lipoprotein of Haemophilus somnus'

## Description

### Technical Field

The present invention relates generally to bacterial antigens. More particularly, the present invention pertains to proteins derived from *Haemophilus. somnus* and the use of the same in vaccine compositions.

### Background

*Haemophilus somnus* is a Gram negative bacterium which is related to several *Actinobacillus* species and appears to be identical to *Histophilus ovis* and *Haemophilus agni* (Philbey *et al., Aust. Vet. J*. (1991) 88:387-390. *H. somnus* causes a number of disease syndromes in animals. The bacterium is commonly associated with thromboembolic meningoencephalitis (ITEME), septicemia, arthritis, and pneumonia (Corbeil, L.B., Can. *J*. *Vet. Res.* (1990) 54:S57-S62; Harris, F.W., and Janzen, E.D., *Can. Vet. J.* (1990) 30:816-822; Humphrey, J.D., and Stephens, L.R., *Vet. Bull.* (1983) 53:987-1004). These diseases can cause significant economic losses to the farm industry. Currently available vaccines are either based on killed whole cells or on outer membrane protein (OMP) preparations. (See, *e.g.* U.S. Patent Nos. 4,981,685 and 4,877,613). However, whole cell bacterins and surface protein extracts often contain immunosuppressive components which can render animals more susceptible to infection. Furthermore, an OMP enriched vaccine has only been shown to offer significant protection against *H. somnus* induced disease in an experimental challenge model (Harland, R.J., *et* *al., Res. Work. Anim. Dis.* 71st (1990) 29:6). Subunit vaccines, *i.e.* vaccines including select proteins separated from the whole bacterium, afford a method for overcoming the problems inherent in the use of the above-described vaccines.

Iron is an essential nutrient for bacterial growth and the ability to acquire iron from a host's iron-limiting environment is necessary to establish and maintain an infection. A correlation between virulence and the ability to scavenge iron from the host has been shown (Archibald, F.S., and DeVoe, I.W.*, FEMS Microbiol. Lett.* (1979) 6:159-162; Archibald, F.S., and DeVoe, I.W., *Infect. Immun.* (1980) 27:322-334; Herrington, D.A., and Sparling, F.P., *Infect. Immun.* (1985) 48:248-251; Weinberg, E.D., *Microbiol. Rev.* (1978) 42:45-66).

Bacteria can scavenge iron from a number of sources. Iron-containing compounds, such as free heme, haemoglobin, myoglobin, transferrin, lactoferrin, catalase, cytochromes, haem-haemopexin, haem-albumin, haemoglobin-haptoglobulin, and the like, can provide iron, depending on the bacterium in question. A limited number of Gram-negative bacteria, including *Haemophilus* species, can utilize haemin as a source of iron.

Bacteria have evolved a number of mechanisms to capture needed iron. Acquisition of iron from host iron sources may be facilitated by the production of haemolysins and cytolysins which lyse host cells and release intracellular iron complexes. Iron can then be captured by a variety of methods. For example, *E. coli* uses siderophores to chelate external iron which is then bound to a cognate receptor for subsequent internalization. Cross, J.G., *Microbiol. Rev.* (1989) 53:517-530; Nellands, *J.B., Annu. Rev. Microbiol.* (1982) 36:285-309. Unlike *E. coli, H. influenzae* appears to capture iron by a siderophore-independent receptor mediated process. Schryvers, A.B.*,* J. *Med. Microbiol.* (1989) 29:121-130; Lee, B.C., *Infect. Immun.* (1992) 60:810-816. Both haemin-binding proteins and haemolysins have been shown in *Plesiomonas shigelloides* (Daskaleros, P.A., et al., Infect. Immun. (1991) 59:2706-2711). Similarly, *H. influenzae* has been shown to possess haemin-binding proteins (Lee, B.C., Infect. Immun. (1992) 60:810-816 and Hanson, M.S. and Hansen, E.J., Mol. Microbiol. (1991) 5:267-278). A transferrin-binding protein has been isolated from *H. somnus* (WO90/12591).

Haemolysins and cytolysins have been shown in a number of other bacteria. A. pleuropneumoniae strains produce several cytolysins. See, e.g. Rycroft, A.N., et al., J. Gen. Microbiol. (1991) 137:561-568 (describing a 120 kDa cytolysin from A. pleuropneumoniae); Chang, Y.F., et al., DNA (1989) 8:635-647 (describing a cytolysin isolated from A. pleuropneumoniae serotype 5); Kamp, E.M., et al., Abstr. CRWAD (1990) 1990:270 (describing the presence of 103, 105 and 120 kDa cytolysins in A. pleuronneumoniae strains) and Welch, R.A., Mol. Microbiol. (1991) 5:521-528 (reviewing cytolysins of gram negative bacteria including cytolysins from A. pleuronneumoniae). One of these cytolysins appears to be homologous to the alpha-hemolysin of E. coli and another to the leukotoxin of Pasteurella haemolytica. Welch, R.A., Mol. Microbiol. (1991) 5:521-528. These proteins have a molecular mass of approximately 105,000 kDa and are protective in mouse and pig animal models against challenge with the homologous serotype. However, cross-serotype protection is limited at best (Higgins, R., et al., Can. J. Vet. (1985) 26:86-89; MacInnes, J.I., et al., Infect. Immun. (1987) 55:1626-1634. The genes for two of these proteins have been cloned and expressed in E. coli and their nucleotide sequence determined. Chang, Y.F., et al., J. Bacteriol. (1991) 173:5151-5158 (describing the nucleotide sequence for an A. pleuronneumoniae serotype 5 cytolysin); and Frey, J., et al., Infect. Immun. (1991) 59:3026-3032 (describing the nucleotide sequence for an A. pleuropneumoniae serotype 1 cytolysin). However, haemin-binding proteins and haemolysins from *H. somnus* have not heretofore been isolated.

The outer membrane of *H. somnus* includes a 40 kDa protein (as determined by SDS-PAGE) which reacts with convalescent serum (Corbeil, L.B., *et al.*, *infect. Immun.* (1987) 55:1381-1386; Goglolewski, R.P., *et al., Infect. Immun.* (1988) 56:2307-2316). Additionally, antibodies directed against a 40 kDa OMP have been shown to prevent infection *in vitro* in a neutralization experiment (Gogolewski *et al.,* supra) and a seroreactive protein of 40 kDa is present in all *H. somnus* isolates that have been tested (Corbeil *et al*., 1987).

A 39 kDa OMP, antigenically distinct from the 40 kDa OMP described above, has also been identified. This protein reacts with convalescent-phase serum and is conserved among all *H. somnus* isolates tested.

An increasing number of bacterial antigens have now been identified as lipoproteins (Anderson, B.E., *et al., J. Bacteriol.* (1988) 170:4493-4500; Bricker, T.M., *et al., Infect. Immun.* (1988) 56:295-301; Hanson, M.S., and Hansen, E.J., *Mol. Microbiol.* (1991) 5:267-278; Hubbard, C.L., *et al., Infect. Immun.* (1991) 59:1521-1528; Nelson, M.B., *et al., Infect. Immun.* (1988) 56:128-134; Thirkell, D., *et al., Infect. Immun.* (1991) 59:781-784). These lipoproteins are generally localized in the envelope of the cell and are therefore exposed to the host's immune system. It has been shown that the murein lipoprotein from the outer membrane of *Escherichia coli* acts as a potent activator of murine lymphocytes, inducing both proliferation and immunoglobulin secretion (Bessler, W., *et al. Z. Immun.* (1977) 153:11-22; Melchers, F., *et al*. *J. Exp. Med.* (1975 142:473-482). The active lipoprotein portion of the protein has been shown to reside in the N-terminal fatty acid containing region of the protein. Recent studies using synthetic lipopeptides based on this protein show that even short peptides, containing two to five amino acids covalently linked to palmitate, are able to activate murine lymphocytes (Bessler, W.G., *et al. J. Immunol.* (1985) 135:1900-1905).

A lipoprotein from *H. somnus* has been positively identified. This protein, termed "LppA", is an OMP with an apparent molecular mass of 40 kDa, as determined by gel electrophoresis. The nucleotide sequence for LppA has been determined (Theisen, M., *et al., Infect. Immun.* (1992) 60:826-831). However, the protective capability of this protein has not previously been studied.

A second lipoprotein, termed "LppB", from Haemophilus somnus is known. It is reported that a genomic library of Haemophilus somnus in E.coli was screened with bovine hyperimmune sera and a clone was found which encoded a strongly seroreactive 40 kDa protein (Theisen, M. et al Abstr. Gen. Meeting. Am. Soc. Microbiol. (92nd meeting), New Orleans, May 1992). It is also reported that the entire DNA insert was sequenced and it was found that the larger of two open reading frames encoded the seroreactive protein. This protein is said to be an LppB lipoprotein.

The present invention is based on the discovery of immunogenic proteins from H. somnus and the isolation of the various genes coding therefor. These proteins, may be the native protein, immunogenic fragments thereof, analogs thereof, or chimeric proteins including the same. Novel subunit vaccines to provide protection from H. somnus infection in vertebrate subjects comprise the LppB protein, fragments or analogs thereof, and/or chimeric proteins comprising the same, either alone or in combination with other immunogenic H. somnus proteins or with other antigens.

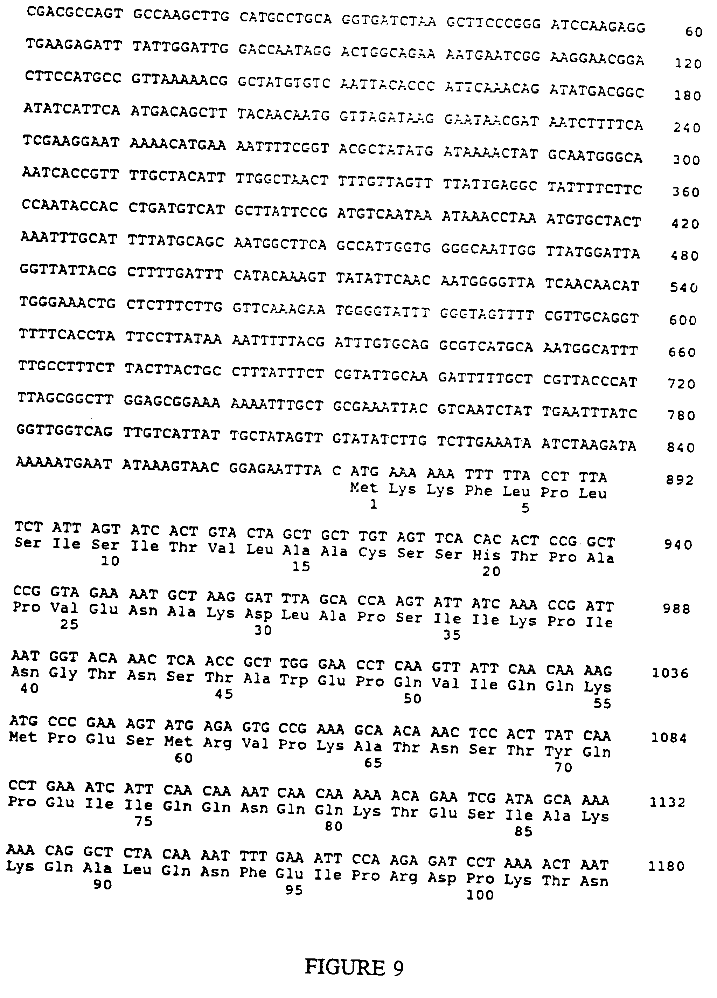

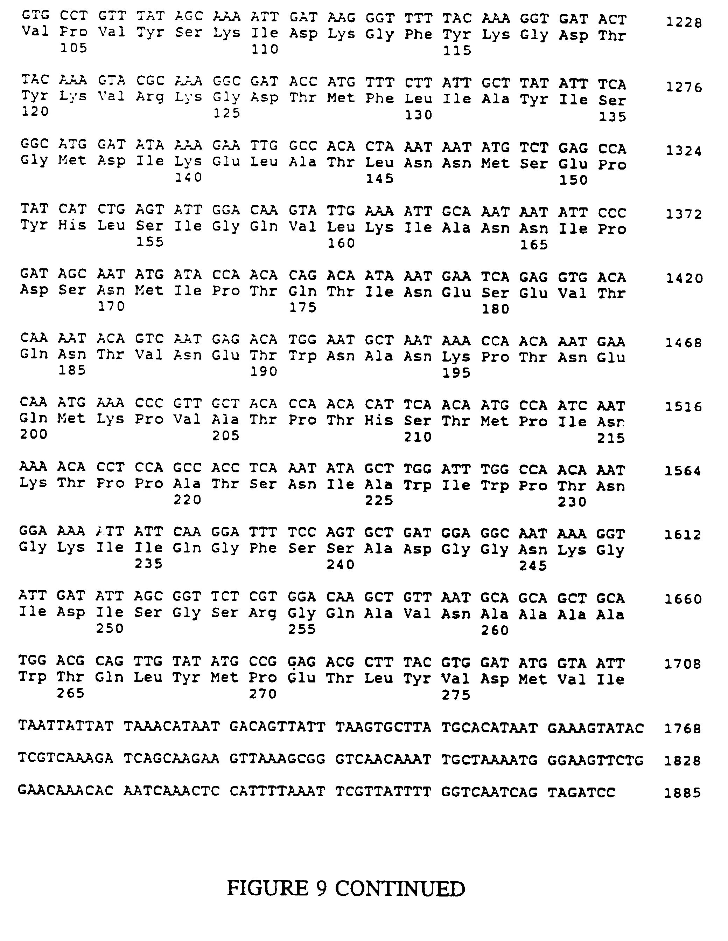

The present invention provides a vaccine composition comprising a pharmaceutically acceptable vehicle and a recombinant immunogenic Haemophilus somnus protein, capable of eliciting a protective immune response against Haemophilus somnus, which protein may be lipidated or non-lipidated and comprises
(a) an amino acid sequence shown at positions 1 to 279, inclusive, of Figure 9; or
(b) an amino acid sequence shown at positions 17 to 279, inclusive, of Figure 9; or
(c) an amino acid sequence which is at least 90% homologous to the amino acid sequence of (a) or (b); or
(d) a fragment of an amino acid sequence according to (a), (b) or (c).

As described in greater detail below, the protein may be non-lipidated or lipidated by a lipid moiety not normally found in association with the protein or lipidated by a lipid moiety usually found in association with the protein.

The immunogenic protein of the vaccine compositions of the present invention may be a fusion protein, that is a protein in which the amino acid sequence of (a), (b), (c) or (d) above is fused to a non-Haemophilus somnus amino acid sequence. Examples of such proteins are discussed herein.

The vaccine compositions may comprise more than one immunogenic protein as described above and may in addition to an LppB protein comprise a Haemophilus somnus protein other than an LppB protein. Other Haemophilus somnus proteins, immunogenic fragments thereof, analogs thereof and chimeric proteins including the same are described herein.

The present invention also provides a method of producing a vaccine composition, said method comprising:
(1) culturing a transformed host cell, the host cell having been transformed with a recombinant vector, under conditions whereby the protein encoded by the coding sequence present in said recombinant vector is expressed, the recombinant vector comprising:
   (i) a nucleotide sequence comprising a coding sequence for an immunogenic Haemophilus somnus protein capable of eliciting a protective immune response against Haemophilus somnus, which protein comprises
      (a) an amino acid sequence shown at positions 1 to 279, inclusive, of Figure 9; or
      (b) an amino acid sequence shown at positions 17 to 279, inclusive, of Figure 9; or
      (c) an amino acid sequence which is at least 90% homologous to the amino acid sequence of (a) or (b); or
      (d) a fragment of an amino acid sequence according to (a), (b) or (c);
         and
   (ii) control sequences that are operably linked to said nucleotide sequence whereby said coding sequence can be transcribed and translated in a host cell, and at least one of said control sequences is heterologous to said coding sequence; and
(2) admixing the expressed protein with a pharmaceutically acceptable vehicle.

The method according to the invention may also comprise the step of transforming a host cell with the recombinant vector to obtain the transformed host cell.

The present invention further provides use of a recombinant immunogenic Haemophilus somnus protein in the manufacture of a vaccine for treating or preventing Haemophilus somnus infection in a vertebrate subject, the protein being capable of eliciting a protective immune response against Haemophilus somnus, being lipidated or non-lipidated and comprising
(a) an amino acid sequence shown at positions 1 to 279, inclusive,
   of Figure 9; or
(b) an amino acid sequence shown at positions 17 to 279, inclusive, of Figure 9; or
(c) an amino acid sequence which is at least 90% homologous to the amino acid sequence of (a) or (b); or
(d) a fragment of an amino acid sequence according to (a), (b) or (c).

Especially provided is the use of such a protein in the manufacture of a vaccine for the treatment of or prevention of thromboembolic meningoencephalitis, septicemia, arthritis, pneumonia, myocarditis, pericarditis, spontaneous abortion, infertility and/or mastitis caused by infection with Haemophilus somnus.

A further aspect of the present invention is a recombinant carrier virus capable of expressing an immunogenic Haemophilus somnus protein, capable of eliciting a protective immune response against Haemophilus somnus, which protein comprises
(a) an amino acid sequence shown at positions 1 to 279, inclusive, of Figure 9; or
(b) an amino acid sequence shown at positions 17 to 279, inclusive, of Figure 9; or
(c) an amino acid sequence which is at least 90% homologous to the amino acid sequence of (a) or (b); or
(d) a fragment of an amino acid sequence according to (a), (b) or (c).

In accordance with the invention there is provided a vaccine composition comprising a pharmaceutically acceptable vehicle and a recombinant carrier virus as described above. The carrier virus may be a pox virus, advantageously the vaccina virus, an adenovirus or a herpes virus.

Also provided by the invention is a pharmaceutical preparation suitable for nucleic acid immunization, which preparation comprises a nucleic acid sequence encoding an immunogenic Haemophilus somnus protein, capable of eliciting a protective immune response against Haemophilus somnus, which protein comprises
(a) an amino acid sequence shown at positions 1 to 279, inclusive, of Figure 9; or
(b) an amino acid sequence shown at positions 17 to 279, inclusive, of Figure 9; or
(c) an amino acid sequence which is at least 90% homologous to the amino acid sequence of (a) or (b); or
(d) a fragment of an amino acid sequence according to (a), (b) or (c),

Such a nucleic acid sequence may be in a form suitable for administration directly to the vertebrate subject or in a form suitable for introduction into cells belonging to the vertebrate subject by gene transfer.

Figure 1 shows the location of the *hly* and *hmb* gene on the plasmids pRAP117, pRAP401 and pRAP501.

Figure 2 depicts the nucleotide sequence of plasmid pRAP501. Also shown are the deduced amino acid sequences of the various open reading frames (ORFs), including ORF1 which encodes the *H. somnus* haemin-binding protein.

Figure 3 shows the ORFs in pRAP501, as deduced from the sequence shown in Figure 2.

Figure 4 depicts the deduced amino acid sequence for the *H. somnus* haemin-binding protein.

Figure 5 shows the nucleotide sequence contained in plasmid pGCH5. The sequence includes the *lkt*A gene from *Pasteurella haemolytica* fused with a truncated *hmb* gene.

Figure 6 depicts the nucleotide sequence contained in plasmid pGCH4. The sequence includes the *lkt*A gene from *P. haemolytica* fused with a truncated *hmb* gene.

Figure 7 depicts the nucleotide sequence and deduced amino acid sequence of the *H. somnus lpp*A region. The sequence of the antisense strand is shown with numbering starting from the 5'-end Shine-Dalgarno (SD) sequence. The transcriptional start of the *lpp*A gene is indicated by 1.

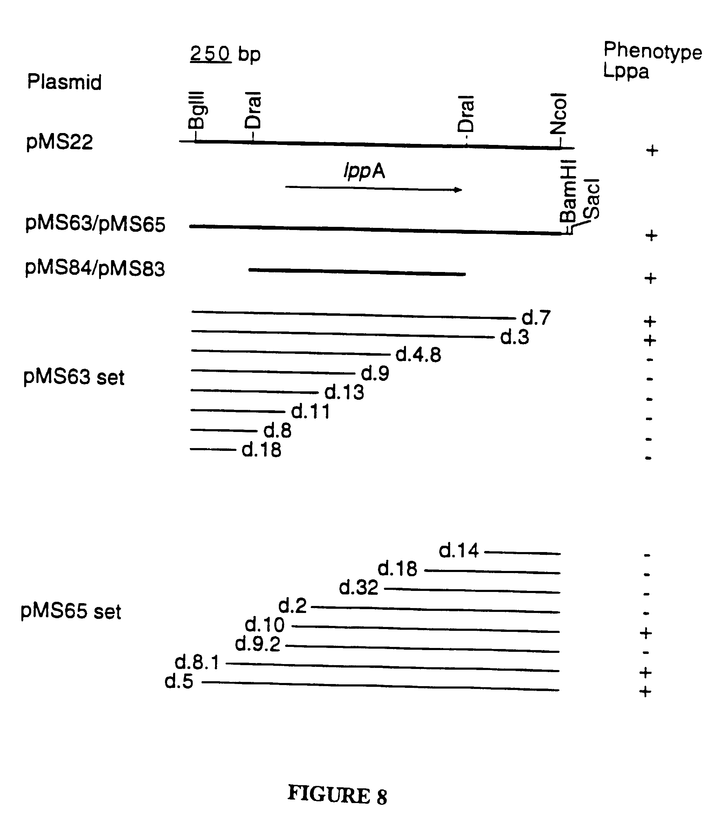

Figure 8 shows the structure and properties of plasmids described in Example 1. The top line shows a partial restriction map of plasmid pMS22 with relevant sites shown. The arrow indicates the location and direction of transcription of the *lpp*A gene. The shaded bars beneath the arrow illustrate the DNA cloned in each of the indicated plasmids. Plasmid names indicated with a slash denote fragments cloned in both orientations. The lower two sets of lines show the DNA remaining in the deletion plasmids used for determining the nucleotide sequence of the *lpp*A gene. The far right column indicates the ability of the various plasmids to direct the synthesis of LppA in JM105.

Figure 9 shows the nucleotide sequence and deduced amino acid sequence of the gene encoding *H. somnus* LppB. The preprotein is encoded by nucleotide positions 872 through 1708 (amino acid residues 1 through 279). The mature protein is encoded by nucleotide positions 920 through 1708 (amino acid residues 17 through 279).

Figure 10 depicts the nucleotide sequence and predicted amino acid sequence of the gene encoding *H. somnus* LppC. The preprotein spans nucleotide positions 108 through 1850 (amino acid residues 1 through 581), with the spanning positions 171 through 1850 (amino acids 22 through 581).

Figure 11 depicts the nucleotide sequence and predicted amino acid sequence contained in plasmid pCRR28. The sequence includes the *lkt*A gene from *P. haemolytica* fused with the *lpp*B gene.

### Detailed Description

The practice of the present invention will employ, unless otherwise indicated, conventional techniques of molecular biology, microbiology, virology, recombinant DNA technology, and immunology, which are within the skill of the art. Such techniques are explained fully in the literature. *See, e.g.,* Sambrook, Fritsch & Maniatis, *Molecular Cloning: A Laboratory Manual,* Second Edition (1989); *DNA Cloning,* Vols. I and II (D.N. Glover ed. 1985); *Oligonucleotide Synthesis* (M.J. Gait ed. 1984); *Nucleic Acid Hybridization* (B.D. Hames & S.J. Higgins eds. 1984); *Animal Cell Culture* (R.K. Freshney ed. 1986); *Immobilized Cells and Enzymes* (IRL press, 1986); Perbal, B., *A Practical Guide to Molecular Cloning* (1984); the series, *Methods In Enzymology* (S. Colowick and N. Kaplan eds., Academic Press, Inc.); and *Handbook of Experimental Immunology,* Vols. I-IV (D.M. Weir and C.C. Blackwell eds., 1986, Blackwell Scientific Publications).

All publications, patents and patent applications cited herein, whether *supra* or *infra,* are hereby incorporated by reference in their entirety.

### A. Definitions

In describing the present invention, the following terms will be employed, and are intended to be defined as indicated below.

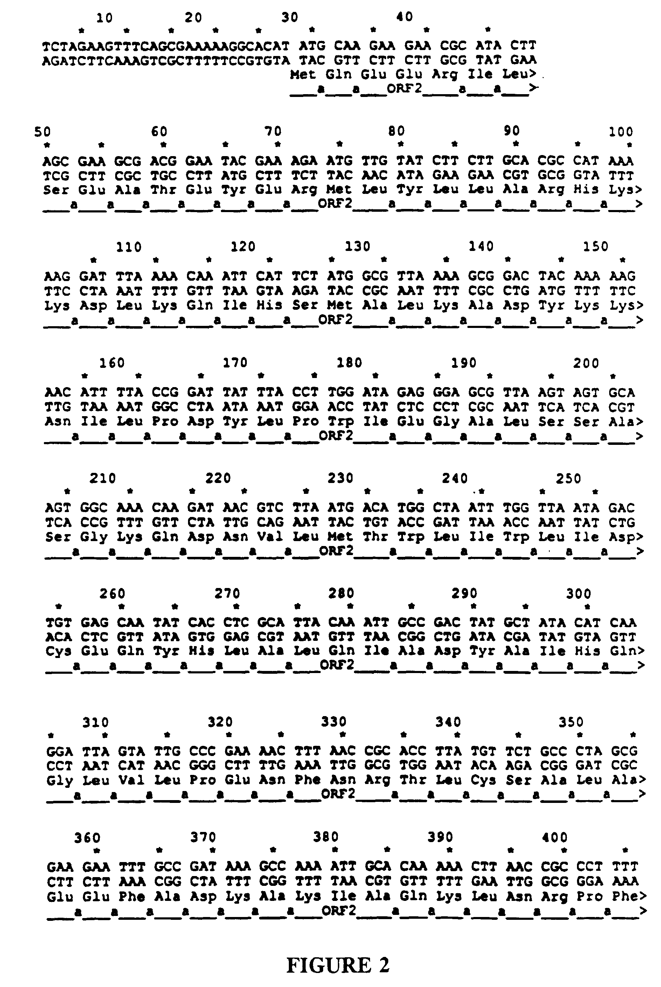

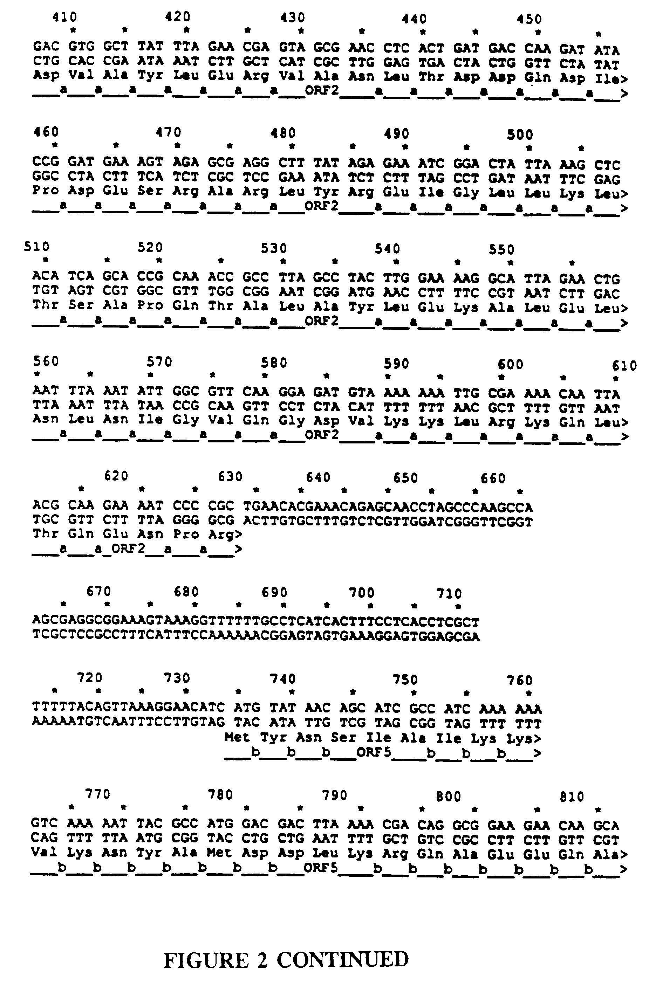

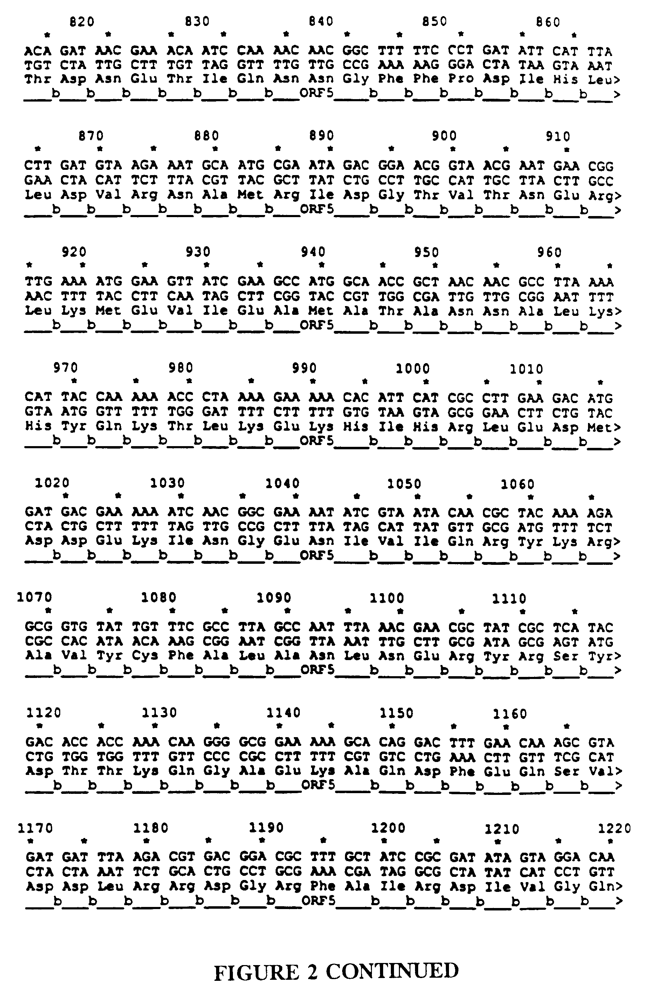

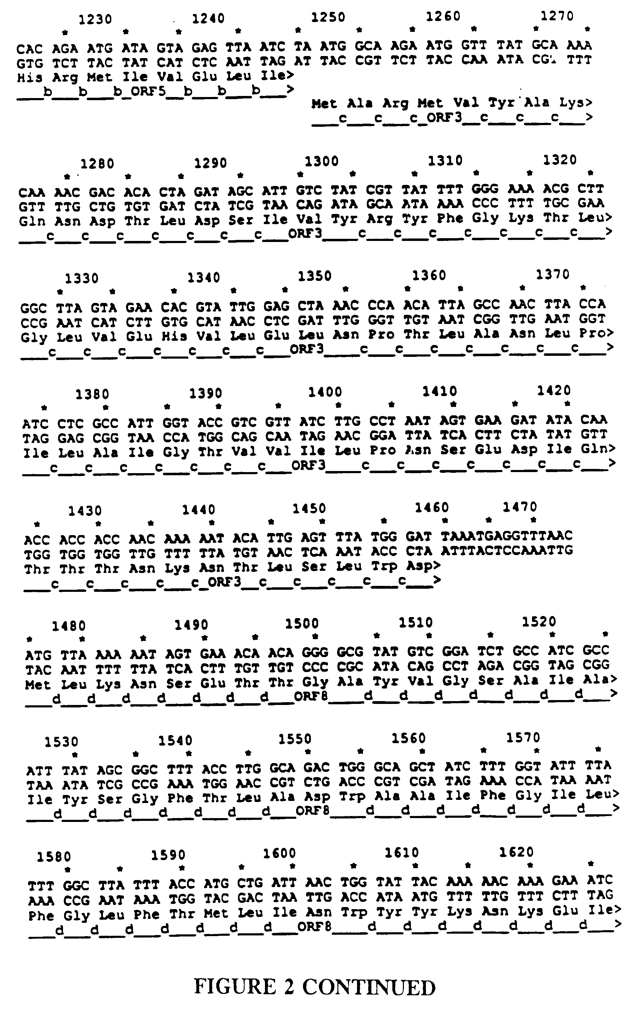

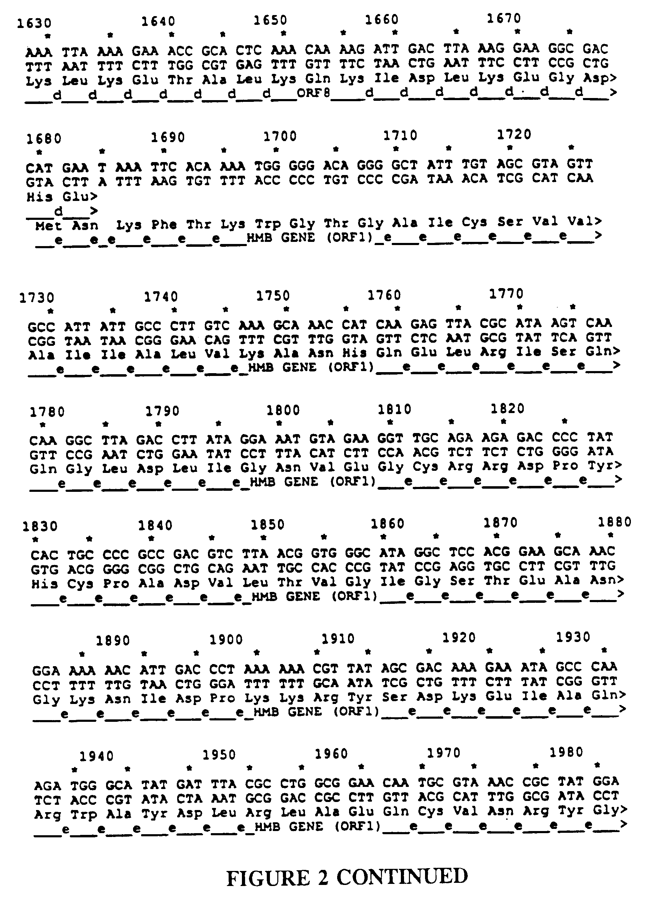

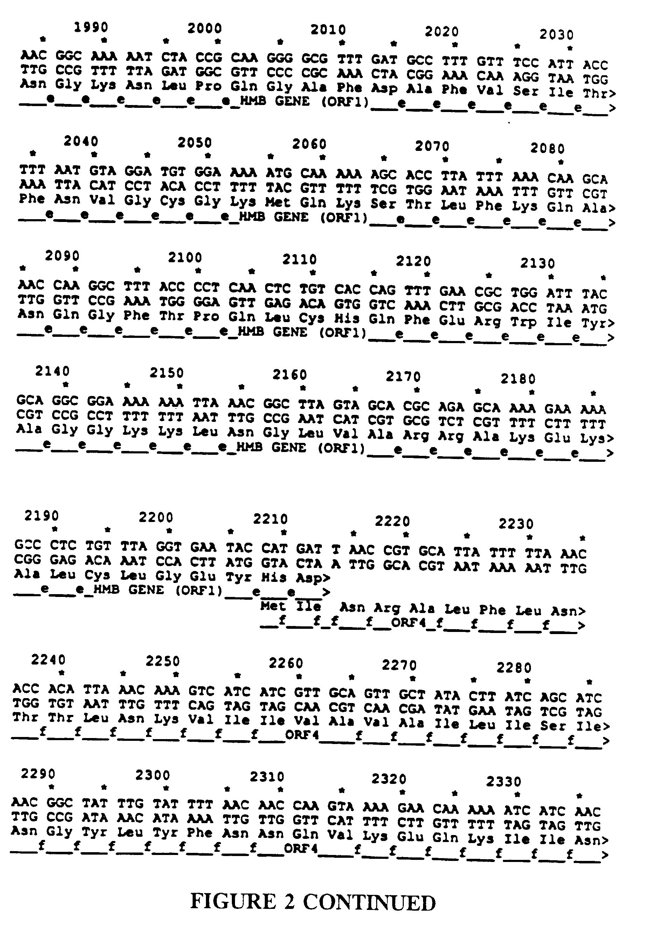

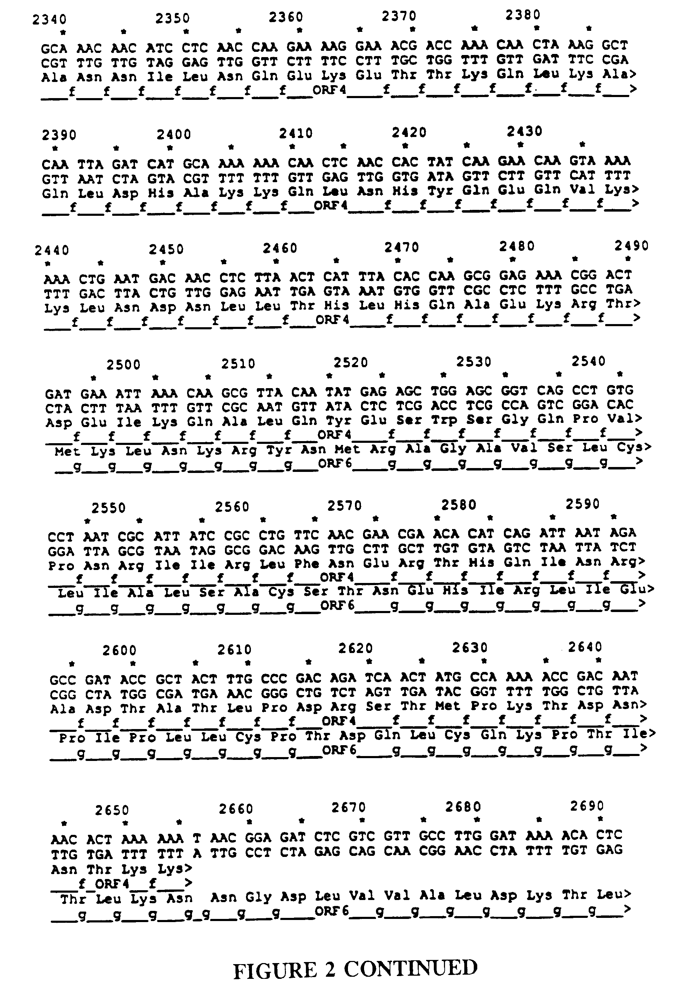

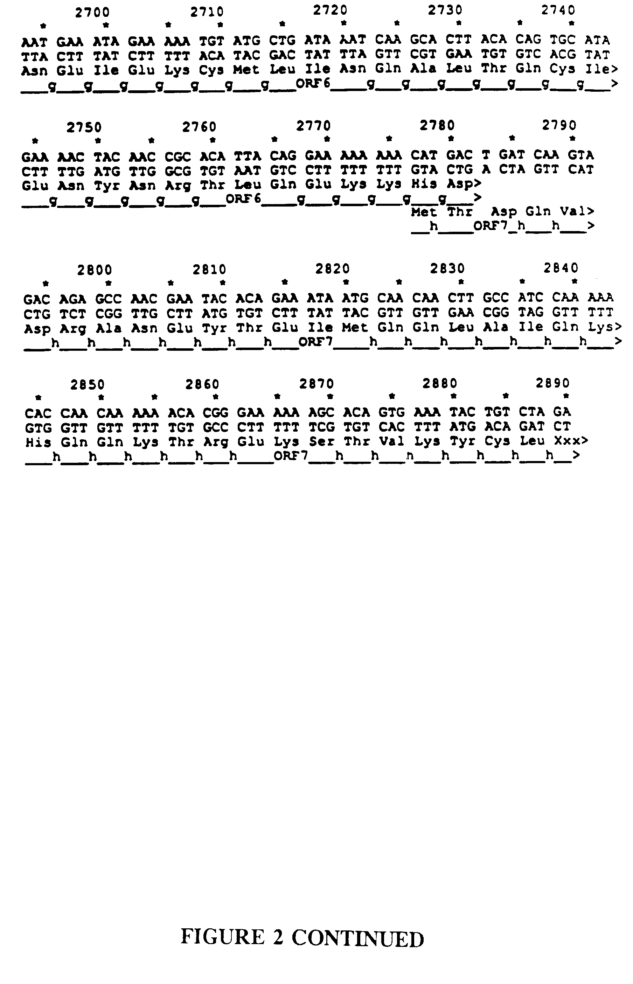

The term "*H. somnus* haemin-binding protein" or a nucleotide sequence encoding the same, intends a protein or a nucleotide sequence, respectively, which is derived from the haemin-binding (*hmb*) gene from *H. somnus* and found in plasmid pRAP117 (ATCC Accession No. 68952) and depicted as ORF1 in Figure 2.

The term *"H. somnus* haemolysin" or a nucleotide sequence encoding the same, intends a protein or a nucleotide sequence, respectively, which is derived from the haemolysin (*hly*) gene found in plasmid pAA504.

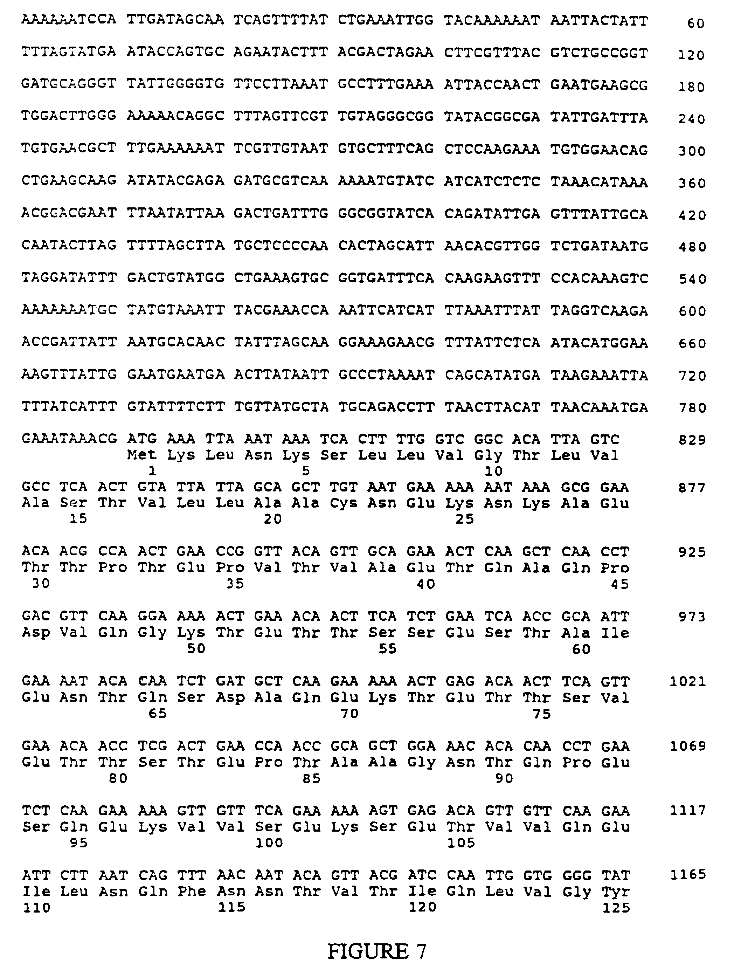

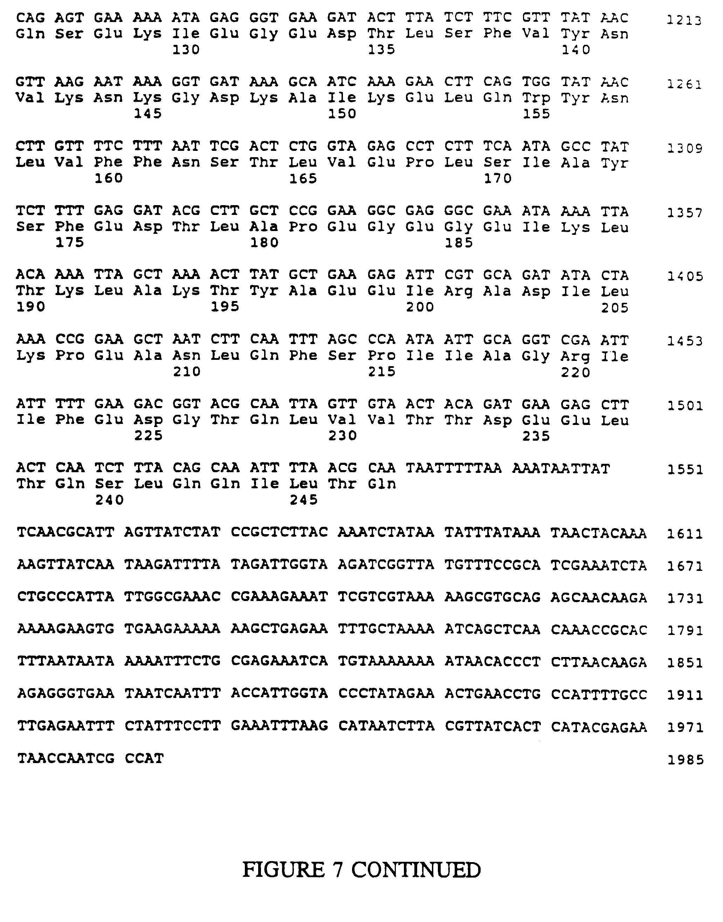

The term "LppA" or a nucleotide sequence encoding the same, intends a protein or a nucleotide sequence, respectively, derived from a contiguous sequence falling within positions 1 through 247, inclusive, of Figure 7.

The term "LppB" or a nucleotide sequence encoding the same, intends a protein or a nucleotide sequence, respectively, derived from a contiguous sequence falling within positions 1 through 279, inclusive, of Figure 9.

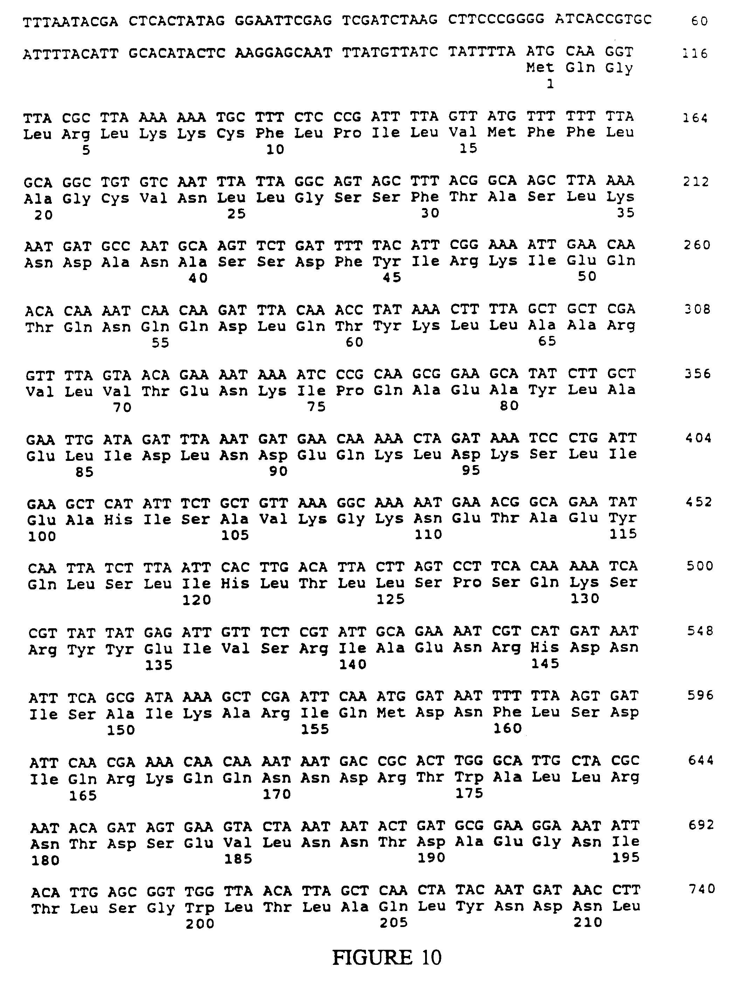

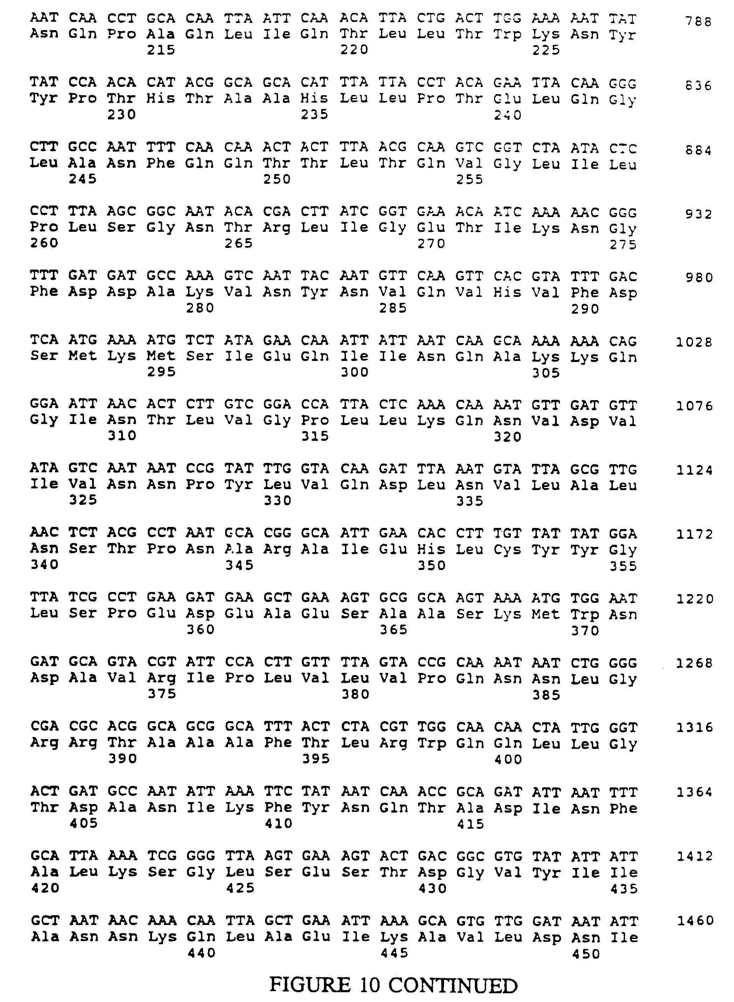

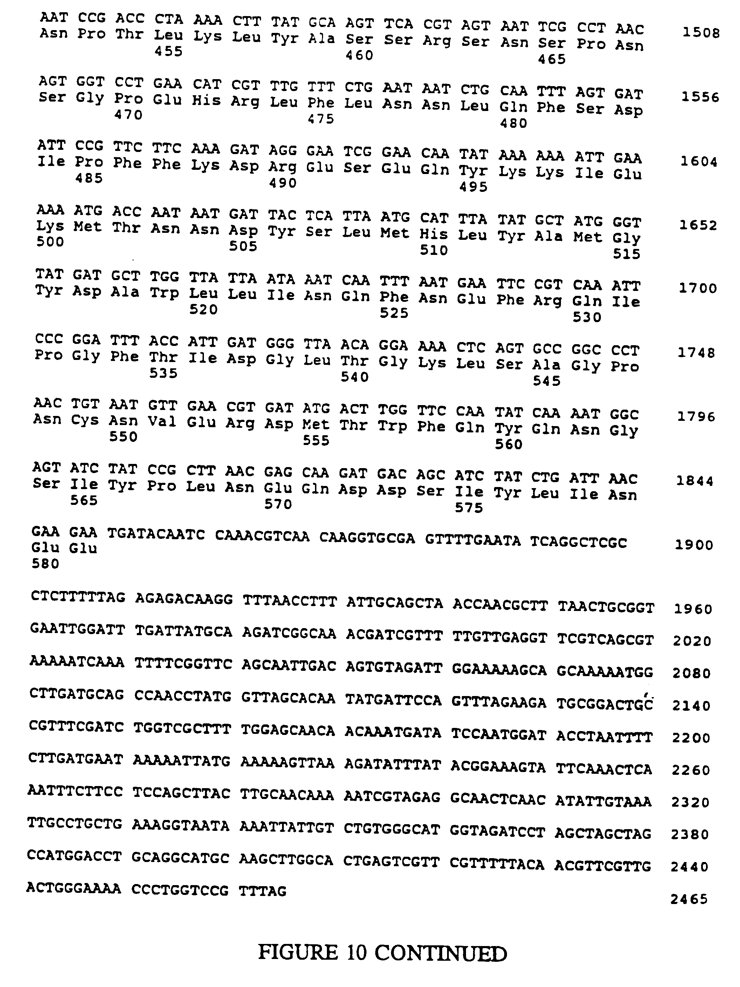

The term "LppC" or a nucleotide sequence encoding the same, intends a protein or a nucleotide sequence, respectively, derived from a contiguous sequence falling within positions 1 through 581, inclusive, of Figure 10.

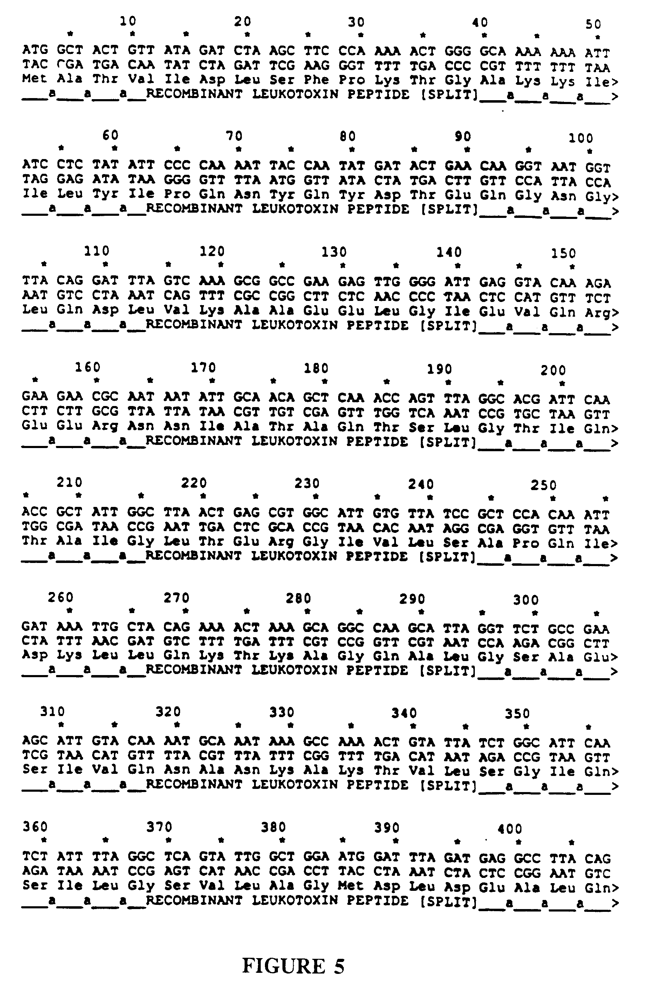

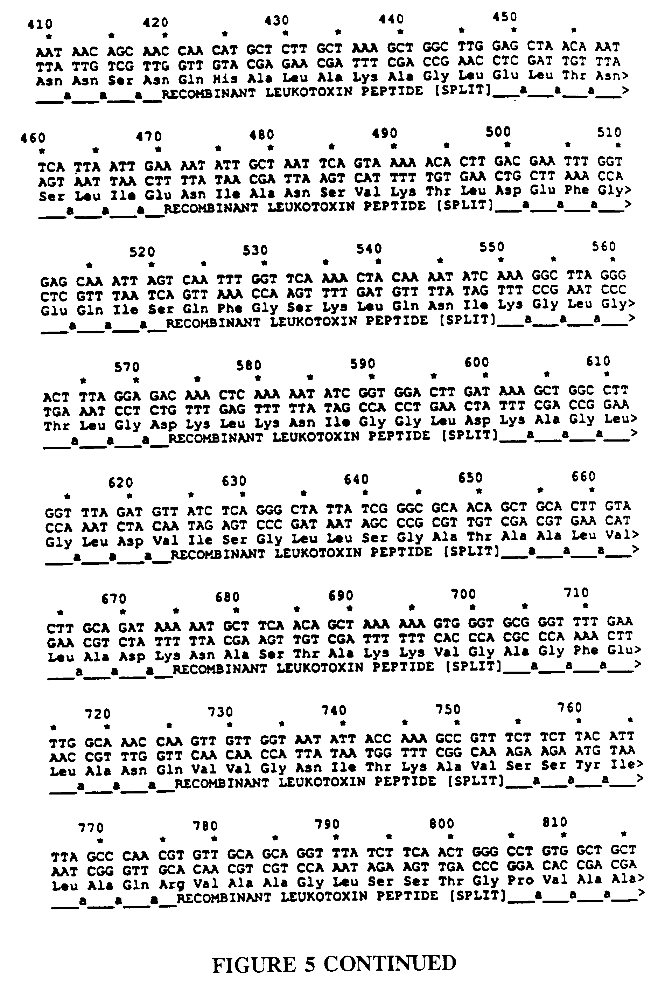

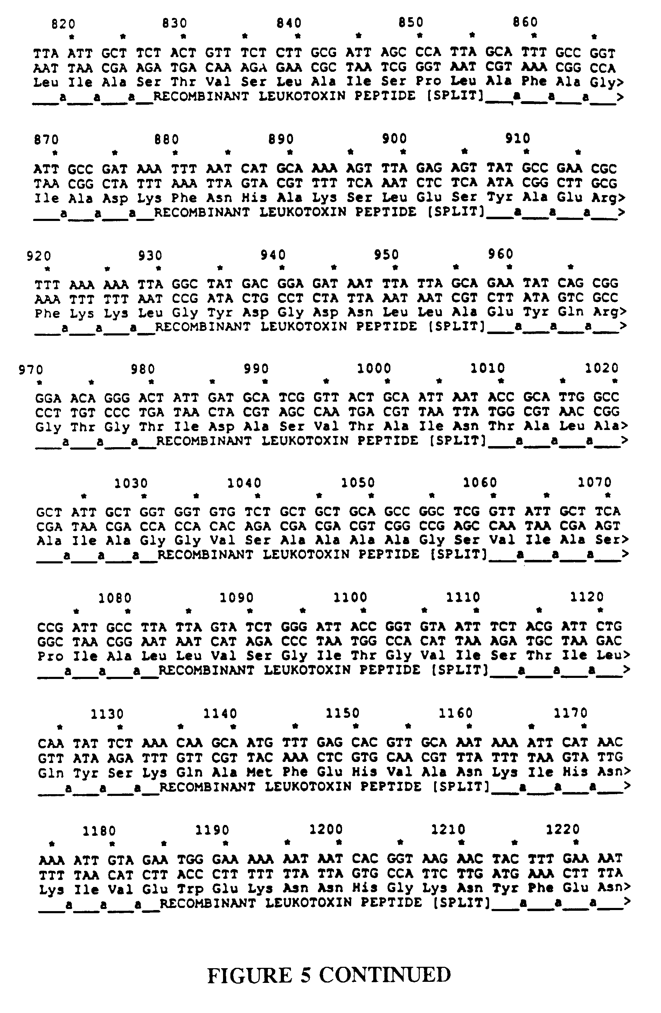

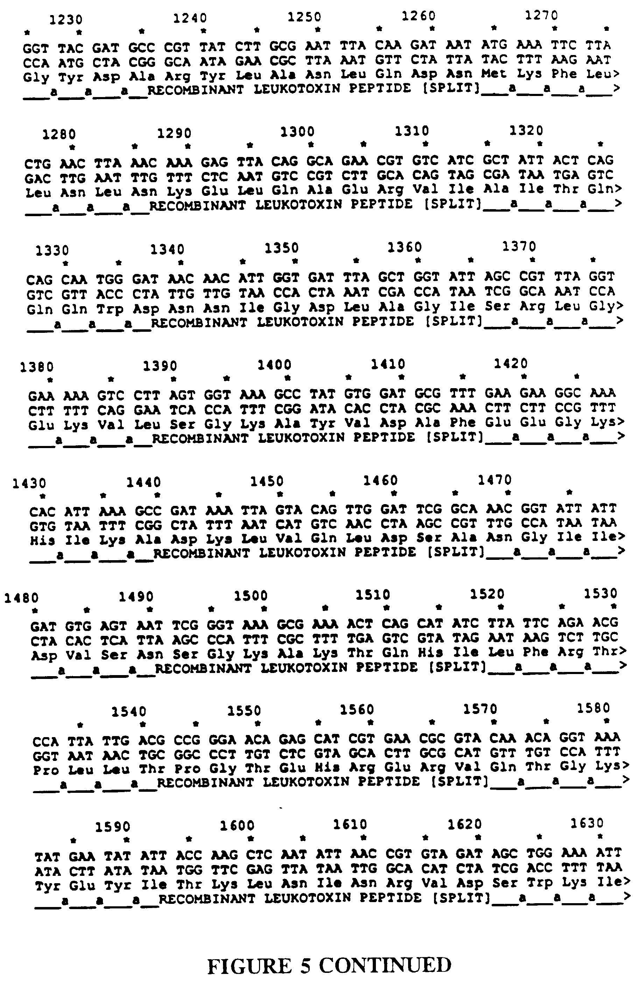

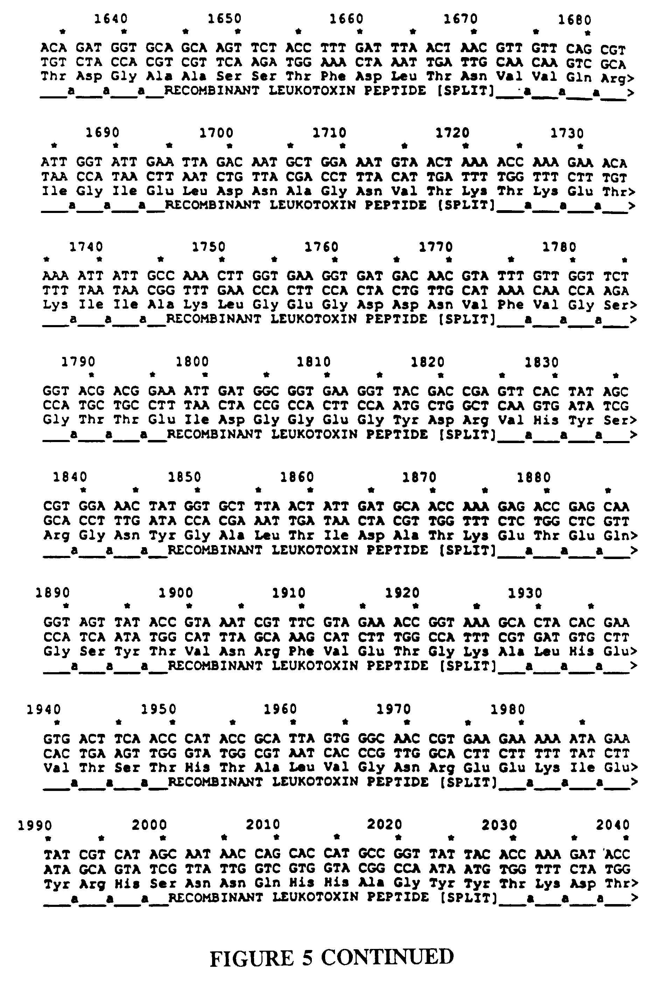

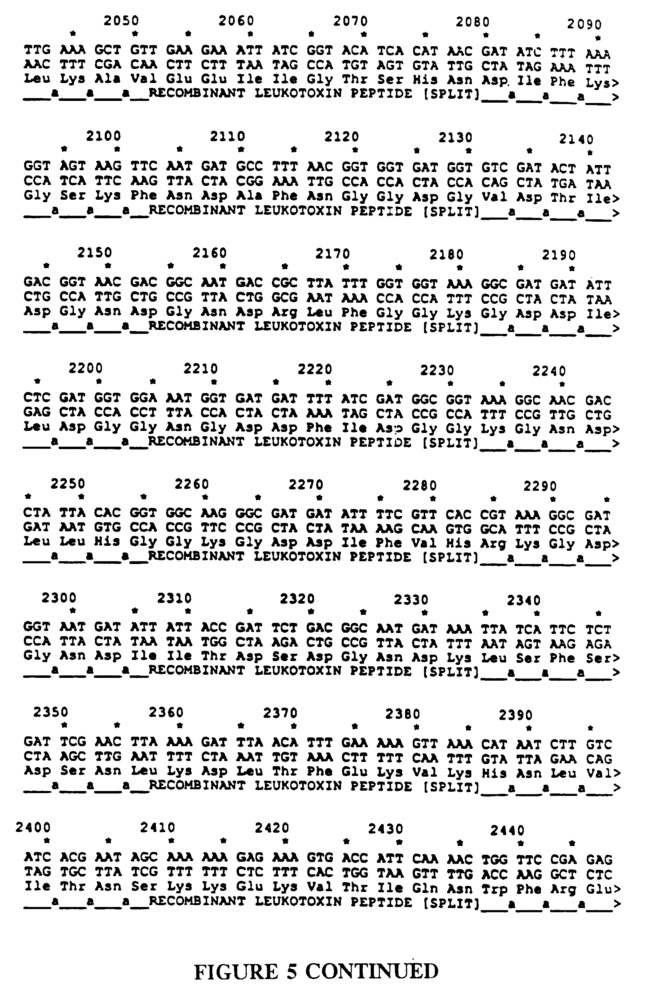

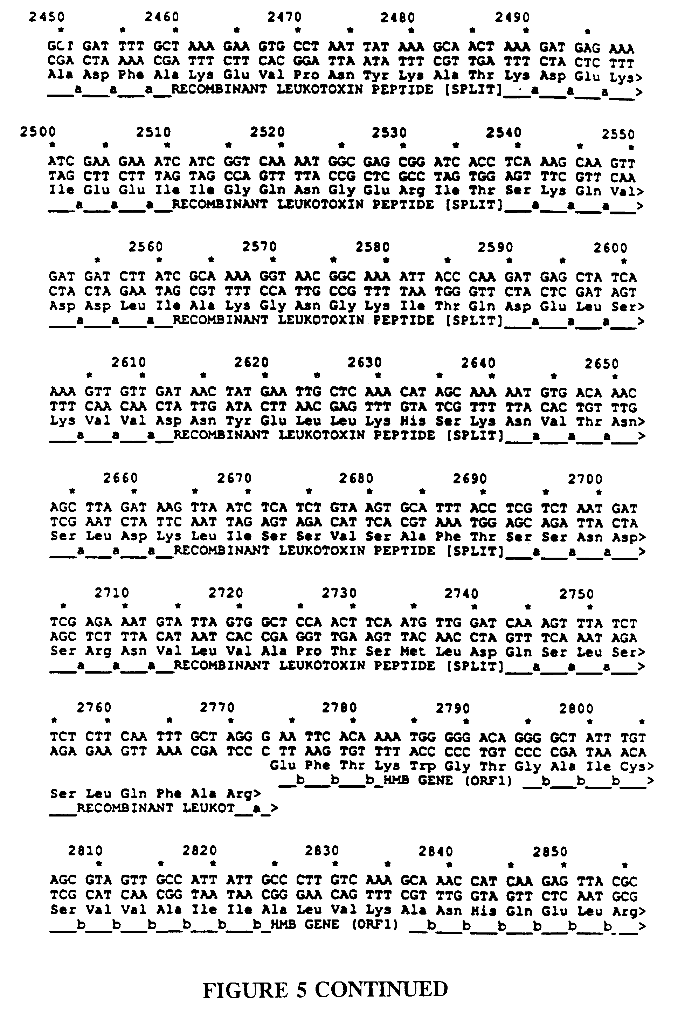

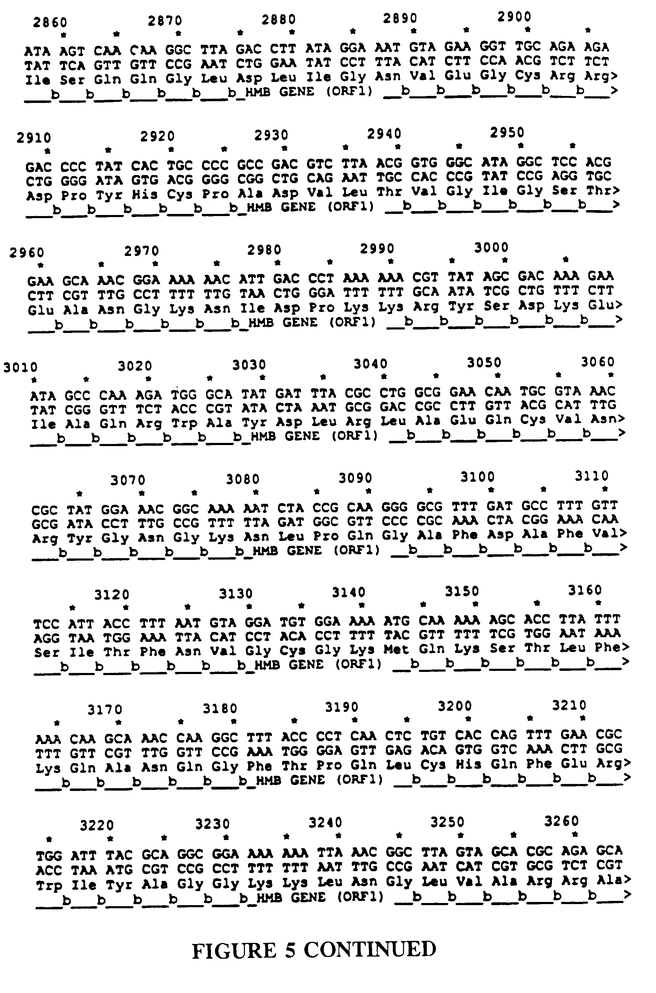

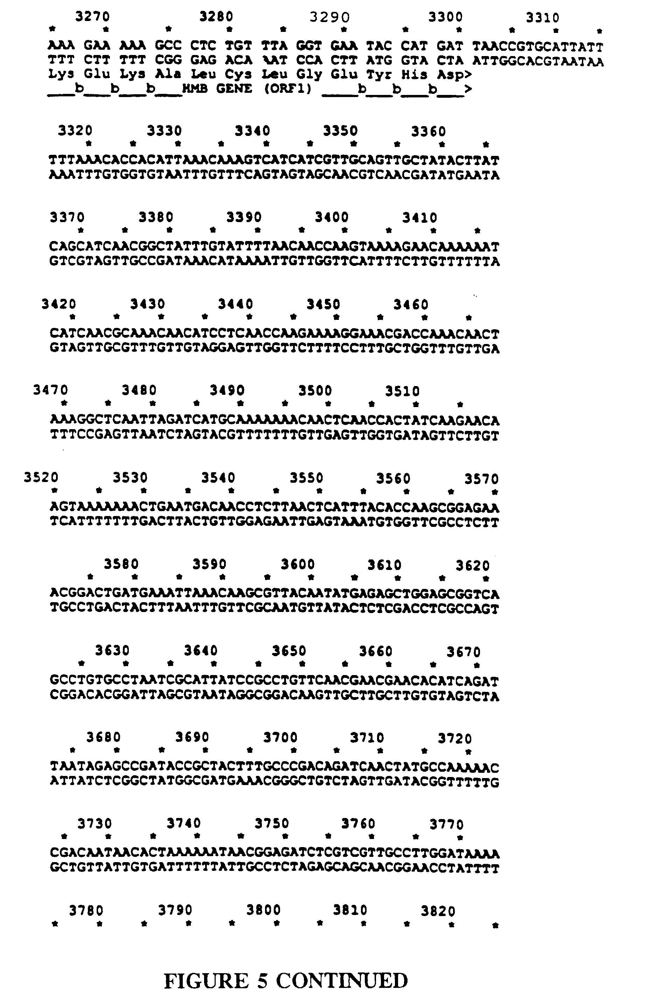

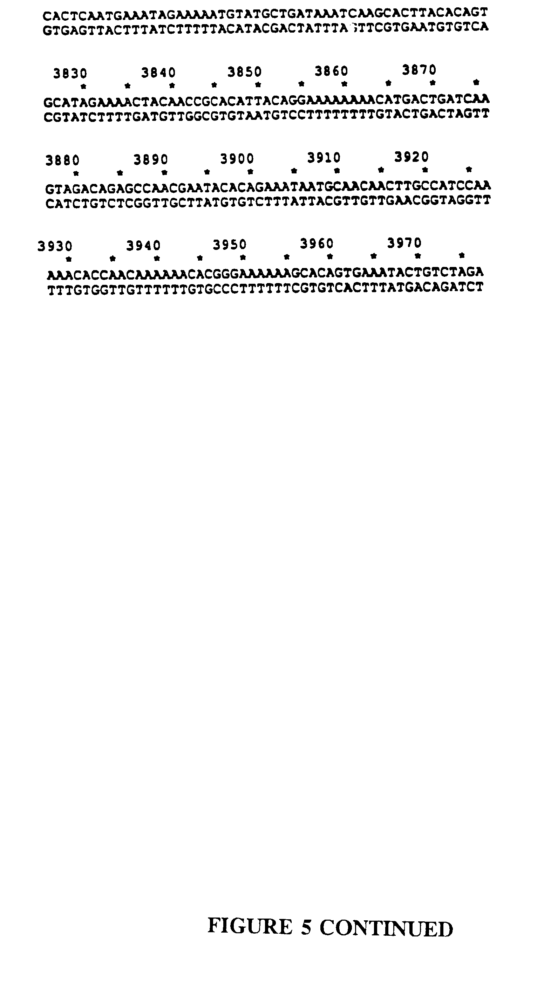

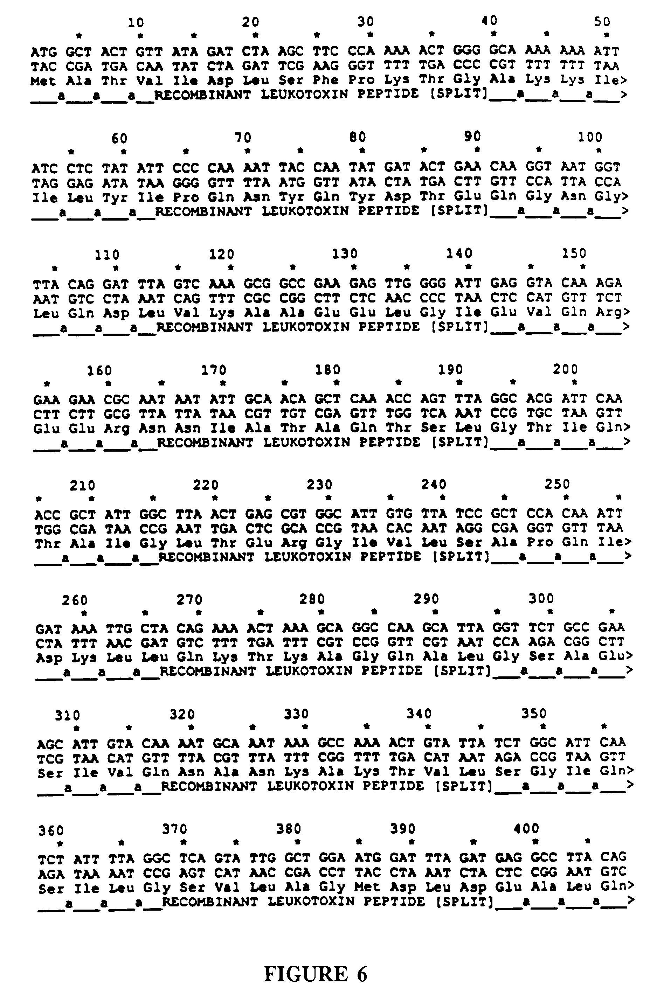

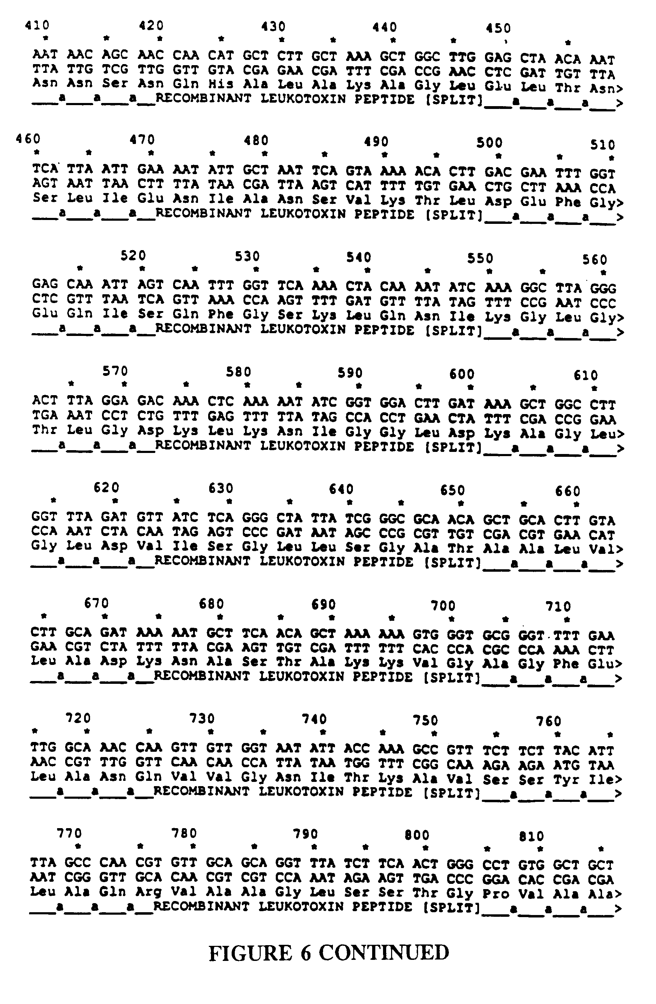

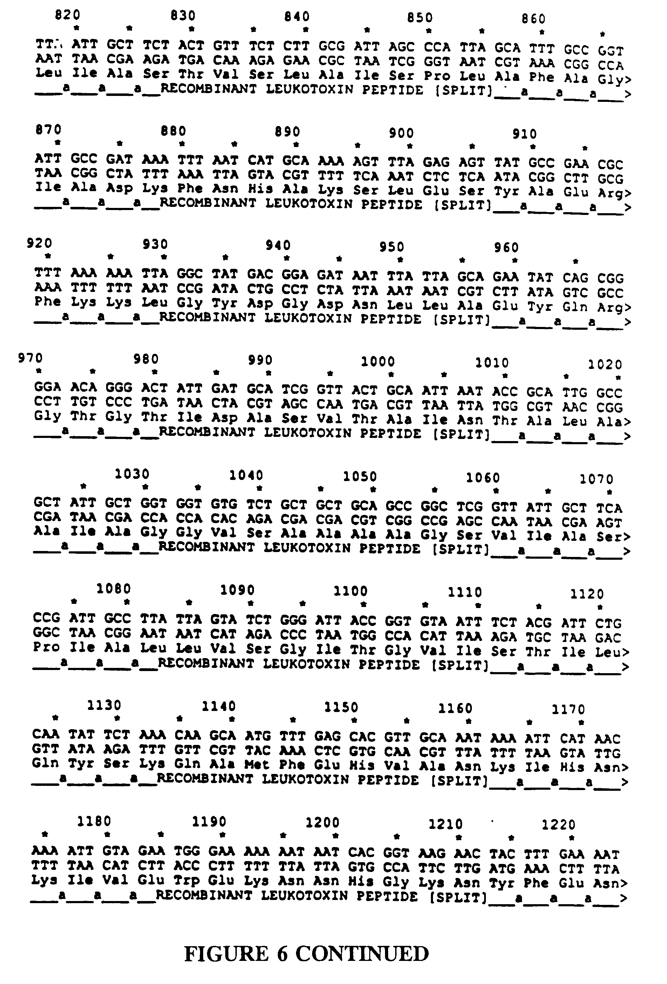

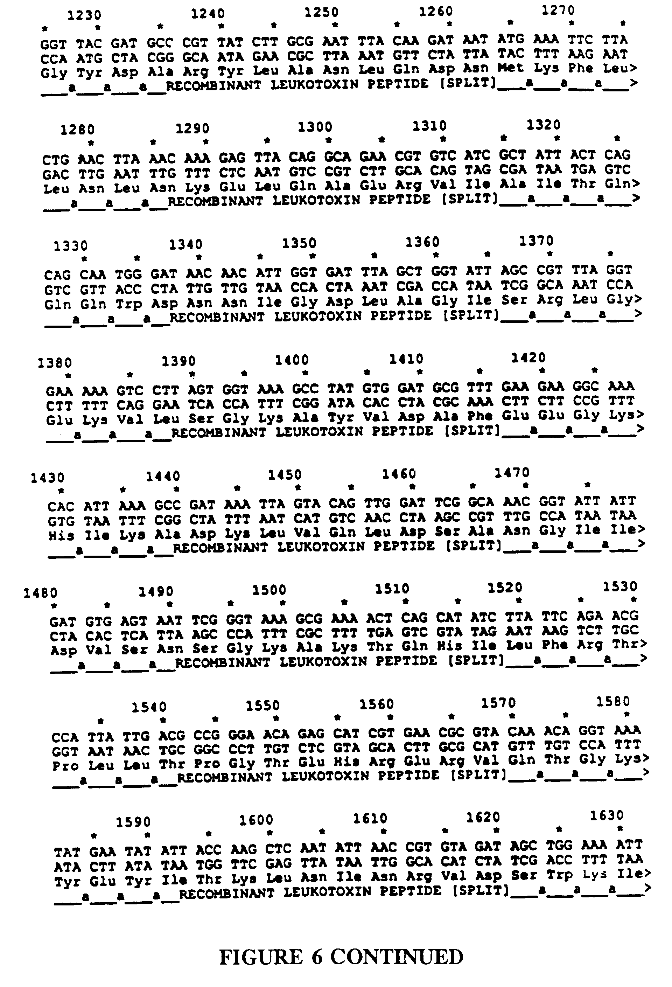

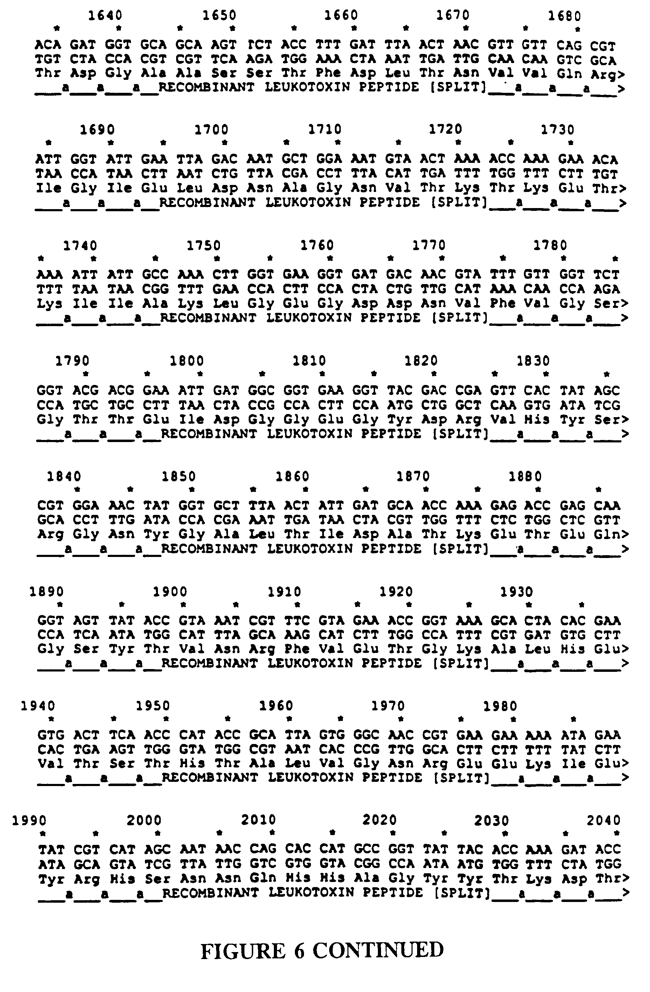

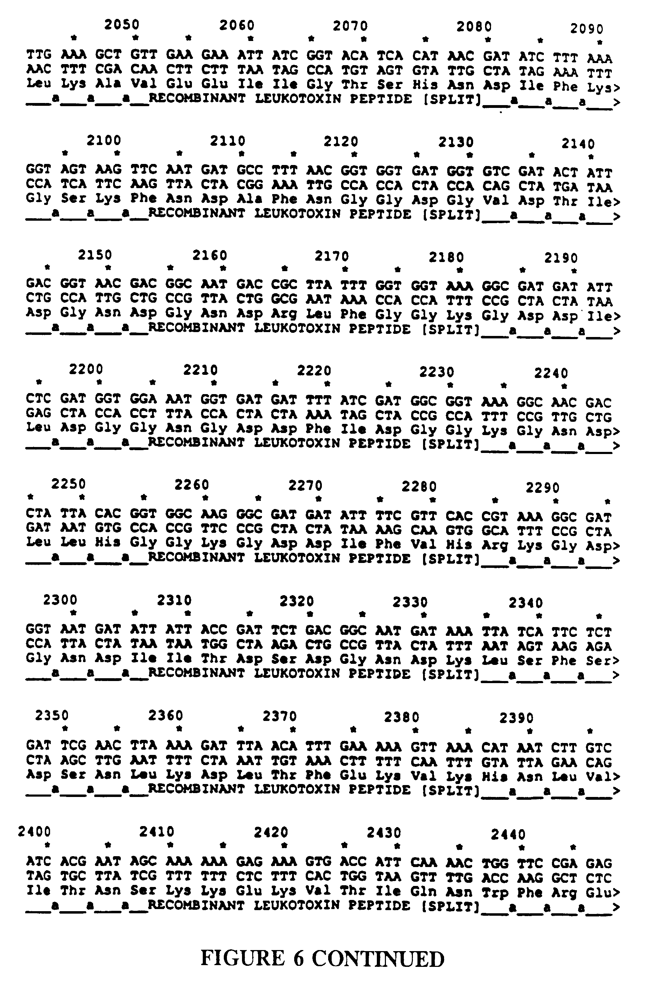

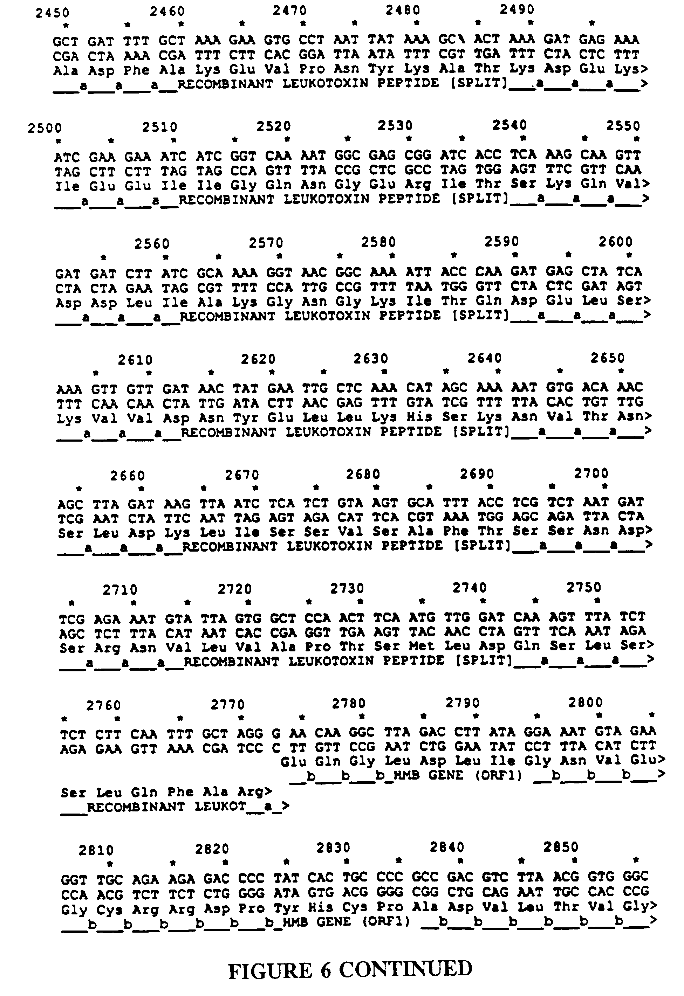

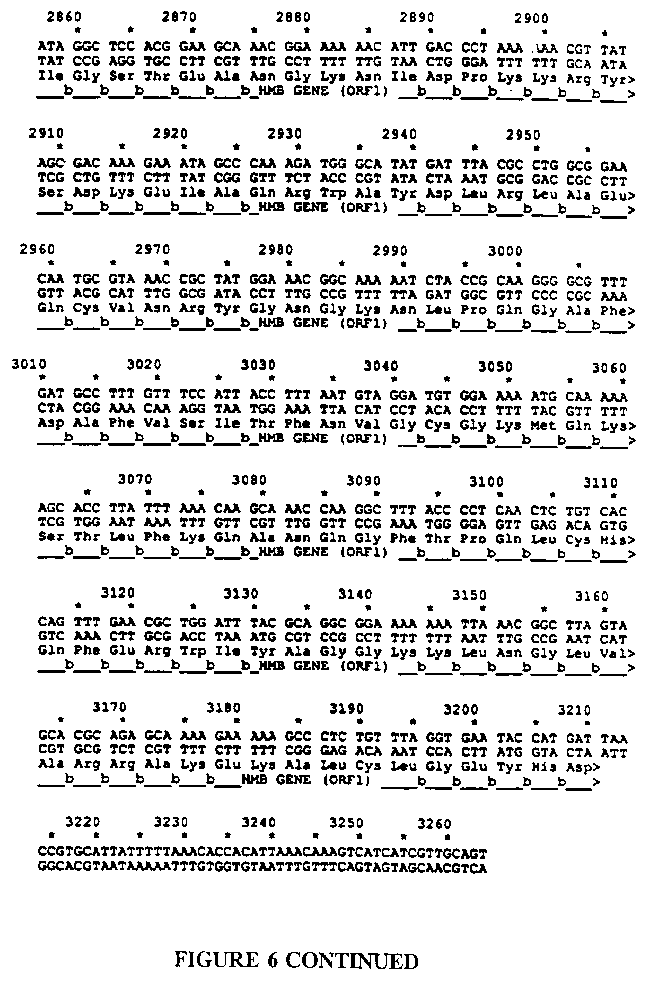

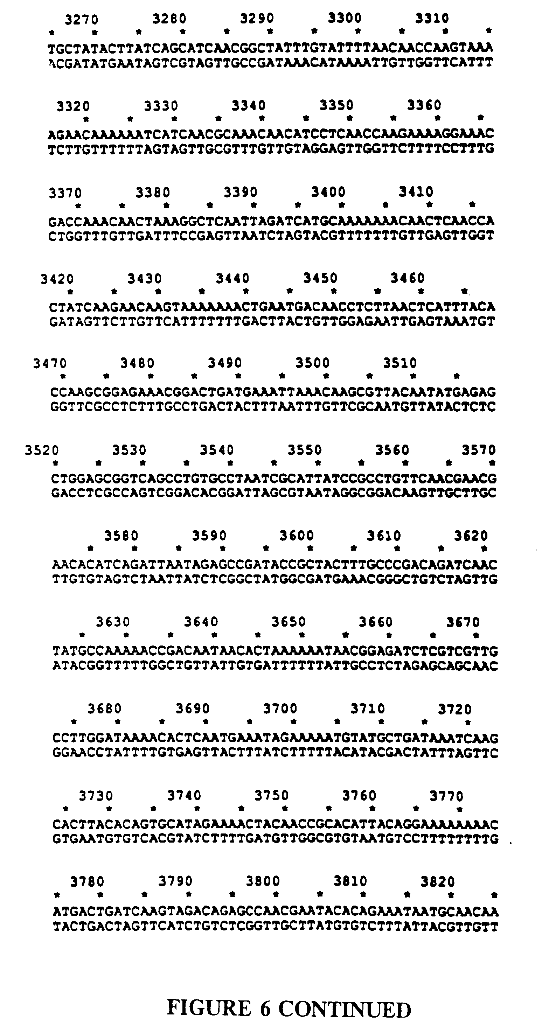

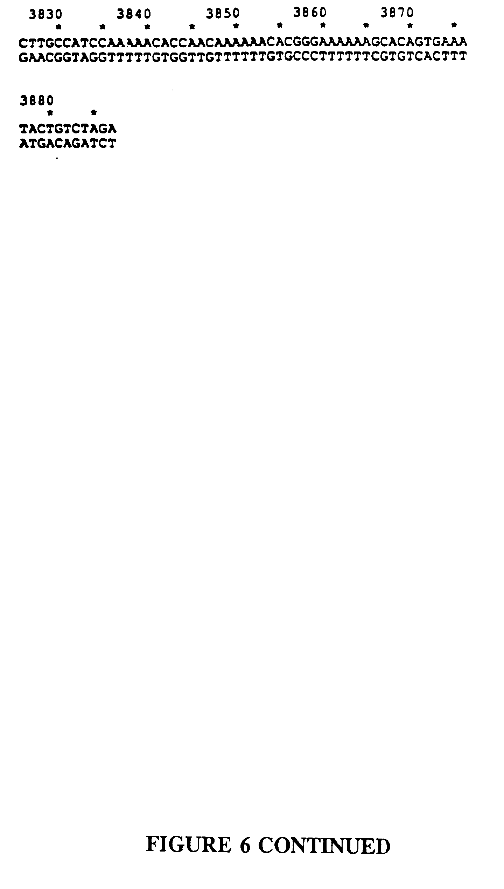

The derived protein or nucleotide sequences need not be physically derived from the genes described above, but may be generated in any manner, including for example, chemical synthesis, isolation (either from *H. somnus* or any other organism expressing the proteins) or by recombinant production, based on the information provided herein. Furthermore, the terms intend proteins having amino acid sequences substantially homologous to contiguous amino acid sequences encoded by the genes. Thus, the terms include both full-length, truncated and partial sequences, as well as analogs and precursor forms of the proteins. Representative truncated sequences derived from the *hmb* gene are present as fusions with a truncated *P. haemolytica* leukotoxin gene in plasmids pGCH5 and pGCH4 and are shown in Figures 5 and 6. Precursor forms of several of the proteins are described further below. The terms also include proteins in neutral form or in the form of basic or acid addition salts depending on the mode of preparation. Such acid addition salts may involve free amino groups and basic salts may be formed with free carboxyls. Pharmaceutically acceptable basic and acid addition salts are discussed further below. In addition, the proteins may be modified by combination with other biological materials such as lipids (both those occurring naturally with the molecule or other lipids that do not destroy activity) and saccharides, or by side chain modification, such as acetylation of amino groups, phosphorylation of hydroxyl side chains, oxidation of sulfhydryl groups, glycosylation of amino acid residues, as well as other modifications of the encoded primary sequence. A protein derived from the *hmb* gene or the *hly* gene need not necessarily display haemin-binding or haemolytic activity, respectively.

An "isolated" protein sequence is a protein sequence which is separate and discrete from a whole organism (live or killed) with which the protein is normally associated in nature. Thus, a protein contained in a cell free extract would constitute an "isolated" protein, as would a protein synthetically or recombinantly produced. An "isolated" nucleotide sequence is a nucleotide sequence separate and discrete from the whole organism with which the sequence is found in nature; or a sequence devoid, in whole or part, of sequences normally associated with it in nature; or a sequence, as it exists in nature, but having heterologous sequences (as defined below) in association therewith.

The term "epitope" refers to the site on an antigen or hapten to which specific B cells and T cells respond. The term is also used interchangeably with "antigenic determinant" or "antigenic determinant site."

An "immunological response" to a composition or vaccine is the development in the host of a cellular and/or antibody-mediated immune response to the composition or vaccine of interest. Usually, such a response includes but is not limited to one or more of the following effects; the production of antibodies, B cells, helper T cells, suppressor T cells, and/or cytotoxic T cells and/or γδ T cells, directed specifically to an antigen or antigens included in the composition or vaccine of interest.

The terms "immunogenic" protein or polypeptide refer to an amino acid sequence which elicits an immunological response as described above. An "immunogenic" protein or polypeptide, as used herein, includes the full-length sequence of the *H. somnus* protein in question, analogs thereof, or immunogenic fragments thereof. By "immunogenic fragment" is meant a fragment of a polypeptide which includes one or more epitopes and thus elicits the immunological response described above. Such fragments can be identified by, *e.g*., concurrently synthesizing large numbers of peptides on solid supports, the peptides corresponding to portions of the protein molecule, and reacting the peptides with antibodies while the peptides are still attached to the supports. Such techniques are known in the art and described in, *e.g*., U.S. Patent No. 4,708,871; Geysen, H.M. *et al.* (1984) *Proc. Natl. Acad. Sci. USA* 81:3998-4002; Geysen, H.M. *et al.* (1986) *Molec. Immunol*. 23:709-715, all incorporated herein by reference in their entireties. Studies with some bacterial lipoproteins have shown that the portion of the molecule responsible for biological activity resides in the N-terminal fatty acid containing region. Short peptides, including two to five amino acids covalently linked to palmitate, have been shown to possess biological activity (Bessler, W.G., *et al. J. Immunol.* (1985) 135:1900-1905). Accordingly, immunogenic fragments, for purposes of the present invention, will usually be at least about 2 amino acids in length, more preferably about 5 amino acids in length, and most preferably at least about 10 to 15 amino acids in length. There is no critical upper limit to the length of the fragment, which could comprise nearly the full-length of the protein sequence, or even a fusion protein comprising two or more epitopes of the *H. somnus* proteins.

The terms "polypeptide" and "protein" are used interchangeably and in their broadest sense, *i.e.*., any polymer of amino acids (dipeptide or greater) linked through peptide bonds. Thus, the term "polypeptide" includes proteins (having both the full-length sequence or fragments thereof), oligopeptides, analogs, muteins, fusion proteins and the like.

"Recombinant" polypeptides refer to polypeptides produced by recombinant DNA techniques; *i.e.,* produced from cells transformed by an exogenous DNA construct encoding the desired polypeptide. "Synthetic" polypeptides are those prepared by chemical synthesis.

A "replicon" is any genetic element (*e.g*., plasmid, chromosome, virus) that functions as an autonomous unit of DNA replication *in vitro* or *in vivo; i.e.*., capable of replication under its own control.

A "vector" is a replicon, such as a plasmid, phage, or cosmid, to which another DNA segment may be attached so as to bring about the replication of the attached segment.

A DNA "coding sequence" or a "nucleotide sequence encoding" a particular protein, is a DNA sequence which is transcribed and translated into a polypeptide *in vitro* or *in vivo* when placed under the control of appropriate regulatory sequences. The boundaries of the coding sequence are determined by a start codon at the 5' (amino) terminus and a translation stop codon at the 3' (carboxy) terminus. A coding sequence can include, but is not limited to, procaryotic sequences, cDNA from eucaryotic mRNA, genomic DNA sequences from eucaryotic (*e.g.*, mammalian) DNA, and even synthetic DNA sequences. A transcription termination sequence will usually be located 3' to the coding sequence.

DNA "control sequences" refers collectively to promoter sequences, ribosome binding sites, polyadenylation signals, transcription termination sequences, upstream regulatory domains, enhancers, and the like, which collectively provide for the transcription and translation of a coding sequence in a host cell. Not all of these control sequences need always be present in a recombinant vector so long as the desired gene is capable of being transcribed and translated.

"Operably linked" refers to an arrangement of elements wherein the components so described are configured so as to perform their usual function. Thus, control sequences operably linked to a coding sequence are capable of effecting the expression of the coding sequence. The control sequences need not be contiguous with the coding sequence, so long as they function to direct the expression thereof. Thus, for example, intervening untranslated yet transcribed sequences can be present between a promoter sequence and the coding sequence and the promoter sequence can still be considered "operably linked" to the coding sequence. Similarly, a coding sequence is "operably linked to" another coding sequence (i.e., in the case of a chimeric protein) when RNA polymerase will transcribe the two coding sequences into mRNA, which is then translated into the polypeptides encoded by the two coding sequences. The coding sequences need not be contiguous to one another so long as the transcribed sequence is ultimately processed to produce the desired protein.

A control sequence "directs the transcription" of a coding sequence in a cell when RNA polymerase will bind the promoter sequence and transcribe the coding sequence into mRNA, which is then translated into the polypeptide encoded by the coding sequence.

A "host cell" is a cell which has been transformed, or is capable of transformation, by an exogenous DNA sequence.

A cell has been "transformed" by exogenous DNA when such exogenous DNA has been introduced inside the cell membrane. Exogenous DNA may or may not be integrated (covalently linked) into chromosomal DNA making up the genome of the cell. In procaryotes and yeasts, for example, the exogenous DNA may be maintained on an episomal element, such as a plasmid. With respect to eucaryotic cells, a stably transformed cell is one in which the exogenous DNA has become integrated into the chromosome so that it is inherited by daughter cells through chromosome replication. This stability is demonstrated by the ability of the eucaryotic cell to establish cell lines or clones comprised of a population of daughter cell containing the exogenous DNA.

Two DNA or polypeptide sequences are "substantially homologous" when at least about 80% (preferably at least about 90%, and most preferably at least about 95%) of the nucleotides or amino acids match over a defined length of the molecule. For the purposes of the present invention, at least 90 % homology is required between two polypeptides for them to be considered "substantially homologous" to one another. As used herein, substantially homologous also refers to sequences showing identity to the specified DNA or polypeptide sequence. DNA sequences that are substantially homologous can be identified in a Southern hybridization experiment under, for example, stringent conditions, as defined for that particular system. Defining appropriate hybridization conditions is within the skill of the art. See, e.g., Sambrook et al., supra; DNA Cloning, vols I & II, supra; Nucleic Acid Hybridization, supa.

The term "functionally equivalent" intends that the amino acid sequence of the subject peptide is one that will elicit an immunological response, as defined above, equivalent to the response elicited by an *H. somnus* haemolysin, haemin-binding protein, LppA, LppB or LppC antigenic peptide having identity with either the entire coding sequence for the various native proteins, or an immunogenic portion thereof.

A "heterologous" region of a DNA construct is an identifiable segment of DNA within or attached to another DNA molecule that is not found in association with the other molecule in nature. Thus, when the heterologous region encodes a bacterial gene, the gene will usually be flanked by DNA that does not flank the bacterial gene in the genome of the source bacteria. Another example of the heterologous coding sequence is a construct where the coding sequence itself is not found in nature (*e.g.*, synthetic sequences having codons different from the native gene). Allelic variation or naturally occurring mutational events do not give rise to a heterologous region of DNA, as used herein.

The term "treatment" as used herein refers to either (i) the prevention of infection or reinfection (prophylaxis), or (ii) the reduction or elimination of symptoms of the disease of interest (therapy). Hence, the vaccines and pharmaceutical compositions according to the invention may be used for prophylaxis or therapy.

By "vertebrate subject" is meant any member of the subphylum chordata, including, without limitation, mammals such as cattle, sheep, pigs, goats, horses, and man; domestic animals such as dogs and cats; and birds, including domestic, wild and game birds such as cocks and hens including chickens, turkeys and other gallinaceous birds. The term does not denote a particular age. Thus, both adult and newborn animals are intended to be covered.

### B. General Methods

Central to the present invention is the discovery of several unique, immunogenic, outer membrane *H. somnus* proteins and in particular the LppB proteins. The genes for these proteins (termed *"hmb," "hly," "lpp*A*," "lpp*B*"* and "*lpp*C" herein) have been isolated and characterized. The *hmb* and various *lpp* genes have been sequenced. The protein products from the *hmb* and *hly* genes bind haemin and display haemolytic activity, respectively, in assays described below.

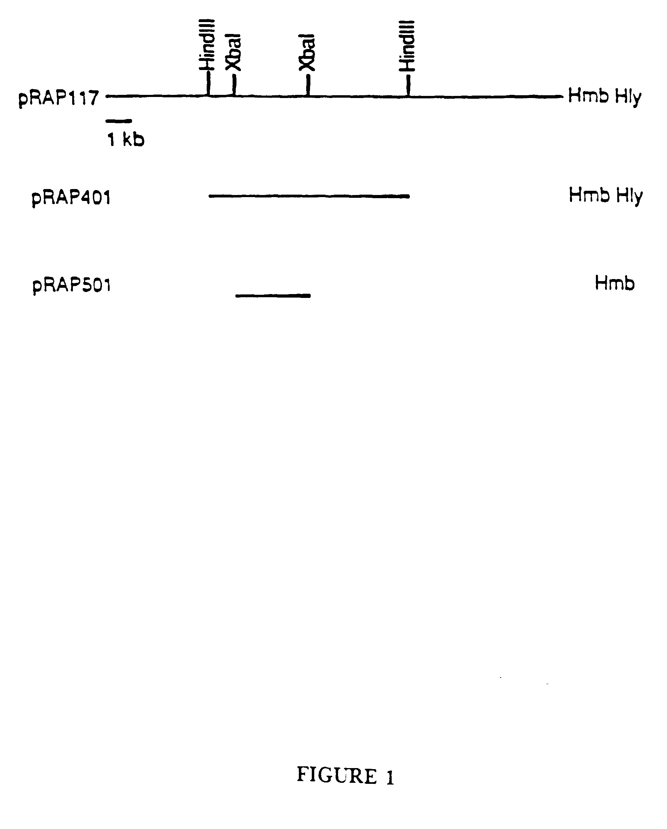

As shown in Figure 1, the *hmb* gene is located on a 3 kb XbaI fragment derived from plasmid pRAP117 (ATCC Accession No. 68952). Western blot analysis of a clone including this fragment detects a protein with an apparent molecular mass of 50 kDa that comigrates with an iron-regulated *H. somnus* protein. The *hly* gene is present in plasmid pAA504 (ATCC Accession No. 68953), as confirmed by probing this plasmid with an 8 kb HindIII fragment from plasmid pRAP117. The *hly* gene is located on the distal end of this fragment (see Figure 1).

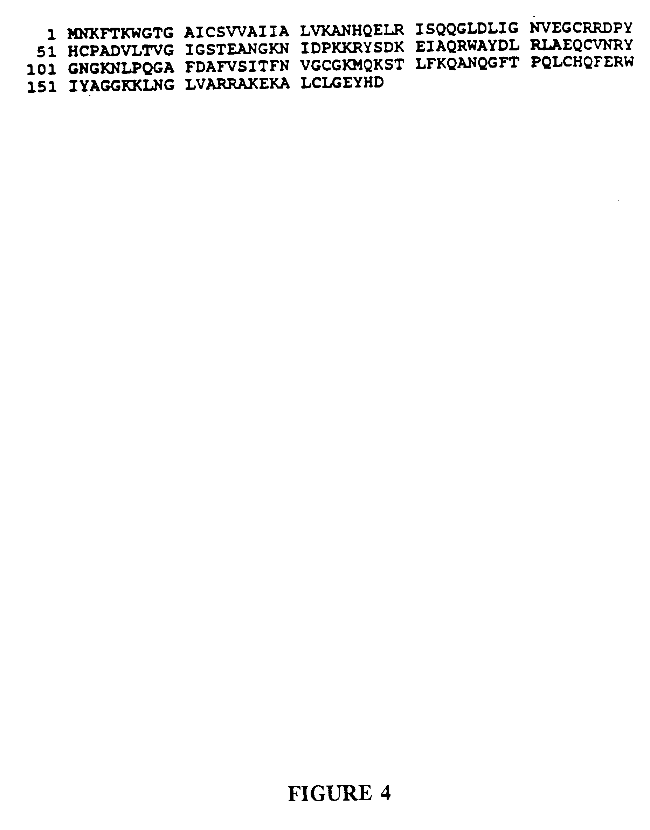

The *hmb* gene is shown as ORF1 in Figure 2. The gene encodes a haemin-binding protein having 178 amino acids. The deduced amino acid sequence for the *H. somnus* haemin-binding protein is shown in Figure 4.

LppA appears to correspond to the major *H. somnus* 40 kDa OMP. The gene encoding LppA, *lpp*A*,* has been cloned and the nucleotide sequence determined. LppA is specified by a single transcript approximately 1300 nucleotides in length. The start point is located at position 757 of Figure 7, suggesting that transcription terminates beyond the 3'-end of the cloned DNA. One open reading frame (ORF) is present, starting at an ATG codon at position 791 and running through position 1531 of Figure 7 (amino acid residues 1 through 247). This region appears to encode the preprotein. The calculated molecular weight based on the sequence is 27,072. This reading frame has been confirmed by sequencing the fusion joint of two independent *lpp*A*::*Tn*pho*A gene fusions. Thus, although the predicted molecular weight is less than expected, the ORF indeed encodes the LppA protein. The anomalous molecular weight is likely due to the lipid nature of the molecule. The region downstream of the *lpp*A gene does not contain ORFs of any significant length. Also, the LppA protein is the only polypeptide specified by the *H. somnus* insert in *E. coli* minicells. Therefore, it is likely that *lpp*A is transcribed as a single cistron.

No significant homology between the complete LppA amino acid sequence and sequences compiled in Genbank have been found.

LppA appears to include a signal sequence. The 21 N-terminal amino acids show strong sequence homology to the signal peptide of other secreted proteins, and the sequence, Leu-Leu-Ala-Ala-Cys, at the putative cleavage site, is identical to the consensus cleavage sequence of lipoproteins from Gram-negative bacteria. Thus the mature protein spans positions 854 through 1531 (amino acid residues 22 through 247), inclusive, of Figure 7. The ORF thus encodes a preprotein having 247 amino acid residues and a mature polypeptide having 226 amino acid residues.

The presence of the lipid moiety on the protein was shown by incorporation of radioactive palmitic acid into the natural *H. somnus* protein. Palmitic acid was also incorporated into the protein when it was recombinantly produced in *E. coli.* Synthesis of the mature LppA lipoprotein was inhibited by globomycin, showing that cleavage of the signal peptide is mediated by signal peptidase II in both organisms. Using site-directed mutagenesis, the Cys residue at the cleavage site was changed to glycine. Radiolabeled palmitate was not incorporated into the mutated protein, showing that lipid modification occurs at the Cys-22 residue.

Lipoprotein, LppB, has been cloned and studied. The gene, *lpp*B*,* also encodes a 40 kDa *H. somnus* outer membrane lipoprotein. This lipoprotein is antigenically distinct from LppA and plasmids harboring the *lpp*B gene do not hybridize to plasmids encoding LppA. Lipid moieties on the molecule were detected as described above. Figure 9 depicts a chromosomal fragment which includes *lpp*B*.* The ORF encoding LppB begins at position 872 and ends with the TAA codon at position 1709. A putative ribosome binding site, GGAG, is located upstream and a seven base pair A/T rich spacer precedes the ATG start codon. The *lpp*B gene encodes a preprotein having 279 amino acids. The first 16 amino acids of LppB appear to specify a signal sequence. Amino acid residues 1 to 13 are followed by a lipoprotein box, Leu-Ala-Ala-Cys. This region strongly resembles signal peptides of other procaryotic lipoproteins, including LppA described above. The mature lipoprotein spans positions 920 through 1708 (amino acid residues 17 through 279) of Figure 9. The calculated molecular mass of LppB is 31307 Daltons. Again, the discrepancy in size is probably due to the lipid nature of the protein.

LppB binds both Congo red and hemin on agar plates. LppA, on the other hand, binds neither of these proteins. It is known that some pathogenic bacteria can adsorb the aromatic dye Congo red and that this ability is strongly correlated with virulence (Daskaleros & Payne *Infect. Immun.* (1985) 48:165-168; Maurelli *et al. Infect. Immun.* (1984) 43:397-401). The molecular basis for this adsorption is unclear, although in *E. coli* and *S. flexneri,* Congo red binding has been associated with the presence of a large virulence plasmid (Maurelli *et al.* 1984). It has also been suggested that the ability of certain species to bind Congo red is related to their ability to sequester iron and that Congo red binding and hemin adsorption is correlated (Prpic *et al.* 1983). The ability of LppB to bind Congo red and hemin can be used as a selection technique in recombinant production.

The gene encoding a third *H. somnus* lipoprotein, LppC, has also been cloned. LppC is a 60 kDa lipoprotein, as determined by gel electrophoresis. The nucleotide sequence and predicted amino acid sequence of LppC is shown in Figure 10. An ORF beginning at position 108 and ending at position 1850 codes for a protein with a calculated molecular weight of 63,336 Daltons. As with LppA and LppB, the preprotein includes a typical procaryotic signal sequence. The signal sequence includes the first 21 amino acids and thus the DNA coding for the mature protein begins at nucleotide position 171. The lipid nature of this protein was confirmed as with LppA and LppB. Like LppB, LppC is able to bind both Congo red and hemin.

As explained above, the LppA, LppB and LppC proteins are normally found in association with lipid moieties. It is likely that the fatty acid moiety present is a palmitic acid derivative. The antigens of the present invention, even though carrying epitopes derived from the LppB lipoprotein, do not require the presence of the lipid moiety. Furthermore, if the lipid is present, it need not be a lipid commonly associated with the lipoprotein, so long as the appropriate immunologic response is elicited. In any event, suitable fatty acids, such as but not limited to, palmitic acid or palmitic acid analogs, can be conveniently added to the desired amino acid sequence during synthesis, using standard techniques. For example, palmitoyl bound to S-glyceryl-L-Cys (Pam₃-Cys) is commercially available (e.g. through Boehringer Mannheim, Dorval, Quebec) and can easily be incorporated into an amino acid sequence during synthesis. See, e.g. Deres, K., *et al. Nature* (1989) 342:561. This is a particularly convenient method for production when relatively short amino acid sequences are used. Similarly, recombinant systems can be used which will process the expressed proteins by adding suitable fatty acids. Representative systems for recombinant production are discussed further below.

An *H. somnus* LppB protein, analogues thereof, immunogenic fragments thereof or chimeric proteins including the same, can be provided in subunit vaccine compositions and thus problems inherent in prior vaccine compositions, such as localized and systemic side reactions, as well as immunosuppressive effects, are avoided. In addition to use in vaccine compositions, the proteins or antibodies thereto can be used as diagnostic reagents to detect the presence of *H. somnus* infection in a subject. Similarly, the gene encoding the protein can be cloned and used to design probes for the detection of *H. somnus* in tissue samples as well as for the detection of homologous genes in other bacterial strains.

It will sometimes be preferable to have more than one epitope of one or more of the proteins in the vaccine compositions of the present invention. In its simplest form, this can be achieved by employing a polypeptide comprising the full-length sequence of the LppB protein (encompassing more than one epitope), or by employing a combination of polypeptides comprising the sequences of two or more of the described proteins. Thus, the vaccine compositions could comprise, for example various combinations such as one of the LppB proteins and one or more of the other H. somnus proteins, or a combination of all of the described proteins.

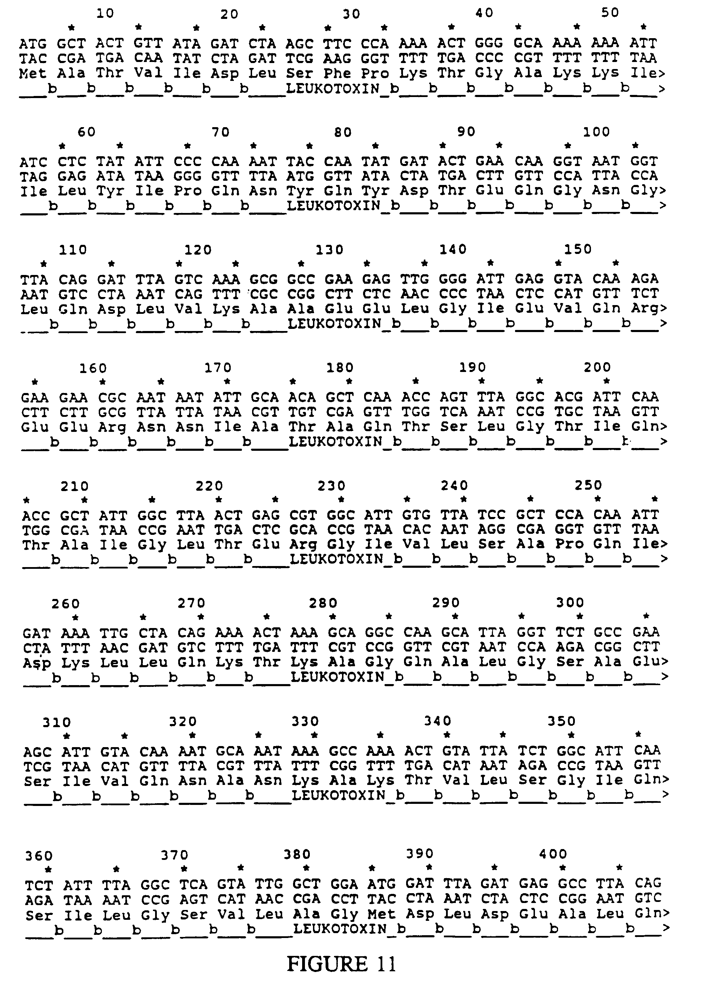

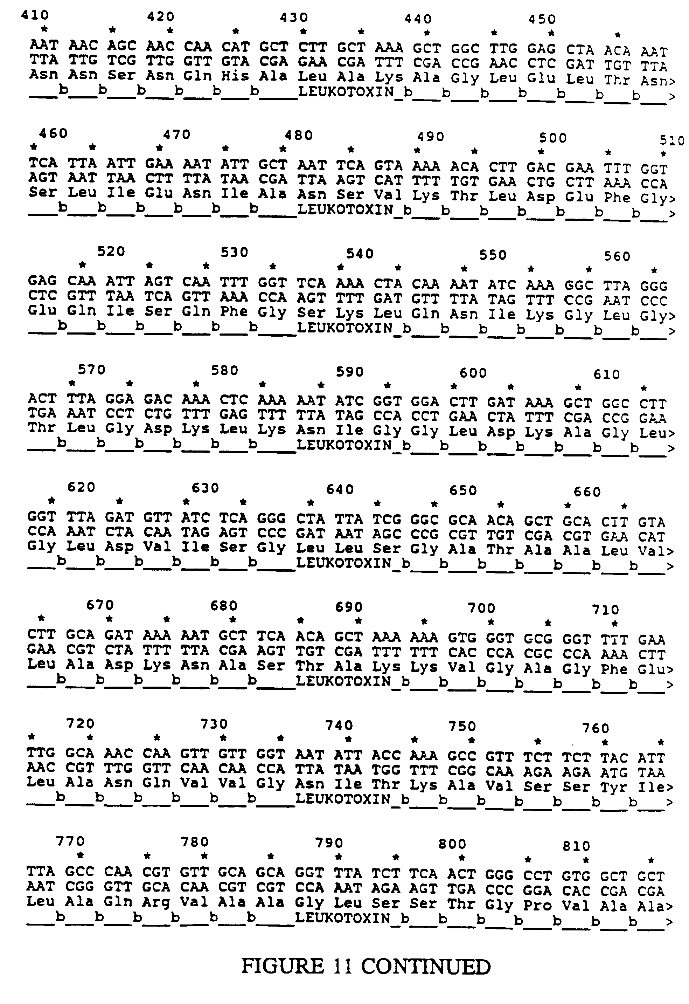

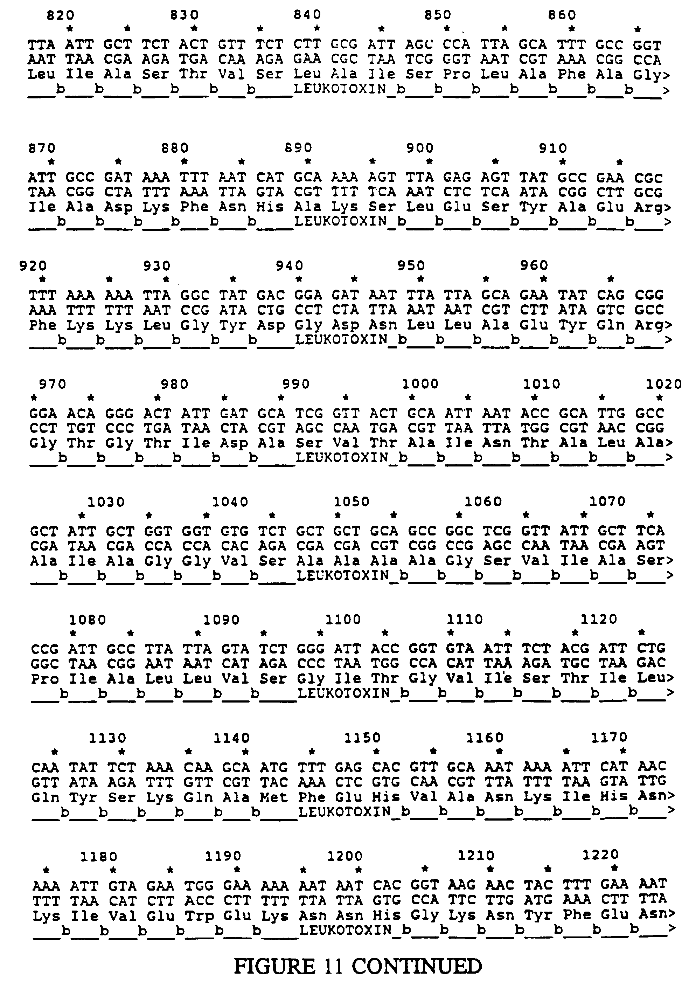

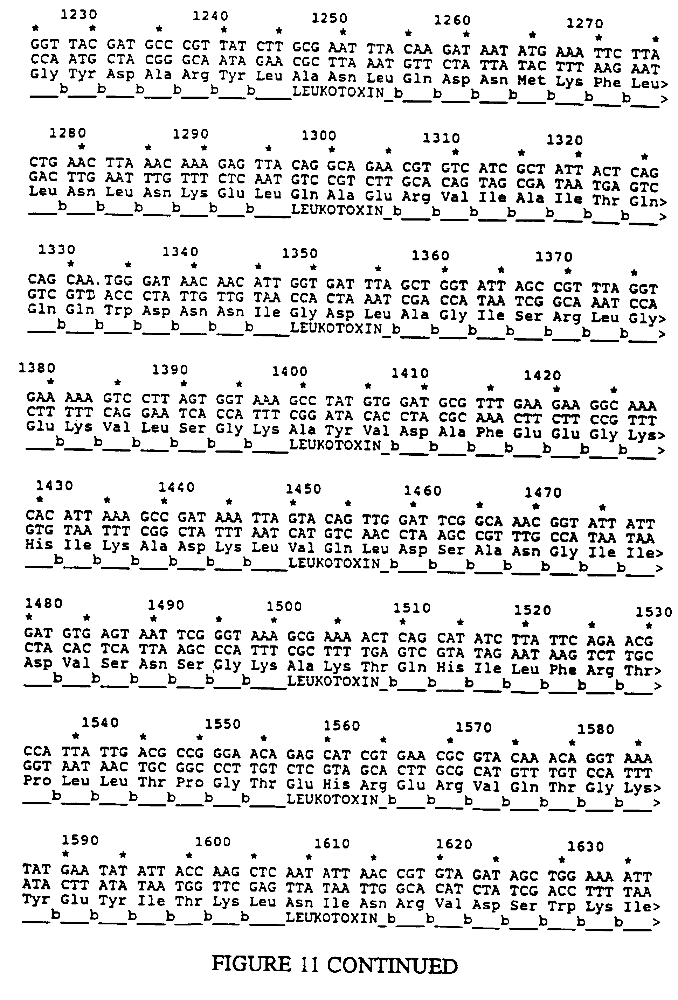

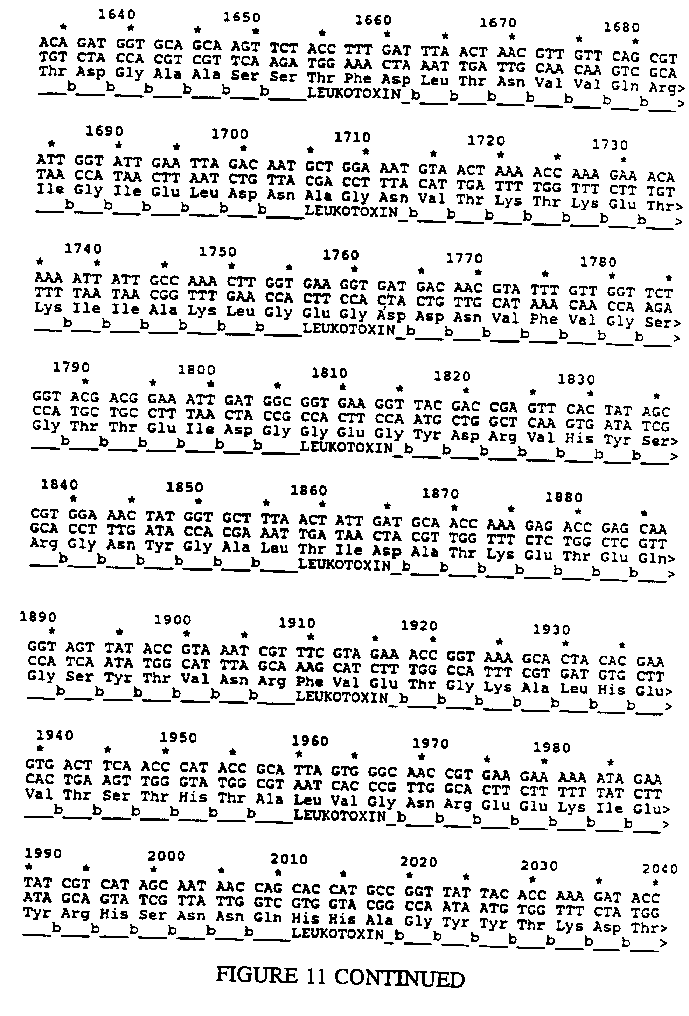

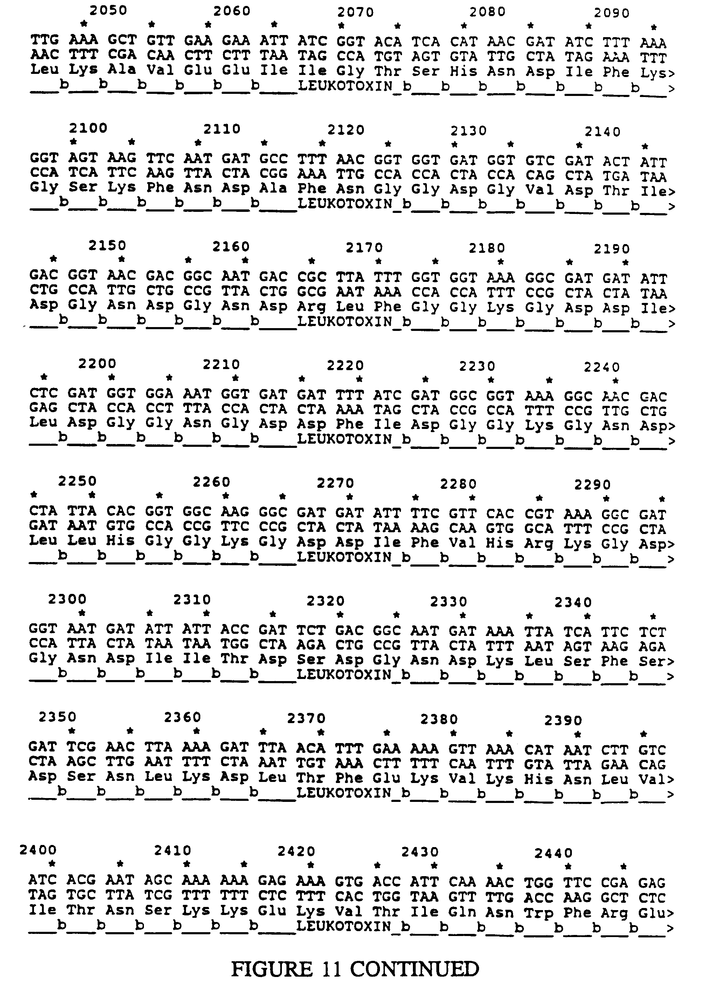

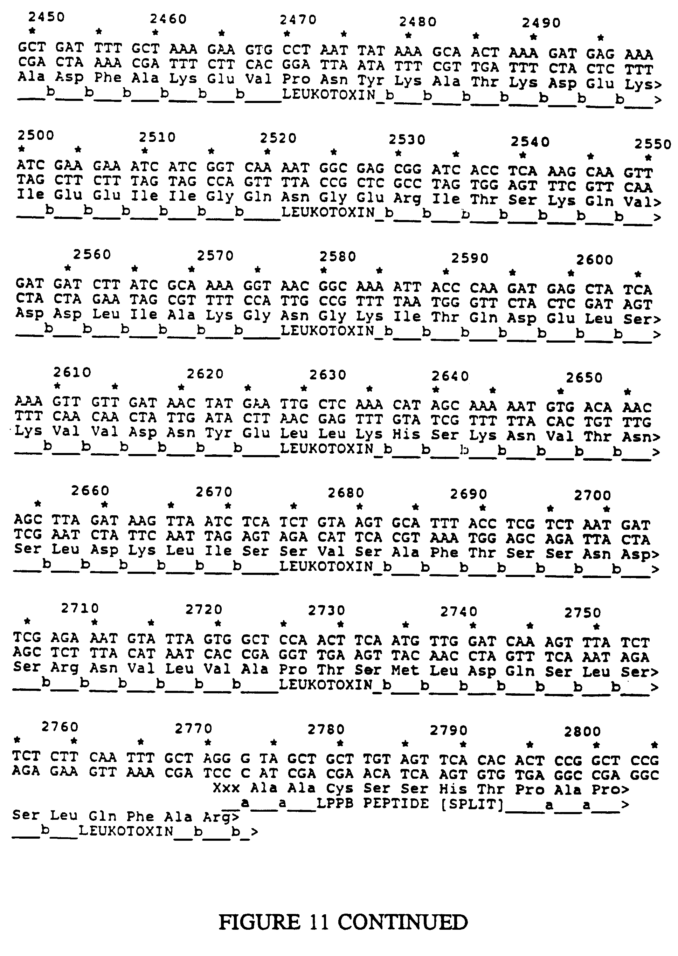

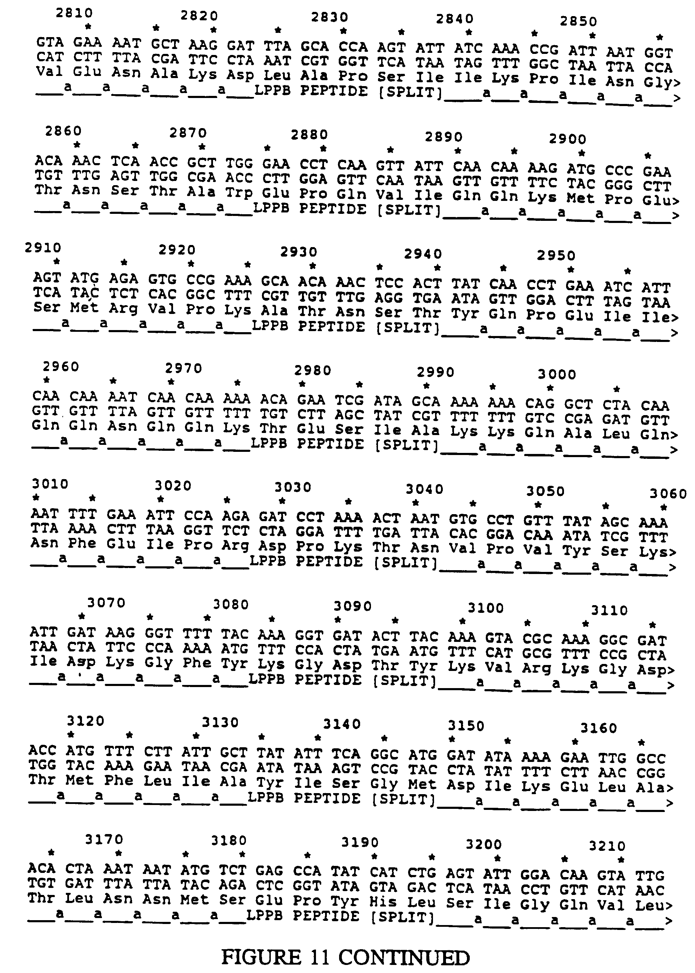

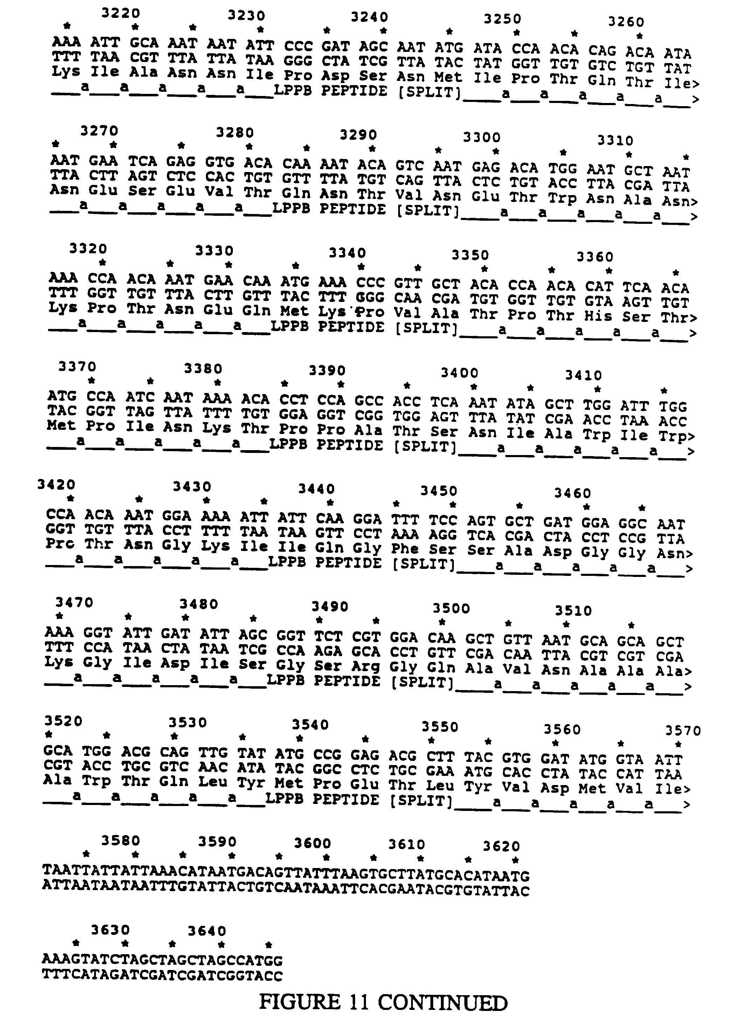

Furthermore, the vaccine compositions of the present invention can include fusion proteins (included in the word "protein" above) comprising fragments of one or more of the *H. somnus* antigens fused to, *i.e.*., a bacterial, fungal, viral or protozoal antigen. For example, chimeric proteins comprising truncated haemin-binding proteins fused to the *P. haemolytica* leukotoxin gene have been constructed and the sequences are depicted in Figures 5 and 6. The chimera in Figure 5 includes a gene coding for a haemin-binding protein lacking the first two amino acid residues of the native product, fused to a truncated leukotoxin molecule, encoded by the *lkt*A gene of *P. haemolytica* (available from ATCC Accession No. 68283). The construct depicted in Figure 6 includes a deletion of the first 32 amino acid residues of the *H. somnus* haemin-binding protein, also fused with the *lkt*A gene of *P. haemolytica.* Similarly, chimeric constructs of *lpp*B*,* fused to the *P. haemolytica lkt*A gene have also been produced and the sequence is depicted in Figure 11. Such chimeric proteins can be produced using recombinant techniques described herein and, e.g., in U.S. Patent No. 4,366,246; Hughes, H.P.A. *et al.* (1992) *Infect. Immun.* 60:565-570; PCT Publication No. WO 88/00971 (published 11 February 1988); and allowed U.S. Patent Application Serial No. 07/571,301.

The vaccine compositions can be used to treat or prevent a wide variety of *H. somnus* infections in animals. Such infections include thromboembolic meningoencephalitis (ITEME), septicemia, arthritis, and pneumonia (Corbelll, L.B., *Can. J. Vet. Res.* (1990) 54:557-562; Harris, F.W., and Janzen, E.D., *Can. Vet. J.* (1990) 30:816-822; Humphrey, J.D., and Stephens, L.R., *Vet. Bull.* (1983) 53:987-1004), as well as myocarditis, pericarditis, spontaneous abortion, infertility and mastitis. Other antigens can also be included in the vaccine compositions, such as the *P. haemolytica* leukotoxin described further below. Thus, the compositions will also serve to prevent diseases caused by these organisms, *i.e.,* respiratory diseases caused by *P. haemolytica,* symptoms of shipping fever and bovine respiratory disease in feedlot cattle, among others.

### Production of the H. somnus Proteins

The above described proteins and active fragments, analogs and chimeric proteins derived from the same, can be produced by a variety of methods. Specifically, the proteins can be isolated directly from *H. somnus* from outer membrane preparations, using standard purification techniques. *See, e.g.* Theisen, M. and Potter, A. *Infect. Immun.* (1992)*,* in press. Alternatively, the proteins can be recombinantly produced as described herein. The proteins can also be synthesized, based on the determined amino acid sequences, using techniques well known in the art.

For example, the proteins can be isolated from bacteria which express the same. This is generally accomplished by first preparing a crude extract which lacks cellular components and several extraneous proteins. The desired proteins can then be further purified *i.e.* by column chromatography, HPLC, immunoadsorbent techniques or other conventional methods well known in the art.

The H. somnus proteins can be conveniently produced as recombinant polypeptides. As explained above, these recombinant products can take the form of partial protein sequences, full-length sequences, or even fusion proteins (*e.g.*, with an appropriate leader for the recombinant host, or with another subunit antigen sequence for *H. somnus* or another pathogen).

The *hmb and hly* genes can be isolated based on the ability of the protein products to bind haemin and display haemolytic activity, respectively. Thus, gene libraries can be constructed and the resulting clones used to transform an appropriate host cell. Colonies can be pooled and screened for clones having these properties. Colonies can also be screened using polyclonal serum or monoclonal antibodies to the desired antigen, for the identification of the *lpp*A, *lpp*B and *lpp*C genes.

Alternatively, once the amino acid sequences are determined, oligonucleotide probes which contain the codons for a portion of the determined amino acid sequences can be prepared and used to screen DNA libraries for genes encoding the subject proteins. The basic strategies for preparing oligonucleotide probes and DNA libraries, as well as their screening by nucleic acid hybridization, are well known to those of ordinary skill in the art. *See, e.g., DNA Cloning:* Vol. I, *supra; Nucleic Acid Hybridization, supra; Oligonucleotide Synthesis,* supra; T. Maniatis *et al.*, *supra.* Once a clone from the screened library has been identified by positive hybridization, it can be confirmed by restriction enzyme analysis and DNA sequencing that the particular library insert contains the desired *H. somnus* gene or a homolog thereof.

Alternatively, DNA sequences encoding the proteins of interest can be prepared synthetically rather than cloned. The DNA sequences can be designed with the appropriate codons for the particular amino acid sequence. In general, one will select preferred codons for the intended host if the sequence will be used for expression. The complete sequence is assembled from overlapping oligonucleotides prepared by standard methods and assembled into a complete coding sequence. *See, e.g.,* Edge (1981) *Nature* 292:756; Nambair *et al.* (1984) *Science* 223:1299; Jay *et al.* (1984) *J. Biol. Chem.* 259:6311.

Once coding sequences for the desired proteins have been prepared or isolated, they can be cloned into any suitable vector or replicon. Numerous cloning vectors are known to those of skill in the art, and the selection of an appropriate cloning vector is a matter of choice. Examples of recombinant DNA vectors for cloning and host cells which they can transform include the bacteriophage λ (*E. coli*)*,* pBR322 (*E. coli*)*,* pACYC177 (*E. coli*)*,* pKT230 (gram-negative bacteria), pGV1106 (gram-negative bacteria), pLAFR1 (gram-negative bacteria), pME290 (non-*E. coli* gram-negative bacteria), pHV14 (*E. coli* and *Bacillus subtilis),* pBD9 *(Bacillus),* pIJ61 *(Streptomyces),* pUC6 *(Streptomyces),* YIp5 (*Saccharomyces*)*,* YCp19 (*Saccharomyces*) and bovine papilloma virus (mammalian cells). See, generally, *DNA Cloning:* Vols. I & II, *supra;* T. Maniatis *et al.*, *supra;* B. Perbal, *supra.*

The gene can be placed under the control of a promoter, ribosome binding site (for bacterial expression) and, optionally, an operator (collectively referred to herein as "control" elements), so that the DNA sequence encoding the desired protein is transcribed into RNA in the host cell transformed by a vector containing this expression construction. The coding sequence may or may not contain a signal peptide or leader sequence. If signal sequences are included, they can either be the native sequences or heterologous sequences. Leader sequences can be removed by the host in post-translational processing. *See, e.g.*, U.S. Patent Nos. 4,431,739; 4,425,437; 4,338,397.

Other regulatory sequences may also be desirable which allow for regulation of expression of the protein sequences relative to the growth of the host cell. Regulatory sequences are known to those of skill in the art, and examples include those which cause the expression of a gene to be turned on or off in response to a chemical or physical stimulus, including the presence of a regulatory compound. Other types of regulatory elements may also be present in the vector, for example, enhancer sequences.

The control sequences and other regulatory sequences may be ligated to the coding sequence prior to insertion into a vector, such as the cloning vectors described above. Alternatively, the coding sequence can be cloned directly into an expression vector which already contains the control sequences and an appropriate restriction site.

In some cases it may be necessary to modify the coding sequence so that it may be attached to the control sequences with the appropriate orientation; *i.e.*, to maintain the proper reading frame. It may also be desirable to produce mutants or analogs of the *H. somnus* protein of interest. Mutants or analogs may be prepared by the deletion of a portion of the sequence encoding the protein, by insertion of a sequence, and/or by substitution of one or more nucleotides within the sequence. Techniques for modifying nucleotide sequences, such as site-directed mutagenesis, are described in, *e.g.,* Sambrook *et al., supra; DNA Cloning,* Vols. I and II, *supra; Nucleic Acid Hybridization, supra.*

The expression vector is then used to transform an appropriate host cell. A number of mammalian cell lines are known in the art and include immortalized cell lines available from the American Type Culture Collection (ATCC), such as, but not limited to, Chinese hamster ovary (CHO) cells, HeLa cells, baby hamster kidney (BHK) cells, monkey kidney cells (COS), human hepatocellular carcinoma cells (*e.g.*, Hep G2), Madin-Darby bovine kidney ("MDBK") cells, as well as others. Similarly, bacterial hosts such as *E. coli, Bacillus subtilis,* and *Streptococcus spp.,* will find use with the present expression constructs. Yeast hosts useful in the present invention include *inter alia, Saccharomyces cerevisiae, Candida albicans, Candida maltosa, Hansenula polymorpha, Kluyveromyces fragilis, Kluyveromyces lactis, Pichia guillerimondii, Pichia pastoris, Schizosaccharomyces pombe* and *Yarrowia lipolytica.* Insect cells for use with baculovirus expression vectors include, *inter alia, Aedes aegypti, Autographa californica, Bombyx mori, Drosophila melanogaster, Spodoptera frugiperda,* and *Trichoplusia ni.*

Depending on the expression system and host selected, the proteins are produced by culturing host cells transformed by an expression vector described above under conditions whereby the protein of interest is expressed. The protein is then isolated from the host cells and purified. If the expression system secretes the protein into the growth media, the protein can be purified directly from the media. If the protein is not secreted, it is isolated from cell lysates. The selection of the appropriate growth conditions and recovery methods are within the skill of the art.

The proteins may also be produced by chemical synthesis such as solid phase peptide synthesis, using known amino acid sequences or amino acid sequences derived from the DNA sequence of the genes of interest. Such methods are known to those skilled in the art. Chemical synthesis of peptides may be preferable if a small fragment of the antigen in question is capable of raising an immunological response in the subject of interest.

The proteins or their fragments can be used to produce antibodies, both polyclonal and monoclonal. If polyclonal antibodies are desired, a selected mammal, (*e.g.*, mouse, rabbit, goat, horse, etc.) is immunized with an antigen of the present invention, or its fragment, or a mutated antigen. Serum from the immunized animal is collected and treated according to known procedures. If serum containing polyclonal antibodies is used, the polyclonal antibodies can be purified by immunoaffinity chromatography, using known procedures.

Monoclonal antibodies to the proteins, and to the fragments thereof, can also be readily produced by one skilled in the art. The general methodology for making monoclonal antibodies by using hybridoma technology is well known. Immortal antibody-producing cell lines can be created by cell fusion, and also by other techniques such as direct transformation of B lymphocytes with oncogenic DNA, or transfection with Epstein-Barr virus. *See, e.g.,* M. Schreier *et al., Hybridoma Techniques* (1980); Hammerling *et al., Monoclonal Antibodies and T-cell Hybridomas* (1981); Kennett *et al., Monoclonal Antibodies* (1980); see also U.S. Patent Nos. 4,341,761; 4,399,121; 4,427,783; 4,444,887; 4,452,570; 4,466,917; 4,472,500, 4,491,632; and 4,493,890. Panels of monoclonal antibodies produced against the antigen of interest, or fragment thereof, can be screened for various properties; *i.e.*., for isotype, epitope, affinity, etc. Monoclonal antibodies are useful in purification, using immunoaffinity techniques, of the individual antigens which they are directed against.

### Vaccine Formulations and Administration

The *H. somnus* proteins can be formulated into vaccine compositions, either alone or in combination with other antigens, for use in immunizing subjects as described below. Methods of preparing such formulations are described in, *e.g., Remington's Pharmaceutical Sciences,* Mack Publishing Company, Easton, Pennsylvania, 15th edition, 1975. Typically, the vaccines of the present invention are prepared as injectables, either as liquid solutions or suspensions. Solid forms suitable for solution in or suspension in liquid vehicles prior to injection may also be prepared. The preparation may also be emulsified or the active ingredient encapsulated in liposome vehicles. The active immunogenic ingredient is generally mixed with a compatible pharmaceutical vehicle, such as, for example, water, saline, dextrose, glycerol, ethanol, or the like, and combinations thereof. In addition, if desired, the vehicle may contain minor amounts of auxiliary substances such as wetting or emulsifying agents and pH buffering agents.

Adjuvants which enhance the effectiveness of the vaccine may also be added to the formulation. Adjuvants may include for example, muramyl dipeptides, avridine, aluminum hydroxide, oils, saponins, cytokines, and other substances known in the art.

The protein may be linked to a carrier in order to increase the immunogenicity thereof. Suitable carriers include large, slowly metabolized macromolecules such as proteins, including serum albumins, keyhole limpet hemocyanin, immunoglobulin molecules, thyroglobulin, ovalbumin, and other proteins well known to those skilled in the art; polysaccharides, such as sepharose, agarose, cellulose, cellulose beads and the like; polymeric amino acids such as polyglutamic acid, polylysine, and the like; amino acid copolymers; and inactive virus particles.

The protein substrates may be used in their native form or their functional group content may be modified by, for example, succinylation of lysine residues or reaction with Cys-thiolactone. A sulfhydryl group may also be incorporated into the carrier (or antigen) by, for example, reaction of amino functions with 2-iminothiolane or the N-hydroxysuccinimide ester of 3-(4-dithiopyridyl propionate. Suitable carriers may also be modified to incorporate spacer arms (such as hexamethylene diamine or other bifunctional molecules of similar size) for attachment of peptides.

Other suitable carriers for the proteins include VP6 polypeptides of rotaviruses, or functional fragments thereof, as disclosed in U.S. Patent No. 5,071,651, incorporated herein by reference. Also useful is a fusion product of a viral protein and the subject immunogens made by methods disclosed in U.S. Patent No. 4,722,840. Still other suitable carriers include cells, such as lymphocytes, since presentation in this form mimics the natural mode of presentation in the subject, which gives rise to the immunized state. Alternatively, the proteins of the present invention may be coupled to erythrocytes, preferably the subject's own erythrocytes. Methods of coupling peptides to proteins or cells are known to those of skill in the art.

Furthermore, the proteins (or complexes thereof) may be formulated into vaccine compositions in either neutral or salt forms. Pharmaceutically acceptable salts include the acid addition salts (formed with the free amino groups of the active polypeptides) and which are formed with inorganic acids such as, for example, hydrochloric or phosphoric acids, or such organic acids as acetic, oxalic, tartaric, mandelic, and the like. Salts formed from free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

Injectable vaccine formulations will contain a "therapeutically effective amount" of the active ingredient, that is, an amount capable of eliciting an immune response in a subject to which the composition is administered. The exact amount is readily determined by one skilled in the art. The active ingredient will typically range from about 1% to about 95% (w/w) of the composition, or even higher or lower if appropriate. With the present vaccine formulations, 50 to 500 µg of active ingredient per ml of injected solution should be adequate to raise an immunological response when a dose of 1 to 3 ml per animal is administered. To immunize a subject, the vaccine is generally administered parenterally, usually by intramuscular injection. Other modes of administration, however, such as subcutaneous, intraperitoneal and intravenous injection, are also acceptable. The quantity to be administered depends on the animal to be treated, the capacity of the animal's immune system to synthesize antibodies, and the degree of protection desired. Effective dosages can be readily established by one of ordinary skill in the art through routine trials establishing dose response curves. The subject is immunized by administration of the vaccine in at least one dose, and preferably two doses. Moreover, the animal may be administered as many doses as is required to maintain a state of immunity to *H. somnus* infection.

Additional vaccine formulations which are suitable for other modes of administration include suppositories and, in some cases, aerosol, intranasal, oral formulations, and sustained release formulations. For suppositories, the vehicle composition will include traditional binders and carriers, such as, polyalkaline glycols, or triglycerides. Such suppositories may be formed from mixtures containing the active ingredient in the range of about 0.5% to about 10% (w/w), preferably about 1% to about 2%. Oral vehicles include such normally employed excipients as, for example, pharmaceutical grades of mannitol, lactose, starch, magnesium, stearate, sodium saccharin cellulose, magnesium carbonate, and the like. These oral vaccine compositions may be taken in the form of solutions, suspensions, tablets, pills, capsules, sustained release formulations, or powders, and contain from about 10% to about 95% of the active ingredient, preferably about 25% to about 70%.

Intranasal formulations will usually include vehicles that neither cause irritation to the nasal mucosa nor significantly disturb ciliary function. Diluents such as water, aqueous saline or other known substances can be employed with the subject invention. The nasal formulations may also contain preservatives such as, but not limited to, chlorobutanol and benzalkonium chloride. A surfactant may be present to enhance absorption of the subject proteins by the nasal mucosa.

Controlled or sustained release formulations are made by incorporating the protein into carriers or vehicles such as liposomes, nonresorbable impermeable polymers such as ethylenevinyl acetate copolymers and Hytrel® copolymers, swellable polymers such as hydrogels, or resorbable polymers such as collagen and certain polyacids or polyesters such as those used to make resorbable sutures. The proteins can also be delivered using implanted mini-pumps, well known in the art.

The proteins can also be administered via a carrier virus which expresses the same. Carrier viruses which will find use with the instant invention include but are not limited to the vaccinia and other pox viruses, adenovirus, and herpes virus. By way of example, vaccinia virus recombinants expressing the proteins can be constructed as follows. The DNA encoding the particular protein is first inserted into an appropriate vector so that it is adjacent to a vaccinia promoter and flanking vaccinia DNA sequences, such as the sequence encoding thymidine kinase (TK). This vector is then used to transfect cells which are simultaneously infected with vaccinia. Homologous recombination serves to insert the vaccinia promoter plus the gene encoding the instant protein into the viral genome. The resulting TKrecombinant can be selected by culturing the cells in the presence of 5-bromodeoxyuridine and picking viral-plaques resistant thereto.

An alternative route of administration involves gene therapy or nucleic acid immunization. Thus, nucleotide sequences (and accompanying regulatory elements) encoding the subject proteins can be administered directly to a subject for *in vivo* translation thereof. Alternatively, gene transfer can be accomplished. by transfecting the subject's cells or tissues *ex vivo* and reintroducing the transformed material into the host. DNA can'be directly introduced into the host organism, *i.e.,* by injection (see International Publication No. WO/90/11092; and Wolff *et al*., *Science* (1990) 247:1465-1468). Liposome-mediated gene transfer can also be accomplished using known methods. *See,* e.g., Hazinski *et al., Am. J. Respir. Cell Mol. Biol. (1991)* 4:206-209; Brigham *et al., Am. J. Med. Sci.* (1989) 298:278-281; Canonico *et al., Clin. Res.* (1991) 39:219A; and Nabel *et al., Science* (1990) 249:1285-1288. Targeting agents, such as antibodies directed against surface antigens expressed on specific cell types, can be covalently conjugated to the liposomal surface so that the nucleic acid can be delivered to specific tissues and cells susceptible to *H. somnus* infection.

Below are examples of specific embodiments for carrying out the present invention. The examples are offered for illustrative purposes only, and are not intended to limit the scope of the present invention in any way.

Example 6 describes the cloning and characterization of LppB. Example 9 describes the construction of leukotoxin-LppB fusion proteins and Example 10 relates to their protective capacity. Example 8 is a comparative Example relating to the protective capacity of LppB, LppB+LppA and LppA. Examples 1 to 5 and 7 relate to other H.somnus proteins that may be included with LppB proteins in vaccine compositions according to the present invention.

### Deposits of Strains Useful in Practicing the Invention

A deposit of biologically pure cultures of the following strains was made with the American Type Culture Collection, 12301 Parklawn Drive, Rockville, Maryland, under the provisions of the Budapest Treaty. The accession number indicated was assigned after successful viability testing, and the requisite fees were paid. The designated deposits will be maintained for a period of thirty (30) years from the date of deposit, or for five (5) years after the last request for the deposit, whichever is longer. Should a culture become nonviable or be inadvertently destroyed, or, in the case of plasmid-containing strains, lose its plasmid, it will be replaced with a viable culture(s) of the same taxonomic description.

| Strain | Deposit Date | ATCC No. |
|---|---|---|
| pRAP117 in *E. coli* JM105 | April 7, 1992 | 68952 |
| pAA504 in *E. coli* MC1061 | April 7, 1992 | 68953 |

### C. Experimental

### Materials and Methods

Enzymes were purchased from commercial sources, and used according to the manufacturers' directions. Radionucleotides and nitrocellulose filters were also purchased from commercial sources.

In the isolation of DNA fragments, except where noted, all DNA manipulations were done according to standard procedures. See Sambrook *et al., supra.* Restriction enzymes, T₄ DNA ligase, *E. coli,* DNA polymerase I, Klenow fragment, and other biological reagents can be purchased from commercial suppliers and used according to the manufacturers' directions. Double stranded DNA fragments were separated on agarose gels.

### Bacterial strains, plasmids and growth condition.

Plasmid pHC79 was used to construct the cosmid library and is commercially available from Boeringer-Mannheim.

*E. coli* strain MC1061 is readily available.

*E. coli* DH5α(φ80, *lacZ*▲M15, *end*A1*, rec*A1, *hsd*R17 *(r*_{*k*}*,m*_{*k*}*+),sup*E44*, thi*-1, ,*gyr*A96, *rel*A1 ▲(*lac*ZYA-*arg*F),U169)/'*lac*l^{q}*pro*AB+*lac*Z▲ M15, Tn5(IKm^{R}) ; and JM105 (*end*A1, *thi, rps*L*, sbc*B15*, hsd*R4*, ▲lac-pro*AB*), [*F*'tra*D36*, pro*AB+*, lac*l^{q}Z▲M15)] are available commercially (i.e. Stratogene) and CC118 *(aro*D139*,* ▲(*ara,leu*)7697, ▲*lac*X74, *pho*A▲20, *gal*E, *gal*K, *thi*, *rps*E, *rpo*B*, arg*Eₐₘ, *rec*A1) from C. Manoil, Harvard University (Manoil, C., and Beckwith, J. *Proc. Natl. Acad. Sci. USA* (1985) 82:8129-8133).

*E. coli* strains were grown in Luria broth (LB) or M9 (Miller, J.H., *Experiments in Molecular Genetics,* (1972) Cold Spring Harbor Laboratory, Cold Spring Harbor, New York). Ampicillin was used at 100 µg/ml and kanamycin at 25 µg/ml unless otherwise indicated.

*H. somnus* strain HS25 has been used in challenge experiments to induce experimental Haemophilosis in calves (Harland, R.J., *et al. Conf. Res. Work. Anim.*. *Dis.* 71st (1990) 29:6). Growth conditions for strain HS25, the plasmid pGH433, and the construction of the genomic library have been described (Theisen, M., and Potter, A.A. *J. Bacteriol.* (1992) 174:17-23). For iron-restricted growth, Brain Heart Infusion broth (BHI-TT) (Difco Laboratories) containing 0.1% Tris base and 0.001% thiamine monophosphate was supplemented with the iron chelator 2,2-dipyridyl (Sigma Chemical Co., St. Louis, Mo) to a final concentration of 100 µM. Iron-replete bacteria were grown in BHI-TT containing 50 µM Fe(NO₃)₃.

### DNA techniques.

Restriction enzymes, Klenow fragment of *E. coli* DNA polymerase I, T4 DNA ligase, and exonuclease III were used as recommended by the suppliers. DNA sequencing was accomplished by the chain termination method, essentially as described by Messing, 1983 (Manoil, C., and Beckwith, J., *Science* (1986) 233:1403-1408). Primer extension was performed as previously described (Theisen, M., *et al. Infect. Immun.* (1992) 60:826-831).

### Screening of H. somnus genomic library.

Recombinant plasmids were transformed into *E. coli* strain JM105 and plated on LB agar plates containing 0.05% Congo red (for LppB and LppC). After two days of incubation at 37°C approximately 0.5% of the colonies turned dark red. Congo red binding colonies were picked and purified to single colonies on identical plates. One of each was then tested for the expression of *H. somnus* antigens by the colony blot method (French, B.T., *et al. Anal. Biochem.* (1986) 156:417-423). LppA was screened by the colony blot method (French, B.T., *et al. Anal. Biochem.* (1986).

### Transposon TnphoA mutagenesis.

Fusions of *lpp*A to Tn*pho*A were created with λ::Tn*pho*A (Gutierrez, C., *et al. J. Mol. Biol.* (1987) 195:289-297). In this system, alkaline phosphatase (AP) activity is only obtained if Tn*pho*A transposes onto a DNA sequence in such a way that AP is fused in frame and downstream of an expressed coding sequence containing appropriate membrane insertional sequences (Hoffman, C.S., and Wright, A. *Proc. Natl. Acad. Sci USA* (1985) 82:5107-5111; Manoil, C., and Beckwith, J. *Proc. Natl. Acad. Sci*. *USA* (1985) 82:8129-8133; Manoil, C., and Beckwith, J. *Science* (1986) 233:1403-1408). Plasmid pMS22 was transformed into strain CC118. The resulting strain was infected with λ::Tn*pho*A and grown for 15 hours at 30°C. Aliquots were plated on LB agar supplemented with 300 µg/ml kanamycin, 100 µg/ml ampicillin, and 40 µg/ml 5-bromo-4-chloro-3-indoyl phosphate (BCIP). The plates were incubated at 30°C for 2-3 days, and plasmid DNA was extracted from five pools of blue colonies and used to transform CC118 cells. Individual AP⁺ (blue) colonies were isolated at 37°C and their plasmid DNA analyzed by restriction mapping.

### PAGE and Immunoblotting.

SDS-PAGE of *H. somnus* and *E. coli* proteins was performed in the Laemmli system (Laemmli, *U.K., Nature* (1970) 227:680-685) or by using the Tricine-SDS polyacrylamide gels with a 16.5%T, 6% C separating gel (Schagger, H., and von Jagow, G. *Anal. Biochem.* (1987) 166:368-379). Transfer of proteins onto nitrocellulose membranes was performed as recommended by the manufacturer. Blots were incubated with bovine serum diluted 1:500 with TBS-1% BSA (10mM Tris-Cl pH 7.5, 140 mM NaCl) for two hours. The antisera used was bovine hyperimmune serum against live *H. somnus* HS25 (Theisen & Potter, 1992) and rabbit serum against *H. somnus* OMPs. After three washes in TBS containing 0.5% Tween 20, seroreactive proteins were detected with goat antibovine-IgG coupled to alkaline phosphatase (Kirkegaard and Perry) at 1:5000 in TBS-1% BSA. Alkaline phosphatase activity was visualized using the NBT/BCIP system as described by the supplier (Promega). Prestained or non-stained protein standards were obtained from BioRad.

### Hybridization techniques.

Northern (RNA) blotting was performed as described by Maniatis. RNA was extracted from *H. somnus* and *E. coli* by standard techniques (Theisen, M. and Potter, A.A. *J. Bacteriol.* (1992) 60:826-831) and electrophoresed through 1.5% agarose gels containing formaldehyde. Three micrograms of RNA was used per lane. The RNA was blotted to nitrocellulose membrane and hybridized to DNA probes labelled at the 5'-end. After hybridization, blots were washed twice in 0.1xSSC, 0.5% SDS for two hours.

### Analysis of plasmid encoded proteins.

Minicells were isolated from cultures of BD1854 containing the appropriate plasmids by centrifugation on a 5% - 25% sucrose gradient, labelled with [³⁵S]methionine, and subjected to SDS-PAGE. The proteins were electroblotted on to nitrocellulose membrane and antigen was detected using hyperimmune serum against HS25. The position of the labelled polypeptides was then determined by autoradiography of the western blot.

### Labeling of proteins with [³H]palmitate.

*E. coli* strain DH5αF'IQ harboring the specified plasmids was grown in M63 medium supplemented with glycerol (0.5% w/v) and casamino acids (2% w/v). *H. somnus* strain HS25 was grown in BHI-TT medium. To exponentially growing cells (4x10⁸ cells/ml), [³H]palmitate (5 mCi/ml) was added to a final concentration of 50 µCi/ml, and incubation was continued for two hours. Labeling was terminated by precipitation with trichloroacetic acid (10% w/v) for 30 min on ice. When indicated, globomycin (Sankyo Co. Tokyo, Japan) (10 mg/ml in dimethyl sulfoxide) was added at 100 µg/ml, 5 min prior to the addition of palmitate. Proteins were pelleted by centrifugation at 15000xg for 20 min, and the pellets were washed twice with methanol to remove lipids. The dried pellets were resuspended in sample buffer and analyzed by Tricine-SDS PAGE. the radiolabeled protein bands in the dried gel were detected by fluorography.

### Oligonucleotide-directed mutagenesis.

A 33-residue synthetic oligonucleotide with the sequence 5'-TGTATTATTAGCAGCTGGTAATGAAAAAAATAA was synthesized to alter the Cys-22 residue of the IppA protein (the underlined base differs from the wild-type sequence). The point mutation in the resulting plasmid pMS67 was verified by DNA sequencing.

### Example 1

### Cloning and Characterization of H. somnus Haemin-Binding Protein and H. somnus Haemolysin

A genomic cosmid library of *H. somnus* HS25 DNA was constructed by cloning fragments, generated by partial *Sau*3A restriction, into the BamHI site of the vector pHC79. The ligated DNA was packaged *in vitro* with a Lambda packaging extract (Promega) and used to infect *E. coli* MC1061. Ampicillin-resistant clones were stored at -70 degrees C. This library was screened for clones which were capable of binding bovine haemin (Sigma) by plating cells on M9 minimal agar (Miller, J.H., *Experiments in Molecular Genetics,* (1972) Cold Spring Harbor Laboratory, Cold Spring Harbor, New York) supplemented with 0.01% haemin. The formation of small dark colonies was indicative of haemin-binding. The library was also screened for clones which displayed haemolytic activity using sheep blood agar plates from Oxoid, Canada. A number of clones exhibiting both the haemin-binding phenotype (Hb+) and the haemolytic phenotype (Hly+). were obtained and two were selected for further study. The plasmid termed pRAP117 (ATCC Accession No. 68952) contained both the haemin-binding (*hmb*) gene and the haemolysin (*hly*) gene on a 25 kb insert (Figure 1). Plasmid pAA504 (ATCC Accession No. 68953) contained the haemolysin (*hly*) gene. pRAP117 and pAA504 were subsequently shown to have similar restriction endonuclease digestion patterns and likely contained overlapping regions of homology. This was confirmed by probing pAA504 DNA with an 8 kb HindIII fragment of pRAP117.

The *hmb* gene was subcloned by ligating the 8 kb HindIII fragment from pRAP117 into the vector pTZ19R (Pharmacia Canada Ltd.). This clone, termed pRAP401 (Figure 1), retained both the haemin-binding and haemolytic activity of the parent. Subsequent subcloning in pTZ19R localized the Hb+, Hly- phenotype to a smaller 3 kb XbaI fragment. This clone was termed pRAP501 (Figure 1). This clone bound haemin but was not haemolytic. Thus, the *hly* gene is located at the distal end of the 8 kb HindIII fragment shown in Figure 1.

Western blotting of the Hb+, Hly- clone with serum raised against HS25 outer membrane proteins (OMPs), detected a protein having an apparent molecular mass of 50,000 kDa that comigrated with an iron-regulated protein from an HS25 OMP-enriched fraction.

### Example 2

### Nucleotide Sequence Analysis of H. somnus Haemin-Binding Protein

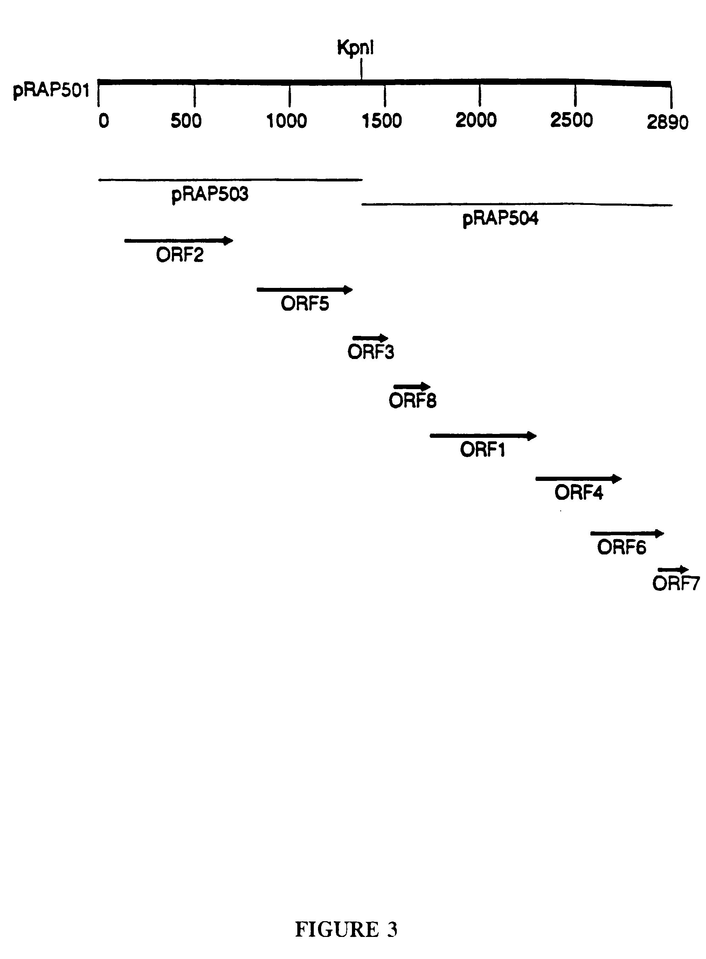

Clone pRAP501 was used to generate Exonuclease III deletions for DNA sequence analysis and sequencing was carried out on single-stranded DNA templates derived from these nested deletions. The sequence is shown in Figure 2. The open reading frames and ribosome binding sites are summarized in Table 1 and Figure 3. As can be seen, there are a total of eight open reading frames which could code for the *hmb* gene. No significant open reading frames were found in the opposite orientation.

**Table 1**

| Predicted Open Reading Frames in Plasmid pRAP501 | | | | | |
|---|---|---|---|---|---|
| | Position | | | Amino | |
| ORF | From | To | Frame | Acids | Potential Ribosome Binding Sites |
| 2 | 29 | 628 | 2 | 200 | AGG at 21 |
| 5 | 735 | 1244 | 3 | 170 | GGA at 727 |
| 3 | 1247 | 1459 | 2 | 71 | GAG at 1236 |
| 8 | 1475 | 1684 | 2 | 70 | AGG at 1466 |
| 1 | 1680 | 2213 | 3 | 178 | AGG at 1672 |
| 4 | 2209 | 2655 | 1 | 149 | AGG at 2198 |
| 6 | 2492 | 2782 | 2 | 97 | GGA at 2486 |
| 7 | 2778 | END | 3 | >37 | GGA at 2767 |

### Example 3

### Localization of the hmb Gene

In order to localize the *hmb* gene, two strategies were used:
(i) subcloning; and
(ii) transposon Tn*pho*A mutagenesis.

A. Subcloning. pRAP501 DNA was digested with *Xba*I*\Kpn*1*,* and the two fragments were ligated into pTZ18 to give plasmids pPRAP503 and pRAP504. pRAP503 contained bases 1 through 1389 from pRAP501 (see Figure 2), while pRAP504 contained the remainder of the insert. Cells containing pRAP504 were capable of binding haemin as determined by plate bioassays, performed as described above, while those containing pRAP503 did not. Therefore, the *hmb* gene was encoded by ORF1, 4, 6, 7, or 8. ORF7 was ruled out due to its small size.

B. Transposon Tn*pho*A mutagenesis. In order to localize the *hmb* gene further, transposon Tn*pho*A mutagenesis was employed. This technique is useful from two points of view:
(i) insertion in an open reading frame will eliminate the function of a gene product; and
(ii) in-frame fusion in an open reading frame coding for a secreted protein will result in blue colonies on BCIP agar due to expression of alkaline phosphatase in the periplasm.
Mutagenesis of CC118/pRAP504 resulted in the isolation of three mutants, two of which were in ORF1 and the other in ORF4. The phenotypes of these mutants are described in Table 2. These results indicate that ORF1 encodes the *hmb* gene. The deduced amino acid sequence for the *hmb* gene is shown in Figure 4. The first 17 amino acids of this protein represent a potential prokaryotic signal peptidase one signal sequence. The identification of ORF1 as the *hmb* gene is supported by the observation that deletions constructed for DNA sequence analysis (see above) which extended into this region abolished haemin-binding, while those outside of this open reading frame had no effect.

**Table 2**

| Properties of Tn*pho*A Fusions | | | | |
|---|---|---|---|---|
| Fusion | ORF | Hmb¹ | Color on BCIP agar | Location (base #)² |
| *pho*A101 | 1 | - | blue | 1824 |
| *pho*A89 | 1 | - | blue | 2100 |
| *pho*A100 | 4 | + | white | N.D. |

| | | | | |
|---|---|---|---|---|
| ¹Hmb = hemin-binding phenotype | | | | |
| ² Location = site of insertion using base numbers from Figure 2 | | | | |
| N.D. = not determined | | | | |

### Example 4

### Expression of hmb

The *hmb* gene was expressed in *E. coli* as a fusion to the *P. haemolytica* leukotoxin gene *lkt*A coded for by plasmid pAA352 (ATCC Accession No. 68283). Plasmid pAA352 was digested with BamHI, treated with mung bean nuclease, and, finally, calf intestinal phosphatase. Two restriction fragments containing the *hmb* gene were then inserted into this vector. The first was a 1.2 kb *Xmn*1\*Sma*I fragment from pRAP501, and the second was a 1.1 kb *Hinc*II fragment from pRAP504. The former starts at the third amino acid residue of ORF1, while the latter starts at the 33rd amino acid residue of the same open reading frame. These plasmids were named pGCH5 and pGCH4, respectively, and their nucleotide plus amino acid sequences are shown in Figures 5 and 6, respectively.

### Example 5

### Cloning and Characterization of LppA

### A. Cloning lppA in E. coli

A genomic library of *H. somnus* HS25 DNA was constructed by cloning 2- to 7-kb fragments, generated by partial *Sau*3A restriction, into the plasmid expression vector pGH433, and positive transformants were detected by the colony blot method (French, B.T., *et al*. *Anal. Biochem.* (1986) 156:417-423) using antiserum against the *H. somnus* strain HS25. Twenty-eight positive clones were identified and kept for further analysis. To identify the plasmid-encoded proteins reacting with the serum, whole cell lysates of IPTG-induced cell cultures were examined by PAGE and subsequent Western blotting. Three plasmids encoding a seroreactive protein with an Mᵣ of approximately 40,000 were identified. One of these, with a DNA insert of 2-kb, was designated pMS22. Using the radiolabeled insert of pMS22 as a probe, it was shown that the three plasmids contained common sequences, indicating that the 40 kDa recombinant proteins were identical. A Western blot of protein synthesized by *E. coli* JM105/pMS22 compared with cell fractions of *H. somnus.* It is apparent that the seroreactive LppA protein is predominantly present in the outer membrane fractions of *H. somnus* and that it comigrates with the recombinant 40 kDa protein. Moreover, serum from calves immunized with the recombinant LppA protein reacts strongly with the native 40 kDa OMP of *H. somnus*.

### B. Analysis of recombinant plasmids

To subclone the *lpp*A gene and construct, plasmids suitable for exonuclease III degradation of the cloned region, the *Bgl*II-*Nco*I fragment of pMS22 was cloned into pTZ18R (Figure 8). Two plasmids, pMS63 and pMS65, with the insert in opposite orientations, were obtained. Both expressed the LppA protein, indicating that the gene is transcribed from a promoter located on the insert DNA. To generate a series of nested deletions, plasmids pMS63 and pMS65 were each cut at the unique *Sac*I and *Bam*HI sites (Figure 8) and subjected to exonuclease degradation, removal of overhang by S1 nuclease, and religation. A number of plasmids were analyzed, the extent of the degradation (as judged by restriction mapping or DNA sequencing) was compared with the phenotype (Figure 8). It appears from this deletion experiment that the *lpp*A gene is located between the deletion endpoints of d.3 and d.8.1 because plasmids with a larger insert are LppA⁺, whereas plasmids with deletion going further into the insert are LppA⁻. This is true with one exception, namely d.10, which produces a seroreactive truncated version of the LppA protein with an Mᵣ of approximately 37,000 (data not shown). DNA sequencing of the deletion endpoints of the two plasmids revealed that in d.10, the α-peptide of *lac*Z is fused in frame with the *lpp*A ORF (see below), thereby allowing the gene to be transcribed from *lac*P or another vector-encoded promoter and translation from the *lac*Z translational start site. In contrast, *lac*Z in d.9 is fused out of frame with the *lpp*A ORF.

### C. DNA sequencing and analysis

The complete DNA sequence of both strands of *lpp*A was determined by the dideoxy method with modified T7 DNA polymerase and single-stranded DNA as the template. The sequence is shown in Figure 7. Only one ORF sufficiently long to encode the *lpp*A gene product is present on the sequenced DNA. It begins with an ATG codon located at position 791-793 and terminates with the TAA stop codon at position 1532-1534. This ORF would encode a polypeptide with a molecular weight of 27,072. The ATG start codon is preceded by a purine-rich sequence AATGAG (underlined bases are complementary to 16 S rRNA), which serves as a ribosome binding site in *E. coli* (Theisen, M., and Potter, A.A. *Infect. Immun.* (1992), in press).

The proposed reading frame was confirmed by sequencing two independent *lpp*A*::*Tn*pho*A gene fusions (see Figure 7). Further proof that the indicated ORF was *lpp*A was obtained by subcloning the *Dra*I fragment of pMS22 (Figure 8) into the *Sma*I site of pTZ18R and generating pMS83 and pMS84, with the insert in opposite orientations. *Dra*I cuts 209 base pairs upstream of the putative ATG start codon and immediately downstream of the TAA stop codon. The *lpp*A protein was expressed in JM105 harboring both plasmids. The N-terminal part of the predicted polypeptide strongly resembles a signal peptide, and the amino acid sequence Leu-Leu-Ala-Ala-Cys at position 842-856 is highly homologous to the consensus cleavage site found in bacterial lipoproteins (von Gabin, A., *et al*. *Proc. Natl. Acad. Sci*. *USA* (1983) 80:652-657).

### D. Identification of the 5' terminus of lppA mRNA.

The 5' terminus of the *lpp*A transcript was determined by primer extension mapping. The DNA used as primer was a synthetic 5'-end labeled oligonucleotide complementary to nucleotides between 817 and 835. mRNA was isolated from the *H. somnus* strain HS25 and the two *E. coli* strains JM105/pMS65(LppA⁺)and JM105/pGH433 (LppA⁻). One major *lpp*A transcript beginning with the A residue at position 756 (Figure 7), is produced in both HS25 and JM105/pMS65. No product was observed in cells harboring the plasmid vector pGH433. A Pribnow box and -35 region, characteristic of *E. coli* promoters (Harley, C.B., and Reynolds, R.P. *Nuc. Acids Res.* (1987) 15:2343-2361), are located at positions 744 through 749 (TATGCT) and position 722 through 727 (TTATCA), respectively.

### E. Post-translational modification of the LppA protein.

Because the deduced amino acid sequence of the LppA protein contains a sequence identical to the consensus sequence Leu-Ala(Gly)-Ala(Gly)-Cys for lipid modification in *E. coli* (von Gabin *et al.,* 1983), the *lpp*A gene product may be a lipoprotein. In order to test whether the LppA protein was lipid modified, [³H]palmitate was incorporated into *H. somnus* HS25 and the two *E. coli* strains, DH5αF'IQ/pMS65 and DH5αF'IQ/pTZ18R. Proteins from whole cell lysates were separated by PAGE and transferred to nitrocellulose membranes. The *lpp*A gene product was identified by immunoblotting with antiserum against HS25. At least ten *H. somnus* proteins were labeled with palmitate. One of these was a 40 kDa protein which reacted strongly with *H. somnus* antiserum, showing that it was the *lpp*A gene product. Palmitate was also incorporated into the recombinant *lpp*A gene product since a radiolabeled, immunoreactive 40 kDa protein comigrating with the LppA protein from HS25 was detected in cells harboring pMS65 but not in the plasmid vector pTZ18R. Thus, the *H. somnus lpp*A gene product is lipid modified in *E. coli.* Treatment of cells with globomycin leads to the accumulation of unprocessed lipoprotein, and both the natural *H. somnus* LppA and recombinant LppA protein are predominantly present as a larger, putative precursor form in globomycin-treated cells.

To determine if lipid modification of the LppA protein occurs at the cysteine residue Cys-22, the cysteine codon (TGT) was changed to a glycine codon (GGT) generating plasmid pMS67. Cells harboring pMS67 were LppA⁺. However, only a seroreactive protein comigrating with the larger precursor form was detected in a Western blot. Globomycin did not alter the mobility of the mutated LppA protein, indicating that the mutated LppA protein was no longer a substrate for signal peptidase II. Moreover, this protein was not labeled with palmitate, showing that lipid modification occurs at the Cys-22 residue.

### Example 6

### Cloning and Characterization of LppB

### A. Cloning of the gene for LppB

A genomic library in plasmid pGH433, constructed as described above, was transformed into JM105 and among several thousand ampicillin-resistant transformants approximately 0.1% were found to bind Congo red on Congo red agar plates (Crb+). The *E. coli* strain JM105 had only a modest ability to bind Congo red on these plates. Twenty Crb+ transformants were screened with hyperimmune serum in a colony blot assay, and five were found to be seroreactive. Western blots (immunoblots) of proteins from whole cells separated on polyacrylamide gels showed that one transformant contained a plasmid (pMS10) encoding an approximately 60 kDa seroreactive protein, three transformants contained plasmids (pMS11, pMS14 and pMS15) encoding an approximately 40 kDa seroreactive protein, and one contained a plasmid (pCRx) coding for a 15 kDa antigen. The radiolabeled DNA insert from pMS11 was found to hybridize to pMS14, pMS15 and *H. somnus,* but not to plasmids pMS10 and pCRx, indicating that the three 40 kDa proteins were identical, but different from the 60 kDa and 15 kDa antigens. Also, the same insert did not hybridize to plasmid pMS22, encoding LppA (Theisen *et al*., 1992) showing that pMS11 encodes a novel 40 kDa protein.

Both JM105/pMS11 and JM105/pMS10 form small dark colonies on minimal plates containing .01% hemin, suggesting that the 40 kDa and 60 kDa proteins could be hemin-binding.

### B. Location of the gene for LppB

The 1.9 kb insert isolated from pMS11 was subcloned in the *Sma*l site of pTZ18R using *E. coli* JM105 as the host strain. Two plasmids, pMS92 and pMS96, were obtained, carrying the insert in opposite orientations. LppB was expressed from both plasmids indicating that *lpp*B is transcribed from a promoter located on the insert DNA. The addition of 2 mM IPTG to the growth medium increased *lpp*B expression from pMS11 approximately four fold (as judged by a western blot) indicating that *lpp*B was on the DNA insert. The indicated plasmids were transformed into a minicell producing strain, and plasmid encoded proteins were analyzed by PAGE. The plasmids pMS11, pMS92 and pMS105 all encode an LppB protein. Thus, LppB must be located downstream on the *Aha*ll site at base 641 in Figure 9.

### C. Nucleotide sequence analysis

To generate a series of nested deletions for sequencing, plasmids pMS92 and pMS96 were each cut at the unique *Sac*l and *Bam*Hl sites present in the vector, subjected to exonuclease degradation, removal of the overhangs by S1 nuclease and religation. Figure 9 shows the sequence of the entire chromosomal fragment. Two large ORFs were identified on the insert. The first ORF starts with an ATG codon at nucleotide 256 and ends with a TAA codon at nucleotide 829. Immediately downstream of this ORF is located a second ORF beginning with an ATG codon at position 872 and ending with a TAA codon at position 1708. The latter appears to correspond to the *lpp*B gene since it is located downstream of the *Aha*ll site at position 641 in Figure 9 and therefore, contained on plasmid second which expressed LppB in the minicell experiment. Upstream from this ORF, there is a putative ribosome binding site GGAG and a seven base pair A/T rich spacer followed by the potential ATG start codon.

The DNA sequence was searched for nucleotide sequence homology in Genbank release 65. Sequences from position 1590 to the end of the cloned DNA in Figure 9 showed 65.5% identity with the *kat*F promoter region from *E. coli* (Mulvey & Loewen, 1989). The *kat*F gene product is a putative sigmafactor which positively regulates catalase HPII (*kat*E) and exonuclease III *(xth)* expression (Sask *et al.* 1989). It is interesting that *H. somnus* has sequences similar to *kat*F because it lacks catalase activity (Sample & Czuprynsky, 1991).

### D. Amino Acid sequence analysis

The ORF in the nucleotide sequence designated *lpp*B encoded 279 amino acid residues, as indicated in Figure 9. The molecular mass of the deduced protein was calculated to be 31307 Daltons. There is a short, hydrophobic region from amino acids 1 to 13 followed by a lipoprotein box, Leu-Ala-Ala-Cys, at the predicted signal peptidase II cleavage site. The hydrophobic-lipoprotein-box sequences strongly resembles the signal peptide of procaryotic lipoproteins, including the recently characterized lipoprotein LppA from *H. somnus*.

The lipid nature of LppB was confirmed as described above.

### Example 7

### Cloning and Characterization of LppC

A genomic library of *H. somnus* DNA was constructed in *E. coli* using the expression vector pGH433, as described above. This library was screened for clones able to bind Congo red by plating cells on LB agar supplemented with ampicillin and 0.05% dye. After two days of incubation at 37°C, approximately 0.1% of the colonies turned dark red. Twenty of these colonies were screened with hyperimmune serum against *H. somnus* in a colony blot assay, and five clones were found to be seroreactive. Western blot analysis of these clones showed that three produced a 40,000 MW protein (LppB; pMS11, pMS14, pMS15), while the other two coded for proteins with molecular weights of 15,000 (pCRR22) and 60,000 (LppC; pMS10). Since Congo Red can act as an analog of porphyrin compounds and one of these clones (pMS10) produced a protein similar in size to other bacterial transferrin receptors, this clone was characterized in more detail.

The DNA insert was subcloned into the vectors pTZ18R and pTZ19R and overlapping deletions were constructed using exonuclease III. The nucleotide sequence of the insert was then determined using the chain termination method and is shown in Figure 10. An open reading frame starting at nucleotide 108 and ending at nucleotide 1850 codes for a protein with a predicted molecular weight of approximately 65,000. The first 21 amino acids of this protein code for a typical procaryotic signal sequence and therefore the DNA coding for the mature protein likely starts at nucleotide 171. This protein has a predicted molecular weight of 63,336, close to the 60,000 MW observed on polyacrylamide gels. This difference can be accounted for by the observation that LppC is lipid modified at the first cysteine of the mature peptide. The predicted amino acid sequence of the mature peptide is shown in Figure 11.

Another construct, pCRR27, was made by taking the insert from pMS10 and subcloning into the vector pTZ18R, giving rise to pCRR26. A *Hind*III digest of pCRR26 was subcloned into the *Hind*III site of pGH432, resulting in plasmid pCRR27. This construct gives a high level of expression of LppC.

The lipid nature of the molecule was confirmed as described above.

### Example 8

### Protective Capacity of LppB, LppB+LppA (Examples) and LppA (Comparative Example)

### A. Antigen Preparation.

The LppA and LppB antigens were extracted from strains JM105/pMS88 and JM105/pMS103, respectively. Bacteria were grown to mid-log phase in one liter of L-broth supplemented with 50 µg/ml of ampicillin. When the absorbance at 600 nm reached 0.6, isopropyl-β,D-thiogalactoside (IPTG) was added to a final concentration of 1 mM and the cultures were incubated with vigorous agitation for 2 h at 37°C. The bacteria were harvested by centrifugation, resuspended in 40 ml of 25% sucrose/50 mM Tris-HCl buffer (pH 8) and frozen at -70°C. The frozen cells were thawed at room temperature and 10 ml of lysozyme (10 mg/ml in 250 mM Tris-HCl, pH 8) was added. After 15 minutes on ice, 300 ml of detergent mix (5 parts of 20 mM Tris-HCl, pH 7.4/300 mM sodium chloride/2% deoxycholic acid/2% Nonidet-P40 and 4 parts of 100 mM Tris-HCl, pH 8/50 mM EDTA/2% Triton X-100) were added. The viscosity was reduced by sonication and protein aggregates were harvested by centrifugation at 27,000 X g for 15 minutes. The pellets were dissolved in a minimal volume of 4 M guanidine hydrochloride. The proteins were analyzed by sodium dodecyl sulfate-polyacrylamide gel electrophoresis and the protein concentration was estimated by comparing the intensity of the Coomassie blue-stained bands to a bovine serum albumin standard.

### B. Vaccine Formulation.

Each vaccine dose was prepared by mixing 100 µg of antigen, alone and in combination, with Emulsigen Plus so that the final volume was 2 ml with an adjuvant concentration of 33% (v/v). Placebo doses were prepared by combining sterile saline with Emulsigen Plus as described above. Each vaccine was mixed by sonication and stored in sterile vaccine vials at 4°C.

### C. Immunization.

All calves were immunized with 2 ml of vaccine administered by intramuscular injection. After three weeks, all animals received a second vaccination as described above. The serological response to vaccination was monitored using serum samples collected prior to vaccination, on the day of the second vaccination, and 10-12 days after the second vaccination.

### D. Vaccine Trial 1.

The objective of this experiment was to determine the serological response to vaccination with two vaccines according to the invention, one comprising LppB and one comprising both LppA + LppB, and, for comparison, with LppA and a placebo. Four of six calves were immunized with these vaccines as described above and the serological response was determined using an enzyme-linked immunosorbent assay (ELISA). The results shown in Table 3 indicate that both antigens elicited an immune response, with LppB being the better of the two. No interference was observed when both antigens were present in the same vaccine.

### E. Vaccine Trial 2.

The objective of this vaccine trial was to determine the protective capacity of LppA (Comparative Example) and LppB using an experimental challenge model. Three groups of eight calves each were vaccinated with LppA, LppB or a placebo formulated as described above. Twelve days after the second vaccination, all animals were challenged by intravenous inoculation of 1 X 10⁸ cfu of *H. somnus* strain HS25. Animals were examined daily for clinical signs of disease for 12 days post-challenge. The results are summarized in Tables 4 to 10. Immunization with LppA reduced the severity of some of the clinical signs of Haemophilosis, including lameness and the daily sick score, while immunization with LppB significantly reduced all clinical signs of disease. Therefore, while both antigens appear to be useful immunogens for the prevention of H. somnus disease, LppB appears to provide improved results over LppA.

### Example 9

### Construction of Leukotoxin-LppB Fusion Proteins

A gene fusion consisting of the *P. haemolytica* leukotoxin gene (*lkt*A), found in plasmid pAA352 (ATCC Accession No. 68283) and LppB, was made in order to increase expression levels. Plasmid pAA352 was digested with BamHI, treated with mung bean nuclease and dephosphorylated with calf intestinal phosphatase. The plasmid pMS11 (described above), containing *lpp*B*,* was digested with *Mae*I and *Acc*I, and the resulting .855 kb fragment was filled in with DNA polymerase I klenow fragment and ligated into the pAA352 vector. Following transformation, clones which reacted with rabbit antisera against LppB in a colony immunoblot were selected, and one such clone, JM105/pCRR28, was shown to produce an IPTG-inducible protein of the correct molecular weight. The predicted nucleotide and amino acid sequence of this fusion is shown in Figure 11.

### Example 10

### Protective Capacity of LktA::LppB

A vaccine trial was conducted using the leukotoxin-LppB fusion protein from Example 9, in order to test its protective capacity. The recombinant protein was prepared from inclusion bodies as described in Example 8. The inclusion bodies were solubilized in 0.5% sodium dodecyl sulfate, and the unbound detergent was removed by dialysis against four litres of tris buffered saline for 48 hours. The proteins were analyzed by SDS-PAGE as described by Laemli (1970), and the protein concentration was estimated by comparing the intensity of the Coomassie blue-stained band to a bovine serum albumin standard (Pierce Chemical Co., Rockford, Illinois). The antigen was formulated in VSA such that the final concentration was 100 µg per ml of LktA::LppB, 30% Emulsigen Plus, 0.9% Tween-80, and 2.5 mg per ml of DDA. The dose volume was 2 cc containing 200 µg of recombinant antigen.

Three groups of eight calves each were included in the trial, and these received the LppB fusion protein vaccine, Somnu-Star (formulated in VSA, obtained from BIOSTAR Inc.) as a positive control and, finally, a placebo. The vaccination and challenge schedule was as described in Example 8. The results of the trial are summarized in Table 11, and it can be seen that vaccination with Somnu-Star or LktA:LppB reduced mortality, clinical score, and weight loss. These results confirm that LppB is a protective antigen of *H. somnus*, and that fusion of the gene coding for LppB to the *P. haemolytica* leukotoxin does not diminish its protective capacity. Since *H. somnus* and *P. haemolytica* vaccines are often formulated together as combination products, this antigen has a further benefit of reducing production costs for such a vaccine.

Thus, *H. somnus* immunogenic LppB protein analogues thereof, immunogenic fragments thereof, and chimeric proteins are disclosed, as are methods of making and using the same. Although preferred embodiments of the subject invention have been described in some detail, it is understood that obvious variations can be made as defined by the appended claims.

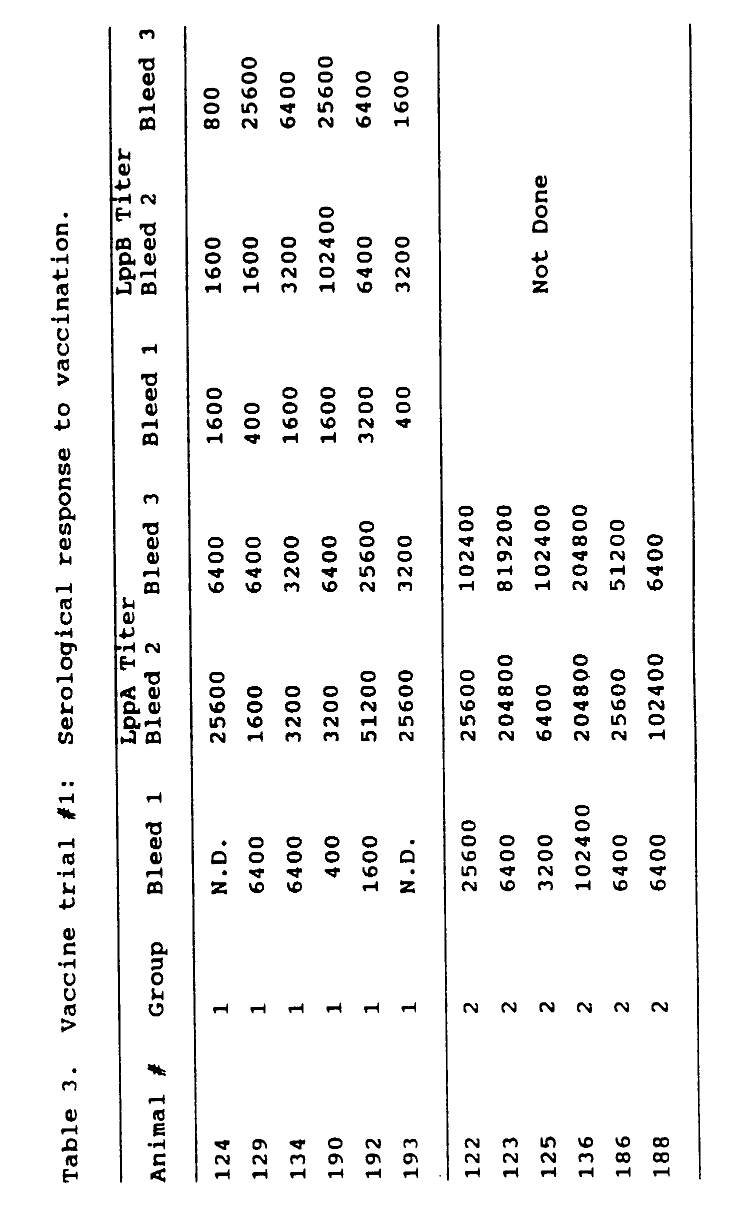

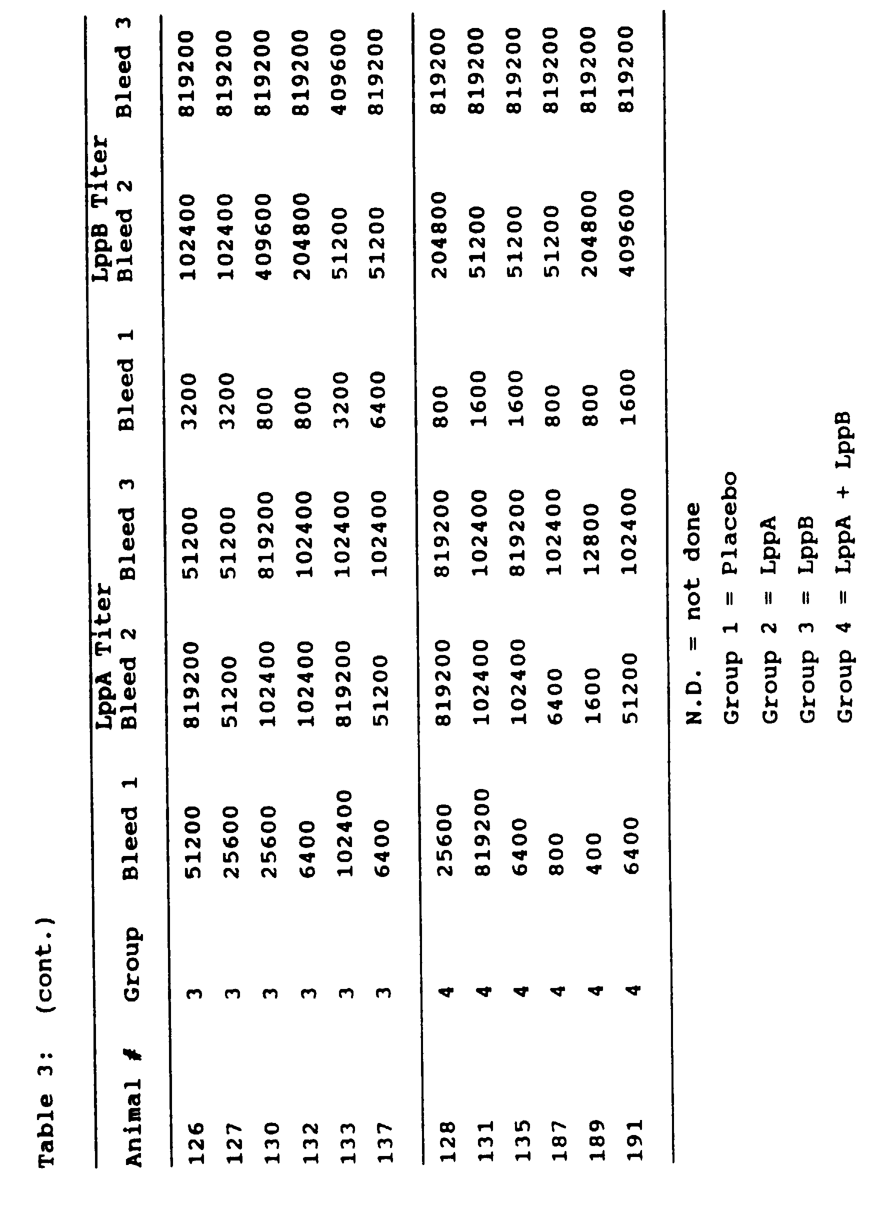

**Table 4.**

| **Vaccine Trial #2: Cumulative Weight Change Per Group** | | | |
|---|---|---|---|
| Day | Placebo | Vac LppA | VacLppB |
| 1 | - 10.4 | - 7.7 | - 3.5 |
| 2 | - 8.6 | - 6.3 | - 3.5 |
| 3 | - 10.4 | - 9.9 | - 4.3 |
| 4 | - 14.4 | - 13.7 | - 7.1 |
| 5 | - 10.8 | - 9.4 | - 4.3 |
| 6 | - 16.2 | - 12.7 | - 7.8 |
| 7 | - 22 | - 18.4 | - 11.9 |
| 8 | - 22.8 | - 17.2 | - 12.4 |
| 9 | - 24.6 | - 20.7 | - 14.4 |
| 10 | - 23.8 | - 21.5 | - 14.7 |
| 11 | - 24 | - 22.5 | - 15.6 |
| 12 | - 27.4 | - 24.5 | - 16.7 |
| Mean | -2.28333 | -2.041667 | -1.391667 |
| Max | - 27.4 | - 24.5 | - 16.7 |

**Table 5.**

| **Vaccine Trail #2: Average Daily Temperatures Per Group** | | | |
|---|---|---|---|
| Day | Placebo | Vac LppA | VacLppB |
| 1 | 39.91 | 39.69 | 39.3 |
| 2 | 39.53 | 39.47 | 39.3 |
| 3 | 39.56 | 39.64 | 39.33 |
| 4 | 39.2 | 39.43 | 39.18 |
| 5 | 39.3 | 39.25 | 39.41 |
| 6 | 38.98 | 39.08 | 39.06 |
| 7 | 39.16 | 39.15 | 39.15 |
| 8 | 39.22 | 39.12 | 38.86 |
| 9 | 38.98 | 39.35 | 38.95 |
| 10 | 39 | 39.42 | 38.83 |
| 11 | 39.2 | 39.37 | 38.98 |
| 12 | 39.38 | 39.13 | 38.86 |
| Mean | 39.285 | 39.34167 | 39.10083 |
| Max | 39.91 | 39.69 | 39.41 |

**Table 6.**

| **Vaccine Trial #2: Average Daily Lameness Score Per Group** | | | |
|---|---|---|---|
| Day | Placebo | Vac LppA VacLppB | |
| 1 | 0 | 0 | 0 |
| 2 | 0.25 | 0 | 0 |
| 3 | 0.2 | 0.143 | 0.063 |
| 4 | 0.2 | 0.083 | 0.125 |
| 5 | 0.2 | 0 | 0.188 |
| 6 | 0.3 | 0.167 | 0.25 |
| 7 | 0.9 | 0.333 | 0.25 |
| 8 | 1.1 | 0.583 | 0.375 |
| 9 | 1 | 0.583 | 0.688 |
| 10 | 1 | 0.5 | 0.625 |
| 11 | 1 | 0.167 | 0.5 |
| 12 | 1.1 | 0.583 | 0.438 |
| Mean | 0.604167 | 0.261833 | 0.291833 |
| Max | 1.1 | 0.583 | 0.688 |

**Table 7.**

| **Vaccine Trial #2: Average Daily Sick Score Per Group** | | | |
|---|---|---|---|
| Day | Placebo | Vac LppA | VacLppB |
| | | | |
| 1 | 0.3 | 0.2 | 0.1 |
| 2 | 0.5 | 0.1 | 0.1 |
| 3 | 0.4 | 0.5 | 0 |
| 4 | 0.3 | 0.3 | 0 |
| 5 | 0.2 | 0.2 | 0.1 |
| 6 | 0.2 | 0.3 | 0.1 |
| 7 | 0.5 | 0.3 | 0.2 |
| 8 | 0.6 | 0.5 | 0.1 |
| 9 | 0.7 | 0.7 | 0.6 |
| 10 | 0.6 | 0.7 | 0.3 |
| 11 | 0.7 | 0.4 | 0.3 |
| 12 | 0.8 | 0.3 | 0.2 |
| Mean | 0.483333 | 0.375 | 0.175 |
| Max | 0.8 | 0.7 | 0.6 |

**Table 8.**

| **Vaccine Trial #2: Daily Number of Calves with Fevers*** | | | |
|---|---|---|---|
| Day | Placebo | Vac LppA | VacLppB |
| 1 | 3 | 3 | 1 |
| 2 | 1 | 1 | 1 |
| 3 | 2 | 2 | 0 |
| 4 | 1 | 2 | 0 |
| 5 | 0 | 1 | 0 |
| 6 | 0 | 1 | 0 |
| 7 | 0 | 1 | 0 |
| 8 | 1 | 1 | 0 |
| 9 | 0 | 2 | 0 |
| 10 | 0 | 1 | 0 |
| 11 | 1 | 1 | 0 |
| 12 | 0 | 1 | 0 |
| Daily Maximum | 3 | 3 | 1 |
| Total | 9 | 17 | 2 |
| * Temperature > = 40.0 | | | |

**Table 9.**

| **Vaccine Trial #2: Daily Number of Calves Sick*** | | | |
|---|---|---|---|
| Day | Placebo | Vac LppA | VacLppB |
| | | | |
| 1 | 4 | 4 | 1 |
| 2 | 4 | 2 | 1 |
| 3 | 5 | 3 | 0 |
| 4 | 4 | 4 | 0 |
| 5 | 4 | 3 | 1 |
| 6 | 4 | 4 | 1 |
| 7 | 6 | 4 | 2 |
| 8 | 7 | 5 | 1 |
| 9 | 7 | 6 | 5 |
| 10 | 7 | 6 | 3 |
| 11 | 7 | 5 | 3 |
| 12 | 7 | 4 | 2 |
| Daily Maximum | 7 | 6 | 5 |
| Total | 62 | 46 | 19 |
| * Clinical Sick Score > 0 (Dead animals counted as sick) | | | |

**Table 10.**

| **Vaccine Trial #2: Summary of Clinical Findings** | | | | |
|---|---|---|---|---|
| **Protection Against *H. somnus* Challenge by Subunit Vaccines** | | | | |
| Vaccines | Calves | Died | Sick | Febrile |
| Placebo | 8 | 3 | 7 | 5 |
| Vaccine LppA | 8 | 2 | 7 | 5 |
| Vaccine LppB | 8 | 0 | 5 | 2 |

**Table 11.**

| **Summary of the LktA::LppB Vaccine trial** | | | | | |
|---|---|---|---|---|---|
| **Group** | **Mortality** | **Mean clinical score** | **Weight change (kg)** | **Serological response** | |
| | | | | **LppB** | **Somnu-Star** |
| Placebo | 2/8 | 1.13 | -5.75 | 5,800 | 8,694 |
| Somnu-Star | 0/8 | 0.38 | -2.38 | 3,201 | 115,057 |
| LktA:LppB | 0/8 | 0.75 | -2.25 | 85,730 | 29,373 |

## Claims

1. A vaccine composition comprising a pharmaceutically acceptable vehicle and a recombinant, immunogenic Haemophilus somnus protein, capable of eliciting a protective immune response against Haemophilus somnus, which protein may be lipidated or non-lipidated and comprises
(a) an amino acid sequence shown at positions 1 to 279, inclusive, of Figure 9; or
(b) an amino acid sequence shown at positions 17 to 279, inclusive, of Figure 9; or
(c) an amino acid sequence which is at least 90% homologous to the amino acid sequence of (a) or (b); or
(d) a fragment of an amino acid sequence according to (a), (b) or (c).

2. A vaccine composition as claimed in claim 1, in which the protein is either non-lipidated or is lipidated by a lipid moiety not normally found in association with the protein.

3. A vaccine composition as claimed in claim 1, in which the protein is lipidated by a lipid moiety usually found in association with the protein.

4. A vaccine composition as claimed in any one of claims 1 to 3, which further comprises an adjuvant.

5. A vaccine composition as claimed in any one of claims 1 to 4, in which the amino acid sequence of (a), (b), (c) or (d) is fused to a non-Haemophilus somnus amino acid sequence.

6. A vaccine composition as claimed in claim 5, wherein the non-Haemophilus somnus amino acid sequence is an amino acid sequence for the P.haemolytica leukotoxin.

7. A vaccine composition as claimed in claim 6, wherein the protein has the sequence shown in Figure 11.

8. A vaccine composition as claimed in any one of claims 1 to 7, which also comprises a Haemophilus somnus protein other than a protein comprising an amino acid sequence as set out in (a), (b), (c) or (d) of claim 1.

9. A method of producing a vaccine composition, said method comprising:
(1) culturing a transformed host cell, the host cell having been transformed with a recombinant vector, under conditions whereby the protein encoded by the coding sequence present in said recombinant vector is expressed, the recombinant vector comprising:
(i) a nucleotide sequence comprising a coding sequence for an immunogenic Haemophilus somnus protein capable of eliciting a protective immune response against Haemophilus somnus, which protein comprises
(a) an amino acid sequence shown at positions 1 to 279, inclusive, of Figure 9; or
(b) an amino acid sequence shown at positions 17 to 279, inclusive, of Figure 9; or
(c) an amino acid sequence which is at least 90% homologous to the amino acid sequence of (a) or (b); or
(d) a fragment of an amino acid sequence according to (a), (b) or (c);
and
(ii) control sequences that are operably linked to said nucleotide sequence whereby said coding sequence can be transcribed and translated in a host cell, and at least one of said control sequences is heterologous to said coding sequence; and
(2) admixing the expressed protein with a pharmaceutically acceptable vehicle.

10. A method as claimed in claim 9, which also comprises transforming a host cell with the recombinant vector to obtain the transformed host cell.

11. Use of a recombinant, immunogenic Haemophilus somnus protein in the manufacture of a vaccine for treating or preventing Haemophilus somnus infection in a vertebrate subject, the protein being capable of eliciting a protective immune response against Haemophilus somnus, being lipidated or non-lipidated and comprising
(a) an amino acid sequence shown at positions 1 to 279, inclusive, of Figure 9; or
(b) an amino acid sequence shown at positions 17 to 279, inclusive, of Figure 9; or
(c) an amino acid sequence which is at least 90% homologous to the amino acid sequence of (a) or (b); or
(d) a fragment of an amino acid sequence according to (a), (b) or (c).

12. Use of a recombinant, immunogenic Haemophilus somnus protein in the manufacture of a vaccine for the treatment of or prevention of thromboembolic meningoencephalitis, septicemia, arthritis, pneumonia, myocarditis, pericarditis, spontaneous abortion, infertility and/or mastitis caused by infection with Haemophilus somnus, the protein being capable of eliciting a protective immune response against Haemophilus somnus, being lipidated or non-lipidated and comprising
(a) an amino acid sequence shown at positions 1 to 279, inclusive, of Figure 9; or
(b) an amino acid sequence shown at positions 17 to 279, inclusive, of Figure 9; or
(c) an amino acid sequence which is at least 90% homologous to the amino acid sequence of (a) or (b); or
(d) a fragment of an amino acid sequence according to (a), (b) or (c).

13. The invention of any one of claims 9 to 12, wherein the protein comprises an amino acid sequence according to (a), (b), (c) or (d) fused to a non-Haemophilus somnus amino acid sequence.

14. The invention as claimed in claim 13, wherein the non-Haemophilus somnus amino acid sequence is an amino acid sequence for the P.haemolytica leukotoxin.

15. The invention as claimed in claim 13, wherein the protein has the sequence shown in Figure 11.

16. A recombinant carrier virus capable of expressing an immunogenic Haemophilus somnus protein, capable of eliciting a protective immune response against Haemophilus somnus, which protein comprises
(a) an amino acid sequence shown at positions 1 to 279, inclusive, of Figure 9; or
(b) an amino acid sequence shown at positions 17 to 279, inclusive, of Figure 9; or
(c) an amino acid sequence which is at least 90% homologous to the amino acid sequence of (a) or (b); or
(d) a fragment of an amino acid sequence according to (a), (b) or (c).

17. A vaccine composition comprising a pharmaceutically acceptable vehicle and a recombinant carrier virus as claimed in claim 16.

18. A vaccine composition as claimed in claim 17, in which the carrier virus is a pox virus, advantageously the vaccina virus, an adenovirus or a herpes virus.

19. A pharmaceutical preparation suitable for nucleic acid immunization, which preparation comprises a pharmaceutically acceptable carrier and a nucleic acid sequence encoding an immunogenic Haemophilus somnus protein, capable of eliciting a protective immune response against Haemophilus somnus, which protein comprises
(a) an amino acid sequence shown at positions 1 to 279, inclusive, of Figure 9; or
(b) an amino acid sequence shown at positions 17 to 279, inclusive, of Figure 9; or
(c) an amino acid sequence which is at least 90% homologous to the amino acid sequence of (a) or (b); or
(d) a fragment of an amino acid sequence according to (a), (b) or (c).

## Patentansprüche

1. Impfzusammensetzung, umfassend ein pharmazeutisch verträgliches Vehikel und ein rekombinantes, immunogenes Haemophilus somnus-Protein, das in der Lage ist, eine protektive Immunantwort gegen Haemophilus somnus hervorzurufen, wobei das Protein lipidiert oder nicht-lipidiert sein kann und umfaßt
(a) eine Aminosäuresequenz, die durch die Positionen 1 bis einschließlich 279 von Figur 9 dargestellt ist, oder
(b) eine Aminosäuresequenz, die durch die Positionen 17 bis einschließlich 279 von Figur 9 dargestellt ist, oder
(c) eine Aminosäuresequenz, die zu mindestens 90% homolog ist zu der Aminosäuresequenz von (a) oder (b), oder
(d) ein Fragment einer Aminosäuresequenz entsprechend (a), (b) oder (c).

2. Impfzusammensetzung nach Anspruch 1, wobei das Protein entweder nicht-lipidiert ist oder mit einer Lipidgruppe lipidiert ist, die normalerweise nicht in Gesellschaft mit dem Protein gefunden wird.

3. Impfzusammensetzung nach Anspruch 1, wobei das Protein mit einer Lipidgruppe lipidiert ist, die normalerweise in Gesellschaft mit dem Protein gefunden wird.

4. Impfzusammensetzung nach einem der Ansprüche 1 bis 3, die darüber hinaus ein Adjuvans umfaßt.

5. Impfzusammensetzung nach einem der Ansprüche 1 bis 4, wobei die Aminosäuresequenz von (a), (b), (c) oder (d) mit einer Aminosäuresequenz verknüpft ist, die nicht von Haemophilus somnus stammt.

6. Impfzusammensetzung nach Anspruch 5, wobei die Aminosäuresequenz, die nicht von Haemophilus somnus stammt, eine Aminosäuresequenz für das P.haemolytica-Leukotoxin ist.

7. Impfzusammensetzung nach Anspruch 6, wobei das Protein die in Figur 11 dargestellte Sequenz aufweist.

8. Impfzusammensetzung nach einem der Ansprüche 1 bis 7, die noch ein Haemophilus somnus-Protein umfaßt, das ein anderes Protein ist, als dasjenige, das eine Aminosäuresequenz umfaßt, die in (a), (b), (c) oder (d) von Anspruch 1 dargestellt ist.

9. Verfahren zum Herstellen einer Impfzusammensetzung, wobei das Verfahren umfaßt:
(1) Züchten einer transformierten Wirtszelle, wobei die Wirtszelle mit einem rekombinanten Vektor unter Bedingungen transformiert worden ist, bei denen dasjenige Protein exprimiert wird, das durch die in dem rekombinanten Vektor vorliegende Codierungssequenz codiert wird, wobei der rekombinante Vektor umfaßt:
(i) eine Nukleotidsequenz, umfassend eine Codierungssequenz für ein immunogenes Haemophilus somnus-Protein, das in der Lage ist, eine protektive Immunantwort gegen Haemophilus somnus hervorzurufen, wobei das Protein umfaßt
(a) eine Aminosäuresequenz, die durch die Positionen 1 bis einschließlich 279 von Figur 9 dargestellt ist, oder
(b) eine Aminosäuresequenz, die durch die Positionen 17 bis einschließlich 279 von Figur 9 dargestellt ist, oder
(c) eine Aminosäuresequenz, die zu mindestens 90% homolog ist zu der Aminosäuresequenz von (a) oder (b), oder
(d) ein Fragment einer Aminosäuresequenz entsprechend (a), (b) oder (c),
und
(ii) Kontrollsequenzen, die funktionsfähig verknüpft sind mit der Nukleotidsequenz, wobei die Codierungssequenz in eine Wirtszelle transkribiert und translatiert werden kann, und wobei mindestens eine der Kontrollsequenzen zu der Codierungssequenz heterolog ist, und
(2) Mischen des exprimierten Proteins mit einem pharmazeutisch verträglichen Vehikel.

10. Verfahren nach Anspruch 9, das darüber hinaus das Transformieren einer Wirtszelle mit dem rekombinanten Vektor umfaßt, um die transformierte Wirtszelle zu erhalten.

11. Verwendung eines rekombinanten, immunogenen Haemophilus somnus-Proteins bei der Herstellung eines Impfstoffes zur Behandlung oder Verhinderung einer Haemophilus somnus-Infektion in einem Vertebraten, wobei das Protein, das in der Lage ist, eine protektive Immunantwort gegen Haemophilus somnus hervorzurufen, lipidiert oder nicht-lipidiert ist und umfaßt
(a) eine Aminosäuresequenz, die durch die Positionen 1 bis einschließlich 279 von Figur 9 dargestellt ist, oder
(b) eine Aminosäuresequenz, die durch die Positionen 17 bis einschließlich 279 von Figur 9 dargestellt ist, oder
(c) eine Aminosäuresequenz, die zu mindestens 90% homolog ist zu der Aminosäuresequenz von (a) oder (b), oder
(d) ein Fragment einer Aminosäuresequenz entsprechend (a), (b) oder (c).

12. Verwendung eines rekombinanten, immunogenen Haemophilus somnus-Proteins bei der Herstellung eines Impfstoffes zur Behandlung oder zur Prävention von thromboembolischer Meningoencephalitis, Septikämie, Arthritis, Pneumonie, Myocarditis, Pericarditis, spontanem Abort, Infertilität und/oder Mastitis, die durch Infektion mit Haemophilus somnus verursacht werden, wobei das Protein in der Lage ist, eine protektive Immunantwort gegen Haemophilus somnus hervorzurufen, und wobei das Protein lipidiert oder nicht-lipidiert ist und umfaßt
(a) eine Aminosäuresequenz, die durch die Positionen 1 bis einschließlich 279 von Figur 9 dargestellt ist, oder
(b) eine Aminosäuresequenz, die durch die Positionen 17 bis einschließlich 279 von Figur 9 dargestellt ist, oder
(c) eine Aminosäuresequenz, die zu mindestens 90% homolog ist zu der Aminosäuresequenz von (a) oder (b), oder
(d) ein Fragment einer Aminosäuresequenz entsprechend (a), (b) oder (c).

13. Erfindung nach einem der Ansprüche 9 bis 12, wobei das Protein eine Aminosäuresequenz entsprechend (a), (b), (c) oder (d), verknüpft mit einer Aminosäuresequenz, die nicht von Haemophilus somnus stammt, umfaßt.

14. Erfindung nach Anspruch 13, wobei die Aminosäuresequenz, die nicht von Haemophilus somnus stammt, eine Aminosäuresequenz für das P.haemolytica-Leukotoxin ist.

15. Erfindung nach Anspruch 13, wobei das Protein die in Figur 11 dargestellte Sequenz aufweist.

16. Rekombinanter Carriervirus, der in der Lage ist, ein immunogenes Haemophilus somnus-Protein zu exprimieren, das in der Lage ist, eine protektive Immunantwort gegen Haemophilus somnus hervorzurufen, wobei das Protein umfaßt
(a) eine Aminosäuresequenz, die durch die Positionen 1 bis einschließlich 279 von Figur 9 dargestellt ist, oder
(b) eine Aminosäuresequenz, die durch die Positionen 17 bis einschließlich 279 von Figur 9 dargestellt ist, oder
(c) eine Aminosäuresequenz, die zu mindestens 90% homolog ist zu der Aminosäuresequenz von (a) oder (b), oder
(d) ein Fragment einer Aminosäuresequenz entsprechend (a), (b) oder (c).

17. Impfzusammensetzung, die ein pharmazeutisch verträgliches Vehikel und einen rekombinanten Carriervirus nach Anspruch 16 umfaßt.

18. Impfzusammensetzung nach Anspruch 17, bei der der Carriervirus ein Pockenvirus, vorteilhafterweise der Vaccina-Virus, ein Adenovirus oder ein Herpesvirus ist.

19. Pharmazeutische Zubereitung, die zur Nucleinsäureimmunisierung geeignet ist, wobei die Zubereitung einen pharmazeutisch verträglichen Träger und eine Nucleinsäuresequenz umfaßt, die ein immunogenes Haemophilus somnus-Protein codiert, das in der Lage ist, eine protektive Immunantwort gegen Haemophilus somnus hervorzurufen, wobei das Protein umfaßt.
(a) eine Aminosäuresequenz, die durch die Positionen 1 bis einschließlich 279 von Figur 9 dargestellt ist, oder
(b) eine Aminosäuresequenz, die durch die Positionen 17 bis einschließlich 279 von Figur 9 dargestellt ist, oder
(c) eine Aminosäuresequenz, die zu mindestens 90% homolog ist zu der Aminosäuresequenz von (a) oder (b), oder
(d) ein Fragment einer Aminosäuresequenz entsprechend (a), (b) oder (c).

## Revendications

1. Composition de vaccin comprenant un véhicule acceptable en pharmacie et une protéine immunogène recombinée de Haemophilus somnus, capable de déclencher une réponse immunitaire protectrice contre Haemophilus somnus, laquelle protéine peut être lipidée ou non lipidée, et comprend :
(a) une séquence d'acides aminés présentée en positions 1 à 279, bornes comprises, de la Figure 9 ; ou
(b) une séquence d'acides aminés présentée en positions 17 à 279, bornes comprises, de la Figure 9 ; ou
(c) une séquence d'acides aminés qui est homologue à au moins 90 % de la séquence d'acides aminés de (a) ou (b) ; ou
(d) un fragment d'une séquence d'acides aminés selon (a), (b) ou (c).

2. Composition de vaccin selon la revendication 1, dans laquelle la protéine est soit non lipidée soit lipidée par un fragment lipidique qui ne se trouve normalement pas en association avec la protéine.

3. Composition de vaccin selon la revendication 1, dans laquelle la protéine est lipidée par un fragment lipidique se trouvant habituellement en association avec la protéine.

4. Composition de vaccin selon l'une quelconque des revendications 1 à 3, qui comprend en outre un adjuvant.

5. Composition de vaccin selon l'une quelconque des revendications 1 à 4, dans laquelle la séquence d'acides aminés de (a), (b), (c) ou (d) est fusionnée à une séquence d'acides aminés non Haemophilus somnus.

6. Composition de vaccin selon la revendication 5, dans laquelle la séquence d'acides aminés non Haemophilus somnus est une séquence d'acides aminés pour la leucotoxine P. haemolytica.

7. Composition de vaccin selon la revendication 6, dans laquelle la protéine a la séquence présentée sur la Figure 11.

8. Composition de vaccin selon l'une quelconque des revendications 1 à 7, qui comprend aussi une protéine de Haemophilus somnus autre qu'une protéine comprenant une séquence d'acides aminés telle qu'indiquée en (a), (b), (c) ou (d) de la revendication 1.

9. Procédé pour produire une composition de vaccin, ledit procédé comprenant :
(1) la mise en culture d'une cellule hôte transformée, la cellule hôte ayant été transformée par un vecteur de recombinaison, dans des conditions grâce auxquelles la protéine codée par la séquence codante présente dans ledit vecteur de recombinaison est exprimée, le vecteur de recombinaison comprenant :
(i) une séquence de nucléotides comprenant une séquence codante pour une protéine immunogène de Haemophilus somnus capable de déclencher une réponse immunitaire protectrice contre Haemophilus somnus, laquelle protéine comprend :
(a) une séquence d'acides aminés présentée en positions 1 à 279, bornes comprises, de la Figure 9 ; ou
(b) une séquence d'acides aminés présentée en positions 17 à 279, bornes comprises, de la Figure 9 ; ou
(c) une séquence d'acides aminés qui est homologue à au moins 90 % de la séquence d'acides aminés de (a) ou (b) ; ou
(d) un fragment d'une séquence d'acides aminés selon (a), (b) ou (c) ;
et
(ii) des séquences de commande qui sont liées de façon opérationnelle à ladite séquence de nucléotides, grâce auxquelles ladite séquence codante peut être transcrite ou traduite dans une cellule hôte, et au moins l'une desdites séquences de commande est hétérologue de ladite séquence codante ; et
(2) mélanger la protéine exprimée avec un véhicule acceptable en pharmacie.

10. Procédé selon la revendication 9, qui comprend aussi la transformation d'une cellule hôte avec le vecteur de recombinaison pour obtenir la cellule hôte transformée.

11. Utilisation d'une protéine immunogène recombinée de Haemophilus somnus dans la fabrication d'un vaccin pour traiter ou prévenir une infection par Haemophilus somnus chez un sujet vertébré, la protéine étant capable de déclencher une réponse immunitaire protectrice contre Haemophilus somnus, étant lipidée ou non limitée, et comprenant :
(a) une séquence d'acides aminés présentée en positions 1 à 279, bornes comprises, de la Figure 9 ; ou
(b) une séquence d'acides aminés présentée en positions 17 à 279, bornes comprises, de la Figure 9 ; ou
(c) une séquence d'acides aminés qui est homologue à au moins 90 % de la séquence d'acides aminés de (a) ou (b) ; ou
(d) un fragment d'une séquence d'acides aminés selon (a), (b) ou (c).

12. Utilisation d'une protéine immunogène recombinée de Haemophilus somnus dans la fabrication d'un vaccin pour le traitement ou la prévention de méningoencéphalite thromboembolique, de septicémie, d'arthrite, de pneumonie, de myocardite, de péricardite, d'avortement spontané, d'infertilité et/ou de mastite, provoqués par une infection par Haemophilus somnus, la protéine étant capable de déclencher une réponse immunitaire protectrice contre Haemophilus somnus, étant lipidée ou non lipidée, et comprenant :
(a) une séquence d'acides aminés présentée en positions 1 à 279, bornes comprises, de la Figure 9 ; ou
(b) une séquence d'acides aminés présentée en positions 17 à 279, bornes comprises, de la Figure 9 ; ou
(c) une séquence d'acides aminés qui est homologue à au moins 90 % de la séquence d'acides aminés de (a) ou (b) ; ou
(d) un fragment d'une séquence d'acides aminés selon (a), (b) ou (c).

13. Invention selon l'une quelconque des revendication 9 à 12, dans laquelle la protéine comprend une séquence d'acides aminés selon (a), (b), (c) ou (d) fusionnée à une séquence d'acides aminés non Haemophilus somnus.

14. Invention selon la revendication 13, dans laquelle la séquence d'acides aminés non Haemophilus somnus est une séquence d'acides aminés pour la leucotoxine P. haemolytica.

15. Invention selon la revendication 13, dans laquelle la protéine a la séquence présentée sur la Figure 11.

16. Virus porteur de recombinaison capable d'exprimer une protéine immunogène de Haemophilus somnus, capable de déclenche une réponse immunitaire protectrice contre Haemophilus somnus, laquelle protéine comprend :
(a) une séquence d'acides aminés présentée en positions 1 à 279, bornes comprises, de la Figure 9 ; ou
(b) une séquence d'acides aminés présentée en positions 17 à 279, bornes comprises, de la Figure 9 ; ou
(c) une séquence d'acides aminés qui est homologue à au moins 90 % de la séquence d'acides aminés de (a) ou (b) ; ou
(d) un fragment d'une séquence d'acides aminés selon (a), (b) ou (c).

17. Composition de vaccin comprenant un véhicule acceptable en pharmacie et un virus porteur de recombinaison selon la revendication 16.

18. Composition de vaccin selon la revendication 17, dans laquelle le virus porteur est un poxvirus, avantageusement le virus de la vaccine, un adénovirus ou un herpèsvirus.

19. Préparation pharmaceutique convenant à une immunisation par acides nucléiques, laquelle préparation comprend un porteur acceptable en pharmacie et une séquence d'acides nucléiques codant pour une protéine de Haemophilus somnus, capable de déclencher une réponse immunitaire protectrice contre Haemophilus somnus, laquelle protéine comprend :
(a) une séquence d'acides aminés présentée en positions 1 à 279, bornes comprises, de la Figure 9 ; ou
(b) une séquence d'acides aminés présentée en positions 17 à 279, bornes comprises, de la Figure 9 ; ou
(c) une séquence d'acides aminés qui est homologue à au moins 90 % de la séquence d'acides aminés de (a) ou (b) ; ou
(d) un fragment d'une séquence d'acides aminés selon (a), (b) ou (c).
